(19) Europäisches Patentamt
European Patent Office
Office européen des brevets

(11) **EP 4 281 129 B1**

(12) **EUROPEAN PATENT SPECIFICATION**

(45) Date of publication and mention of the grant of the patent:
**10.12.2025 Bulletin 2025/50**

(21) Application number: **22701277.0**

(22) Date of filing: **21.01.2022**

(51) International Patent Classification (IPC):
***A61L 27/18*** *(2006.01)* ***A61L 27/52*** *(2006.01)*

(52) Cooperative Patent Classification (CPC):
**A61L 27/18; A61L 27/52;** A61L 2400/06; A61L 2430/24

(86) International application number:
**PCT/NL2022/050029**

(87) International publication number:
**WO 2022/158978 (28.07.2022 Gazette 2022/30)**

(54) **INJECTABLE CUSHIONING HYDROGELS**

INJIZIERBARE DÄMPFENDE HYDROGELE

HYDROGELS D'AMORTISSEMENT INJECTABLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.01.2021 NL 2027371**

(43) Date of publication of application:
**29.11.2023 Bulletin 2023/48**

(73) Proprietors:
- **Supra B B.V.**
  **5612 AX Eindhoven (NL)**
- **Stichting Amsterdam UMC**
  **1081 HV Amsterdam (NL)**

(72) Inventors:
- **BOSMAN, Anton Willem**
  **5613 LK Eindhoven (NL)**
- **SMIT, Theodoor Henri**
  **5262 XB Vught (NL)**
- **KERKHOFFS, Gino Matheus Melanie Johannes**
  **3634 AN Loenersloot (NL)**
- **STORNEBRINK, Tobias**
  **1054 VA Amsterdam (NL)**

(74) Representative: **Arnold & Siedsma**
**Bezuidenhoutseweg 57**
**2594 AC The Hague (NL)**

(56) References cited:
**US-A1- 2017 210 843** **US-A1- 2017 340 774**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 281 129 B1

## Description

### FIELD OF THE INVENTION

The invention relates to injectable liquid hydrogel formulations comprising polymer gellants that after cross-linking with an aqueous auxiliary composition result in elastic and cushioning hydrogels that also comprise reversible cross-links which favor stress relaxation. These cushioning hydrogels can be used in the prevention and treatment of osteoarthritis.

### BACKGROUND OF THE INVENTION

A joint or articulation in a vertebrate is the connection between bones of the skeleton. Synovial joints, such as in ankle, knee and fingers, are able to transmit large forces perpendicular to their surface and substantial, almost frictionless, movement along. This is possible through the presence of cartilage covering the ends of the bones, and synovium that lubricates the joint and is contained within a flexible membrane. In contrast to bone, cartilage does not contain nerves, so loading and sliding do not cause any pain. Cartilage is composed of proteoglycans, collagens and chondrocytes, the cells within the cartilage. These cells experience an all-sided hydraulic pressure and hardly any shear (distortional) stress. Under these conditions, they produce new collagens and proteoglycans to repair micro-damage.

Cartilage can be damaged by trauma, substantial overuse and/or inflammation. If the matrix is damaged, most often by the failure of collagens, proteoglycans are released and the hydraulic pressure inside the cartilage is lost. The chondrocytes inside the cartilage lose volume and experience a more direct force from joint loading and consequently a distortional strain. As a result, they lose their spherical shape and become elongated, which induces a cellular reaction leading to inflammation and the production of enzymes that further break down the matrix. This vicious circle of degradation may evolve unnoticed for several years until eventually the entire articular cartilage layer is broken down to the underlying bone; a painful condition called osteoarthritis, which is the most common type of joint disease.

Although osteoarthritis is a multifactorial disease, involving synovial inflammation and degeneration of the cartilage matrix, it is becoming increasingly accepted that mechanical trauma forms the onset of the disease in a (very) large percentage of patients. The inventors have found that mechanical trauma results in radiologically invisible but mechanically measurable bruises in the articular cartilage. In such cartilage bruises, the area of impact is 50-90% softer than the adjacent cartilage, because the collagen II network is disrupted by the impact and because water-binding proteoglycans are released, thus reducing the hydrostatic pressure of the cartilage matrix. Further loading, from additional trauma or simply from daily activities, drives the cartilage into a vicious circle of matrix damage, loss of water, increased shear loading of chondrocytes, inflammatory and katabolic gene expressions, and subsequent further breakdown of the matrix. This causes the bruised area to expand and the quality of the cartilage to deteriorate. Typically, this vicious circle of degeneration takes 10-15 years to become painful and clinically apparent, only to find out that the cartilage has deteriorated to such an extent that nothing can be done anymore to heal the join, and invasive and mutilating surgery is needed. As high mechanical loading forms a stimulus for cartilage degeneration, dampening this mechanical load can decrease its destructive stimulus. As shown by D.L. Bader et al., Arthritis, 2011, 2011, 979032, restoration of hydrostatic pressure drives chondrocytes into an anabolic, matrix producing mode, which can regenerate the damage induced by trauma. On the other hand, complete unloading results in disuse atrophy of cartilage, showing that there is specific mechanical loading regime necessary for the regeneration of cartilage that is a tradeoff between the loading and unloading of cartilage.

Hence, there is a need for a suitable material that dampens mechanical loading of articular cartilage, yet not resulting in complete unloading of the joint. Furthermore, the material should restore the hydrostatic pressure around the chondrocytes and thereby stimulate them to produce new matrix and heal the damaged cartilage tissue. Such an implant could prevent traumatic cartilage bruises from progressing into symptomatic osteoarthritis by providing a mechanical environment that eliminates high shear stresses through joint loading and instead cushions the damaged cartilage and stimulate healing of the cartilage tissue. Clearly, the material that is used for such an implant should be able to dissipate energy (in order to spread and reduce loading on the bruised cartilage), able to withstand large loading forces without failure (in order to be used in weightbearing joints during the activities of daily life), injectable (in order for the intervention to be minimally invasive), and be biodegradable within a certain amount of time (in order to make it a temporary prevention or treatment and eliminate the risk of long-term immunological foreign body reactions).

Current management of osteoarthritis focuses on a temporary suppression of symptoms - for example by means of oral or local pain medication - until the symptoms are so severe that only surgical fusion or replacement of the joint can provide relief.

There are only limited possibilities to stop, slow or reverse the vicious circle of cartilage degradation. Furthermore, to the best of the knowledge of the inventors, there are no techniques available to prevent or treat the first (traumatic) focal (osteo)chondral (micro-)damage that initiates the vicious circle of cartilage degradation.

Previously, patients with osteoarthritis in the knee received an external distraction device, which reduced the mechanical loads in the knee joint while retaining their mobility. After one year of follow-up, new articular cartilage had grown in the joints of most of these patients, resulting in significant clinical benefit (reduced pain, improved quality of life), as shown by K. Wiegant et al., Osteoarthritis Cartilage, 21, 2013, pp 1660-1667. While joint distraction provides favorable results in the knee, the application of an external distractor is an inconvenient therapy with considerable risk for infections. Furthermore, in comparison to knee distraction, external distraction of other joints such as the ankle appeared more problematic (E.C. Rodriguez-Merchan., Arch. Bone Jt. Surg., 5, 2017, pp 208-212).

Another approach for treatment that is being investigated is tissue engineering of new cartilage using human derived cells and/or proteins. WO2011/059325 A2 discloses injectable hydrogels comprising hyaluronic acid grafted with tyramine modified dextran that cross-link *in situ* and that can be loaded with living cells that can grow new cartilage whereby the polysaccharide-based hydrogel readily degrades due to enzymatic hydrolysis by hyaluronidases (1-3 weeks).

E. Jooybar et al., Acta Biomat. 83, 2019, pp 233-244, disclose, for example, injectable hydrogels comprising hyaluronic acid grafted with tyramine modified dextran that cross-link *in situ.* The disclosed hydrogels comprise additionally platelet lysate obtained from human peripheral blood and human bone marrow derived mesenchymal stem cells (hMSCs) and are shown to be useful for tissue engineering of new cartilage whereby the hydrogel is degraded by hyaluronidase within a week. Accordingly, these hydrogels act as temporary cartilage substitutes that fill the defects in cartilage and release drugs to enhance regeneration of cartilage.

Hydrogels can be characterized by three-dimensional networks of polymer chains that can absorb polar solvents such as water. They find applications in water management and in medical applications including bone transplants, tissue adhesives, drug delivery systems and pharmaceuticals. Hydrogels can occur in a cross-linked form or in an uncross-linked form. Cross-linking usually provides stiffer gels due to a strong limitation in the molecular mobility of the polymer chains. Cross-linking can be achieved chemically by the formation of covalent bonds (permanent) or physically by the formation of reversible bonds (dynamic), such as hydrogen bonds or ionic interactions. Obviously, cross-linking can also be achieved by both chemical and physical means. An example of a reversible bond based on hydrogen bonding is a dimer of ureidopyrimidone-derivatives (example of 4H-units).

WO2006/118460 A1 discloses supramolecular hydrogel materials comprising water gellants that are comprised of hydrophilic polymers to which at least two 4H-units are covalently attached via urethane-alkyl moieties. However, these reversible hydrogen-bonded hydrogel materials are insufficient in strength for several applications and their viscosity is too high at biomedically relevant temperatures to allow administration via liquid processing techniques like injection through a syringe.

EP1972661A1 discloses supramolecular hydrogels that comprise 4H-units together with urea bonding-motifs in a hydrophilic polymer. These hydrogels are thermo-reversible due to their supramolecular nature and the absence of covalent cross-links in the hydrogels. However, their reversible nature may also result in dissolving of the gel when an excess water is present such as inside the body.

M.M.C. Bastings et al., Adv. Healthcare Mat., 3, 2014, pp 70-78, disclose that a hydrogel of a specific embodiment of EP1972661A1, consisting of a poly(ethyleneglycol) of 10.000 Da with 4H-unit end groups, can be rendered injectable only when dissolved in strongly basic aqueous solution with a pH higher than 8.5, which is not favored for biomedical applications. Moreover, the resulting hydrogel had very limited elastic strength and was only used for drug delivery in the myocardium.

P.Y.W. Dankers et al., Adv. Mater., 24, 2012, pp 2703-2709 disclose transient hydrogel networks based on specific embodiments of EP1972661A1, being supramolecular polymers consisting of polyethylene glycols which are end-functionalized with 4H-units. The transient nature of these hydrogels 4H-units results again in soft hydrogels. Moreover, it is shown that these drug-loaded hydrogels dissolve within days both *in vitro* as *in vivo,* thereby preventing their possible use in load bearing applications in which long (multiple weeks or months) residence times are needed.

US10358521E1 discloses adhesive injectable hydrogels comprising 4H-units and chemically cross-linkable moieties with adhesive properties towards tissue with adhesive lap shear strengths of at least 10 kPa and Young's moduli below 100 kPa at 25 °C. The hydrogels are disclosed to be useful in biomedical or cosmetic products for controlled drug delivery, tissue engineering, wound care, tissue-adhesion, or as tissue sealant, artificial cartilage material, cardiac patch, transdermal patch, cardio-vascular structure, and coating for a medical device. Application as artificial cartilage material requires injection of the adhesive hydrogel to or into existing damaged or degenerated cartilage where the hydrogel adheres to the cartilage, such as inside cracks or other defects. Typically, the damaged cartilage needs to be conditioned first before applying the artificial cartilage material. This pre-conditioning may require surgery. Merely injecting the adhesive hydrogel will not result in (artificial) cartilage material. The adhesive hydrogel needs to be injected to or into existing damaged or degenerated cartilage along with bio-additives that are needed to initiate cartilage growth, such as for example stem cells, autologous chondrocytes, or platelet-rich plasma (PRP).

From H. Wang et al., Adv. Mat., 27, 2015, pp 3717-3736, it follows that adaptable hydrogels, which are hydrogels that comprise reversible crosslinks, are promising platforms for tissue growth. Through breaking and re-forming of the reversible linkages, such as 4H-units, adaptable hydrogels can be locally modified to permit complex cellular functions

while maintaining their long-term integrity and provision of mechanical cues needed for functional tissue growth. This in clear contrast to permanent (hydrogel) materials. In addition, these adaptable materials can have biomimetic viscoelastic properties that make them well suited for medical applications in which new tissues need to be formed. Especially, the specific visco-elastic nature of such hydrogels appears to be a factor to modulate cell behavior as shown in A.R. Cameron et al. Biomaterials 35, 2014, pp 1857-1868 for the differentiation of human mesenchymal stem cells. This differentiation is a direct result of the visco-elastic nature of the hydrogels investigated that induces increased expressions of molecular cell differentiation factors. Moreover, human nasal chondrocytes have been shown in C. Correia et al. Tissue Engin. A 18, 2012, pp 1979-1991, to detect and respond to physical forces, thus resembling joint loading, by enhancing cartilage tissue development in a frequency- and amplitude-dependent manner when they were encapsulated in a thermo-reversible polysaccharide hydrogel *in vitro.*

US2017/340774 A1 discloses an injection formulation that effectively relieves pain caused by arthritis, protects cartilage, and suppresses synovial inflammation even with single injection into a joint, and thus completed the present invention. Such an injection exhibits high biocompatibility and sustainability in a human body by being easily injectable with a syringe due to low viscosity at the time of injection and causing a reaction between two types of polyethylene glycol (PEG) derivatives to gradually form a peptide bond, which leads to the formation of hydrogel containing hyaluronic acid and exhibiting excellent viscoelasticity.

Because of the shortcomings of current treatments of osteoarthritis, there is a need for an improved minimal-invasive treatment.

It is therefore an objective of the invention to provide improved and/or minimal-invasive methods for the treatment or prevention of osteoarthritis in a vertebrate, wherein said method preferably does not require implanting solid devices, the use of external distraction devices and/or administering tissue-derived active ingredients.

It is a further objective of the invention to provide compositions for use in improved and/or minimal-invasive methods for the treatment or prevention of osteoarthritis in a vertebrate, wherein said compositions preferably do not comprise tissue-derived active ingredients.

Yet another objective of the invention is to provide improved and/or minimal-invasive methods for the treatment or prevention of osteoarthritis in a vertebrate, and compositions for use in these methods, wherein said treatment and prevention of osteoarthritis comprises the prevention of initial (micro-)damage to the cartilage in a vertebrate at risk of developing osteoarthritis, the subclinical phase, wherein only initial (micro-)damage to the cartilage is present, up to the symptomatic phase, wherein cartilage degeneration has proceeded.

## SUMMARY OF THE INVENTION

The inventors have unexpectedly found that the formation of certain hydrogels in the cavity of a synovial joint of a vertebrate provides a cushion or a cushioning effect in between the articular joint surfaces of said synovial cavity, thereby spreading and reducing the joint contact loads in such a way that cartilage tissue is preserved for further damage and/or regeneration of cartilage tissue is induced. Additionally, this results in internal distraction without aid/use of other products or devices. These hydrogels can effectively absorb shocks between the joint surfaces because they are highly elastic and are able to dissipate stress very well. This is due to the presence of reversible hydrogen bonded cross-links in the hydrogel mediated by the incorporated moieties. Furthermore, the cushion which is loaded by physiological joint forces, creates hydrostatic pressure that promotes matrix deposition by the chondrocytes that comprises collagens, proteoglycans and non-collagenous proteins.

In this way, the origination of focal (osteo-)chondral (micro-)damage can be prevented. When focal (osteo-) chondral (micro-)damage is already present, the invention ensures that pain is reduced and further cartilage degeneration is prevented or reduced.

Administration of the liquid precursors of these hydrogels to the cavity of the synovial joint may be performed right after trauma or overloading, or even prior to expected damage via minimal invasive techniques. The liquid precursors of this hydrogel can also be administered to the cavity of an already degenerated osteoarthritic synovial joint. In this case, the resulting cushion may acutely contribute to pain reduction and reduction or prevention of further cartilage degeneration.

Accordingly, the medical uses as disclosed herein can be applied to all three stages (a), (b) and (c) of the development of osteoarthritis as depicted in Figure 1.

These hydrogels show limited bio-degradability in the cavity of a synovial joint of a vertebrate and no adhesion to cartilage material. Moreover, these hydrogels comprise chemical and/or physical cross-links, preferably chemical and physical cross-links.

Accordingly, in a first aspect, the invention provides a kit of parts, consisting of compositions (C1) and (C2) in separate containers, for use in the treatment or prevention of osteoarthritis in a vertebrate, wherein:

(a) composition (C1) is a liquid hydrogel formulation comprising 50.0 - 99.7 wt.% of water, based on the weight of composition (C1), 0.3 - 50.0 wt.% of a polymer gellant according to Formula (VIII) and 0 - 20 wt.% of further ingredients:

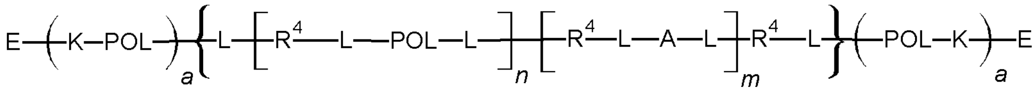

(VIII)

wherein:

- *m* and *n* are each integers independently in the range of 1 to 20;
- *a* is an integer chosen from 0 or 1;
- K is a linking group selected from amide, ester or ether;
- E is a group that can react with another group E initiated or mediated by one or more compounds Y, wherein the one or more compounds Y are selected from the group consisting of oxidant, oxidase, peroxidase and combinations thereof, in aqueous auxiliary formulation (C2), thereby forming one or more covalent bonds between different groups E, or E is a group that can react with one or more cross-linkers Z in aqueous auxiliary formulation (C2), wherein the one or more cross-linkers Z have two or more reactive groups, thereby forming covalent bonds between E and the one or more cross-linkers Z;
- POL is a linear polymeric group having an average molecular weight $M_n$, as determined by end group titration, of about 250 to about 30000 Da;
- A represents moieties selected from the group consisting of Formulas (I-B) to (V-B), wherein A is connected to L via the bonds marked with an asterisk:

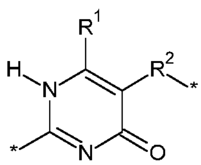

(I-B)

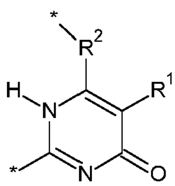

(II-B)

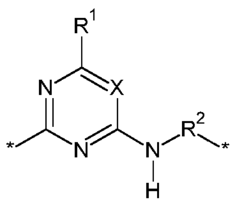

(III-B)

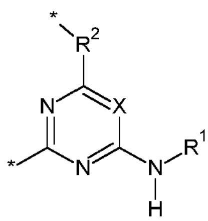

(IV-B)

$*\text{-}R^3\text{-}*$ (V-B)

- X is N or $CR^1$;
- each L independently is a urethane or urea linking group, with the proviso that any moiety A according to Formula (I-B) and (II-B) is always coupled to a urea linking group L on the 2-position of the 4-pyrimidone, any moiety A according to Formula (III-B) and (IV-B) is always coupled to a urea linking group L on the 4-position of the 1,3,5-triazine for X=N, or on the 2-position of the pyrimidine for X is $CR^1$, and any moiety A according to Formula (V-B) is always coupled to two urea linking groups L;
- $R^1$ is selected from the group consisting of hydrogen and $C_1$ - $C_{20}$ alkyl;
- $R^2$ is selected from the group consisting of $C_1$ - $C_{20}$ alkylene;
- $R^3$ is selected from the group consisting of a direct covalent bond, linear $C_1$ - $C_{24}$ alkylene groups, branched $C_2$ - $C_{24}$ alkylene groups, cyclic $C_3$ - $C_{24}$ alkylene groups, $C_6$ - $C_{24}$ arylene groups, $C_7$ - $C_{24}$ alkarylene groups and $C_7$ - $C_{24}$ arylalkylene groups, wherein the alkylene groups, arylene groups, alkarylene groups and arylalkylene groups optionally comprise 1 - 5 heteroatoms selected from the group consisting of O, N and S; and
- $R^4$ is selected from the group consisting of linear or branched $C_2$ - $C_{20}$ alkylene groups and cyclic $C_3$ - $C_{24}$

alkylene groups; and

(b) composition (C2) is an aqueous auxiliary formulation comprising 75.0 - 99.9 wt.% of water, based on the weight of composition (C2), 0 - 5 wt.% of further ingredients and 0.1 - 20 wt.% of:

- one or more dissolved cross-linkers Z having two or more reactive groups that can chemically cross-link the polymer gellant according to Formula (VIII) by forming covalent bonds with two or more groups E; or
- one or more dissolved compounds Y, selected from the group consisting of oxidant, oxidase, peroxidase and combinations thereof, that initiate or mediate chemical cross-linking of two or more groups E on the polymer gellant according to Formula (VIII);

wherein said treatment comprises combining compositions (C1) and (C2) to form a liquid hydrogel composition (C3) and administering said liquid hydrogel composition (C3) to the synovial fluid or to the cavity of a synovial joint of the vertebrate, to form a cross-linked hydrogel (C4) in the cavity of the synovial joint of the vertebrate.

In a second aspect, the invention concerns a kit of parts, consisting of compositions (C1) and (C2) in separate containers, for use in the treatment or prevention of osteoarthritis in a vertebrate, wherein

(a) composition (C1) is a liquid hydrogel formulation comprising 50.0 - 99.7 wt.% of water, based on the weight of composition (C1), 0.3 - 50.0 wt.% of a polymer gellant according to Formula (VIII) and 0 - 20 wt.% of further ingredients:

$$E\left(K-POL\right)_a\left\{L\left[R^4-L-POL-L\right]_n\left[R^4-L-A-L\right]_m R^4-L\right\}\left(POL-K\right)_a E$$

(VIII)

wherein:

- *m* and *n* are each integers independently in the range of 1 to 20;
- *a* is an integer chosen from 0 or 1;
- K is a linking group selected from amide, ester or ether;
- E is a group that can react with another group E initiated or mediated by one or more compounds Y, wherein the one or more compounds Y are selected from the group consisting of oxidant, oxidase peroxidase and combinations thereof, in aqueous auxiliary formulation (C2), thereby forming one or more covalent bonds between different groups E, or E is a group that can react with one or more cross-linkers Z in aqueous auxiliary formulation (C2), wherein the one or more cross-linkers Z have two or more reactive groups, thereby forming covalent bonds between E and the one or more cross-linkers Z;
- POL is a linear polymeric group having an average molecular weight $M_n$, as determined by end group titration, of about 250 to about 30000 Da;
- A represents moieties selected from the group consisting of Formulas (I-B) to (V-B), wherein A is connected to L via the bonds marked with an asterisk:

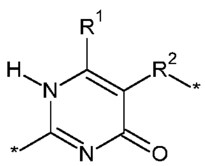

(I-B)

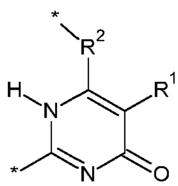

(II-B)

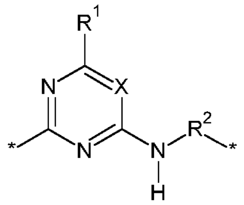

(III-B)

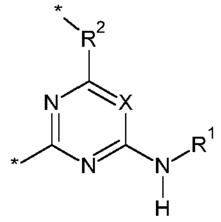

(IV-B)

*-$R^3$-* (V-B)

- X is N or $CR^1$;
- each L independently is a urethane or urea linking group, with the proviso that any moiety A according to Formula (I-B) and (II-B) is always coupled to a urea linking group L on the 2-position of the 4-pyrimidone, any moiety A according to Formula (III-B) and (IV-B) is always coupled to a urea linking group L on the 4-position of the 1,3,5-triazine for X=N, or on the 2-position of the pyrimidine for X is $CR^1$, and any moiety A according to Formula (V-B) is always coupled to two urea linking groups L;
- $R^1$ is selected from the group consisting of hydrogen and $C_1$ - $C_{20}$ alkyl;
- $R^2$ is selected from the group consisting of $C_1$ - $C_{20}$ alkylene;
- $R^3$ is selected from the group consisting of a direct covalent bond, linear $C_1$ - $C_{24}$ alkylene groups, branched $C_2$ - $C_{24}$ alkylene groups, cyclic $C_3$ - $C_{24}$ alkylene groups, $C_6$ - $C_{24}$ arylene groups, $C_7$ - $C_{24}$ alkarylene groups and $C_7$ - $C_{24}$ arylalkylene groups, wherein the alkylene groups, arylene groups, alkarylene groups and arylalkylene groups optionally comprise 1 - 5 heteroatoms selected from the group consisting of O, N and S; and
- $R^4$ is selected from the group consisting of linear or branched $C_2$ - $C_{20}$ alkylene groups and cyclic $C_3$ - $C_{24}$ alkylene groups; and

(b) composition (C2) is an aqueous auxiliary formulation comprising 75.0 - 99.9 wt.% of water, based on the weight of composition (C2), 0 - 5 wt.% of further ingredients and 0.1 - 20 wt.% of:

- one or more dissolved cross-linkers Z having two or more reactive groups that can chemically cross-link the polymer gellant according to Formula (VIII) by forming covalent bonds with two or more groups E; or
- one or more dissolved compounds Y, selected from the group consisting of oxidant, oxidase, peroxidase and combinations thereof, that initiate or mediate chemical cross-linking of two or more groups E on the polymer gellant according to Formula (VIII);

wherein said treatment comprises in a first step administering one of the compositions (C1) or (C2), preferably composition (C1), to the synovial fluid or to the cavity of a synovial joint of the vertebrate and in a second step administering the other of the compositions (C1) or (C2), to form a liquid hydrogel composition (C3) in the cavity of the synovial joint of the vertebrate that reacts to a cross-linked hydrogel (C4).

In a third aspect, the invention concerns a kit of parts, consisting of an injectable hydrogel ('composition C5') for use in the treatment or prevention of osteoarthritis in a vertebrate, wherein said treatment comprises administering the injectable hydrogel to the synovial fluid or to the cavity of a synovial joint of the vertebrate after which it forms a cross-linked hydrogel that comprises chemical and/or physical cross-links, whereby the physical cross-links are chosen from hydrogen bonding, ionic interactions, hydrophobic interactions and Van der Waals forces, wherein the chemical crosslinks are covalent bonds formed by self-condensation or by reaction between dissimilar functional groups, and wherein the injectable hydrogel comprises 50.0 - 99.7 wt.% of water and 0.3 -50.0 wt.% of a hydrophilic polymer that is the precursor for the cross-linked hydrogel.

## BRIEF DESCRIPTION OF THE FIGURE

Figure 1 schematically depicts the different stages of (the development of) osteoarthritis that are relevant to the present invention. Stage (a) primarily concerns prevention, stage (b) concerns the prevention of further degeneration and promotion of intrinsic cartilage repair, and stage (c) concerns symptom relief.

## DETAILED DESCRIPTION OF THE INVENTION

In a first aspect, the invention concerns a kit of parts, consisting of compositions (C1) and (C2) in separate containers, for use in the treatment or prevention of osteoarthritis in a vertebrate, wherein:

(a) composition (C1) is a liquid hydrogel formulation comprising 50.0 - 99.7 wt.% of water, based on the weight of composition (C1), 0.3 - 50.0 wt.% of a polymer gellant according to Formula (VIII) and 0 - 20 wt.% of further ingredients:

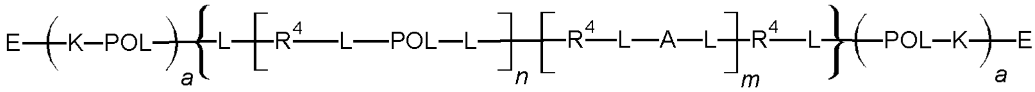

(VIII)

wherein:

- *m* and *n* are each integers independently in the range of 1 to 20;
- *a* is an integer chosen from 0 or 1;
- K is a linking group selected from amide, ester or ether;
- E is a group that can react with another group E initiated or mediated by one or more compounds Y, wherein the one or more compounds Y are selected from the group consisting of oxidant, oxidase, peroxidase and combinations thereof, in aqueous auxiliary formulation (C2), thereby forming one or more covalent bonds between different groups E, or E is a group that can react with one or more cross-linkers Z in aqueous auxiliary formulation (C2), wherein the one or more cross-linkers Z have two or more reactive groups, thereby forming covalent bonds between E and the one or more cross-linkers Z;
- POL is a linear polymeric group having an average molecular weight $M_n$, as determined by end group titration (of its precursor, *i.e.* of $FG^1$-POL-$FG^1$), of about 250 to about 30000 Da;
- A represents moieties selected from the group consisting of Formulas (I-B) to (V-B), wherein A is connected to L via the bonds marked with an asterisk:

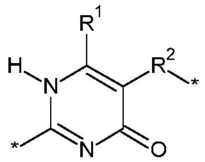

(I-B)

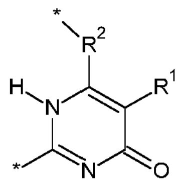

(II-B)

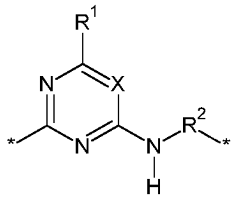

(III-B)

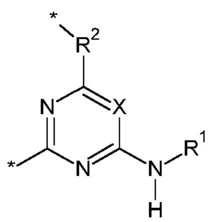

(IV-B)

*-R-* (V-B)

- X is N or $CR^1$;
- each L independently is a urethane or urea linking group, with the proviso that any moiety A according to Formula (I-B) and (II-B) is always coupled to a urea linking group L on the 2-position of the 4-pyrimidone, any moiety A according to Formula (III-B) and (IV-B) is always coupled to a urea linking group L on the 4-position of the 1,3,5-triazine for X=N, or on the 2-position of the pyrimidine for X is $CR^1$, and any moiety A according to Formula (V-B) is

always coupled to two urea linking groups L;

- $R^1$ is selected from the group consisting of hydrogen and $C_1$ - $C_{20}$ alkyl;
- $R^2$ is selected from the group consisting of $C_1$ - $C_{20}$ alkylene;
- $R^3$ is selected from the group consisting of a direct covalent bond, linear $C_1$ - $C_{24}$ alkylene groups, branched $C_2$ - $C_{24}$ alkylene groups, cyclic $C_3$ - $C_{24}$ alkylene groups, $C_6$ - $C_{24}$ arylene groups, $C_7$ - $C_{24}$ alkarylene groups and $C_7$ - $C_{24}$ arylalkylene groups, wherein the alkylene groups, arylene groups, alkarylene groups and arylalkylene groups optionally comprise 1 - 5 heteroatoms selected from the group consisting of O, N and S; and
- $R^4$ is selected from the group consisting of linear or branched $C_2$ - $C_{20}$ alkylene groups and cyclic $C_3$ - $C_{24}$ alkylene groups; and

(b) composition (C2) is an aqueous auxiliary formulation comprising 75.0 - 99.9 wt.% of water, based on the weight of composition (C2), 0 - 5 wt.% of further ingredients and 0.1 - 20.0 wt.%, of:

- one or more dissolved cross-linkers Z having two or more reactive groups that can chemically cross-link the polymer gellant according to Formula (VIII) by forming covalent bonds with two or more groups E; or
- comprising one or more dissolved compounds Y, selected from the group consisting of oxidant, oxidase, peroxidase and combinations thereof, that initiate or mediate chemical cross-linking of two or more groups E on the polymer gellant according to Formula (VIII);

wherein said treatment comprises combining compositions (C1) and (C2) to form a liquid hydrogel composition (C3) and administering said liquid hydrogel formulation (C3) to the synovial fluid or to the cavity of a synovial joint of the vertebrate, to form a cross-linked hydrogel (C4) in the cavity of the synovial joint of the vertebrate.

In a second aspect, the invention concerns a kit of parts, consisting of compositions (C1) and (C2) in separate containers, for use in the treatment or prevention of osteoarthritis in a vertebrate, wherein

(a) composition (C1) is a liquid hydrogel formulation comprising 50.0 - 99.7 wt.% of water, based on the weight of composition (C1), 0.3 - 50.0 wt.% of a polymer gellant according to Formula (VIII) and 0 - 20 wt.% of further ingredients:

$$E\!-\!\left(K\!-\!POL\right)_a\!\left\{L\!-\!\left[R^4\!-\!L\!-\!POL\!-\!L\right]_n\!\left[R^4\!-\!L\!-\!A\!-\!L\right]_m\!R^4\!-\!L\right\}\!\left(POL\!-\!K\right)_a\!-\!E$$

(VIII)

wherein:

- *m* and *n* are each integers independently in the range of 1 to 20;
- *a* is an integer chosen from 0 or 1;
- K is a linking group selected from amide, ester or ether;
- E is a group that can react with another group E initiated or mediated by one or more compounds Y, wherein the one or more compounds Y are selected from the group consisting of oxidant, oxidase, peroxidase and combinations thereof, in aqueous auxiliary formulation (C2), thereby forming one or more covalent bonds between different groups E, or E is a group that can react with one or more cross-linkers Z in aqueous auxiliary formulation (C2), wherein the one or more cross-linkers Z have two or more reactive groups, thereby forming covalent bonds between E and the one or more cross-linkers Z;
- POL is a linear polymeric group having an average molecular weight $M_n$, as determined by end group titration (of its precursor, *i.e.* of $FG^1$-POL-$FG^1$), of about 250 to about 30000 Da;
- A represents moieties selected from the group consisting of Formulas (I-B) to (V-B), wherein A is connected to L via the bonds marked with an asterisk:

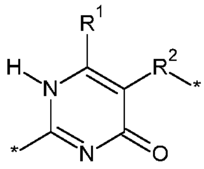

(I-B)

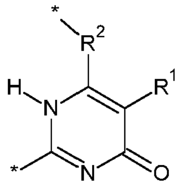

(II-B)

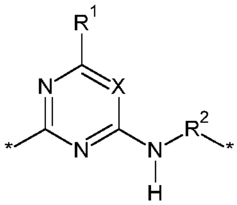

(III-B)

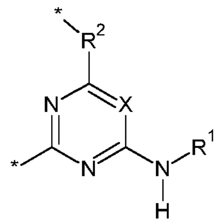

(IV-B)

$*-R^3-*$ (V-B)

- X is N or $CR^1$;
- each L independently is a urethane or urea linking group, with the proviso that any moiety A according to Formula (I-B) and (II-B) is always coupled to a urea linking group L on the 2-position of the 4-pyrimidone, any moiety A according to Formula (III-B) and (IV-B) is always coupled to a urea linking group L on the 4-position of the 1,3,5-triazine for X=N, or on the 2-position of the pyrimidine for X is $CR^1$, and any moiety A according to Formula (V-B) is always coupled to two urea linking groups L;
- $R^1$ is selected from the group consisting of hydrogen and $C_1$ - $C_{20}$ alkyl;
- $R^2$ is selected from the group consisting of $C_1$ - $C_{20}$ alkylene;
- $R^3$ is selected from the group consisting of a direct covalent bond, linear $C_1$ - $C_{24}$ alkylene groups, branched $C_2$ - $C_{24}$ alkylene groups, cyclic $C_3$ - $C_{24}$ alkylene groups, $C_6$ - $C_{24}$ arylene groups, $C_7$ - $C_{24}$ alkarylene groups and $C_7$ - $C_{24}$ arylalkylene groups, wherein the alkylene groups, arylene groups, alkarylene groups and arylalkylene groups optionally comprise 1 - 5 heteroatoms selected from the group consisting of O, N and S; and
- $R^4$ is selected from the group consisting of linear or branched $C_2$ - $C_{20}$ alkylene groups and cyclic $C_3$ - $C_{24}$ alkylene groups; and

(b) composition (C2) is an aqueous auxiliary formulation comprising 75.0 - 99.9 wt.% of water, based on the weight of composition (C2), 0 - 5 wt.% of further ingredients and 0.1 - 20 wt.% of:

- one or more dissolved cross-linkers Z having two or more reactive groups that can chemically cross-link the polymer gellant according to Formula (VIII) by forming covalent bonds with two or more groups E; or
- one or more dissolved compounds Y, selected from the group consisting of oxidant, oxidase, peroxidase and combinations thereof, that initiate or mediate chemical cross-linking of two or more groups E on the polymer gellant according to Formula (VIII);

wherein said treatment comprises in a first step administering one of the compositions (C1) or (C2), preferably composition (C1), to the synovial fluid or to the cavity of a synovial joint of the vertebrate and in a second step administering the other of the compositions (C1) or (C2), to form a liquid hydrogel composition (C3) in the cavity of the synovial joint of the vertebrate that reacts to a cross-linked hydrogel (C4).

As will be appreciated by those skilled in the art, A can also encompass moieties that are tautomers of Formulas (I-B) to (IV-B).

## THE POLYMER GELLANT

The polymer gellant used in the liquid hydrogel formulation ('composition C1') has a structure according to Formula (VIII):

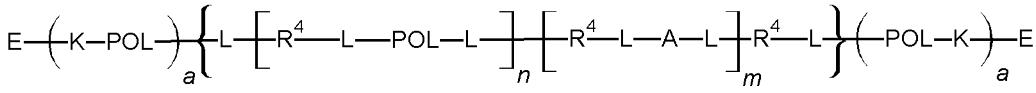

(VIII)

wherein:

- *m* and *n* are each integers independently in the range of 1 to 20;
- *a* is an integer chosen from 0 or 1;
- K is a linking group selected from amide, ester or ether;
- E is a group that can react with another group E initiated or mediated by one or more compounds Y, wherein the one or more compounds Y are selected from the group consisting of oxidant, oxidase, peroxidase and combinations thereof, in aqueous auxiliary formulation (C2), thereby forming one or more covalent bonds between different groups E, or E is a group that can react with one or more cross-linkers Z in aqueous auxiliary formulation (C2), wherein the one or more cross-linkers Z have two or more reactive groups, thereby forming covalent bonds between E and the one or more cross-linkers Z;
- POL is a linear polymeric group having an average molecular weight $M_n$, as determined by end group titration (of its precursor, *i.e.* of $FG^1$-POL-$FG^1$), of about 250 to about 30000 Da;
- A represents moieties selected from the group consisting of Formulas (I-B) to (V-B), wherein A is connected to L via the bonds marked with an asterisk:

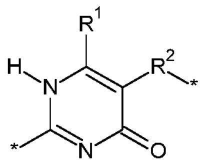

(I-B)

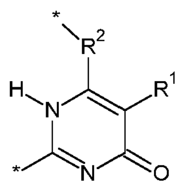

(II-B)

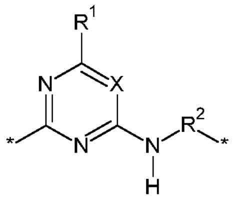

(III-B)

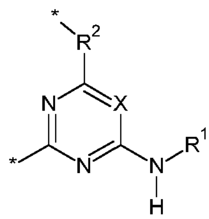

(IV-B)

*-$R^3$-* (V-B)

- X is N or $CR^1$;
- each L independently is a urethane or urea linking group, with the proviso that any moiety A according to Formula (I-B) and (II-B) is always coupled to a urea linking group L on the 2-position of the 4-pyrimidone, any moiety A according to Formula (III-B) and (IV-B) is always coupled to a urea linking group L on the 4-position of the 1,3,5-triazine for X=N, or on the 2-position of the pyrimidine for X is $CR^1$, and any moiety A according to Formula (V-B) is always coupled to two urea linking groups L;
- $R^1$ is selected from the group consisting of hydrogen and $C_1$ - $C_{20}$ alkyl;
- $R^2$ is selected from the group consisting of $C_1$ - $C_{20}$ alkylene;
- $R^3$ is selected from the group consisting of a direct covalent bond, linear $C_1$ - $C_{24}$ alkylene groups, branched $C_2$ - $C_{24}$ alkylene groups, cyclic $C_3$ - $C_{24}$ alkylene groups, $C_6$ - $C_{24}$ arylene groups, $C_7$ - $C_{24}$ alkarylene groups and $C_7$ - $C_{24}$ arylalkylene groups, wherein the alkylene groups, arylene groups, alkarylene groups and arylalkylene groups optionally comprise 1 - 5 heteroatoms selected from the group consisting of O, N and S; and
- $R^4$ is selected from the group consisting of linear or branched $C_2$ - $C_{20}$ alkylene groups and cyclic $C_3$ - $C_{24}$ alkylene

groups.

The following moieties in the polymer gellant having the structure according to Formula (VIII) are randomly distributed along the polymer chain *(i.e.* a statistical polymer as opposed to a block copolymer):

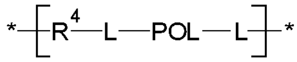 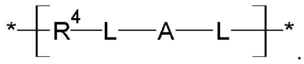

The linear polymeric groups POL in the polymer gellant may comprise any type of polymer backbone known in the art.

The linear polymeric groups POL in the polymer gellant preferably has a number average molecular weight ($M_n$), as determined by end group titration as known in the art, such as hydroxy value determination or standard test method ASTM E222-10, of 600 to 10000 Da, more preferably 1000 to 5000 Da, even more preferably 1100 to 4000 Da, and most preferably 1200 to about 3000 Da.

The polymer gellant preferably has a number average molecular weight ($M_n$), as determined using SEC with a GPC-system equipped with a KD-804 column (Showa Denko, 500 - 400000 Da) using RI detection with DMF comprising 10 mM LiBr as eluent at a flow rate of 1.0 mL/min at 50 °C, with the SEC-data being relative to PEO/PEG-standards, of 1000 - 40000 Da, more preferably 1200 to 20000 Da, even more preferably 2000 to 10000 Da, and most preferably 2000 to 5000 Da.

The linear polymeric groups POL in the polymer gellant can be of natural origin or of synthetic origin. In a preferred embodiment, the linear polymeric groups POL are hydrophilic. Preferably, the linear polymeric groups POL are of synthetic origin. If the linear polymeric group POL is hydrophilic and of natural origin, it is preferably selected from the group consisting of proteins (e.g. proteins selected from the group consisting of collagen, gelatin, and fibrin); polysaccharides (e.g. polysaccharides selected from the group consisting of hyaluronic acid, dextran, agar, agarose, xantham gums, natural gum, alginate, chitosan and inulin) and synthetic derivatives of these polymers of natural origin, preferably gelatin or chitosan.

If the linear polymeric group POL is of synthetic origin, it can be any synthetic linear polymeric group, preferably having a solubility in water of at least 1 g/L at 20°C, more preferably at least 10 g/L at 20 °C.

The linear polymeric group POL is preferably selected from the group consisting of polyethers, polyesters, polycarbonates, polyamides, polyoxazolines, polyacrylates, polymethacrylates, polyolefins, hydrogenated polyolefins, polysiloxanes, polycarbonates, (per)fluorinated polyethers, polyvinylenes, or co-polymers of such polymers, and combinations thereof. More preferably, the linear polymeric group POL is a polyether, polyester, polycarbonate, polyamide, polyoxazoline, polyacrylate, polymethacrylate, or a co-polymer of such polymers and combinations thereof. Even more preferred are polyethers, polyesters, polycarbonates, polyoxazolines or copolymers thereof. Very preferably, the linear polymeric group POL is selected from polyethylene glycols and polyoxazolines, most preferably from polyoxazolines.

Although some of the above listed linear polymeric groups POL themselves may not be hydrophilic per se, copolymerizing them with the right amount of water-soluble linear polymeric groups POL, or use of a combination of these linear polymeric groups POL, may lead to hydrophilic character.

The number of repeating units *m* preferably is not higher than 3, most preferably not higher than 1.

The number of repeating units *n* preferably is at least 2 and not higher than 10, most preferably at least 2 and not higher than 6.

In a preferred embodiment, moiety A in Formula (VIII) is represented by Formula (I-B), in which $R^1$ is selected from the group consisting of hydrogen and $C_1$ - $C_{20}$ alkyl, preferably methyl, and $R^2$ is selected from the group consisting of $C_1$ - $C_{20}$ alkylene, preferably, ethylene. Most preferably, moiety A in Formula (VIII) is:

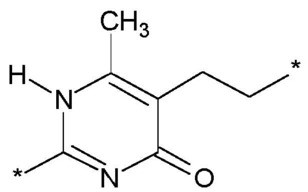

and *-L-A-L-* is:

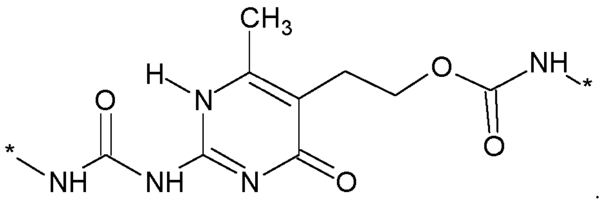

In another preferred embodiment, moiety A in Formula (VIII) is represented by Formula (V-B) and wherein *-L-A-L-* is:

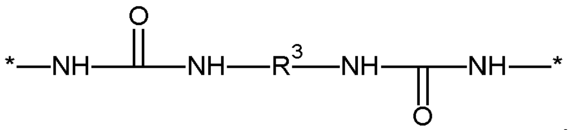

,

in which $R^3$ is selected from the group consisting of a covalent bond, linear $C_2$ - $C_{12}$ alkylene groups and cyclic $C_3$ - $C_{12}$ alkylene groups, more preferably from a covalent bond and linear $C_4$ - $C_{10}$ alkylene groups.

$R^4$ is preferably selected from the group consisting of branched and/or cyclic $C_3$ - $C_{24}$ alkylene groups, more preferably from 3-(2-ethylene)-3-methyl-5,5-dimethyl-cyclohexylene and 4,4'-dicyclohexylenemethane. In another preferred embodiment, *- L-$R^4$-L-* is selected from:

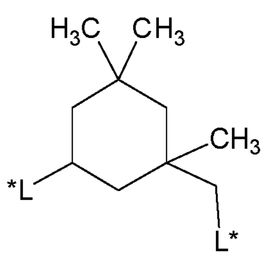

or

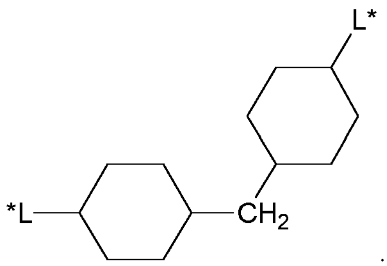

.

In another preferred embodiment, $R^4$ is selected from the group consisting of linear $C_2$ - $C_{20}$ alkylene groups, more preferably $R^4$ is 1,6-*n*-hexylene. The inventors have established that cross-linked hydrogels (C4) wherein $R^4$ is selected from the group consisting of linear $C_2$ - $C_{20}$ alkylene groups, wherein $R^4$ preferably is 1,6-*n*-hexylene, maintain their mechanical and chemico-physical characteristics for a longer period of time, *i.e.* biodegradation takes longer.

In a preferred embodiment, the group E is a group that can react with another group E forming one or more covalent bonds between different groups E in the presence of one or more compounds Y, wherein the one or more compounds Y are selected from the group consisting of oxidant, oxidase, peroxidase and combinations thereof, that mediate or initiate the reaction. Preferably, this reaction occurs via an oxidative pathway mediated by an oxidant. E is preferably a group selected from the group consisting of hydroxy aryl, multihydroxy aryl, thiol, dithioether -S-S-$R^9$ wherein $R^9$ is a $C_1$ - $C_6$ alkyl or alanyl, $C_2$ - $C_6$ alkynyl, $C_2$ - $C_6$ alkenyl, methacrylate and acrylate. An example of a dithioether group is dithiocysteine. More preferably, E comprises moieties selected from hydroxyaryl or multihydroxy aryl, even more preferably dihydroxyaryl, most preferably 3,4-dihydroxyphenyl.

Group E preferably has a molecular weight of about 100 to about 1000 Da, more preferably of about 120 to about 500 Da, even more preferably of about 120 to about 300 Da, and most preferably of about 130 to about 250 Da.

Most preferably:

$$*—L\left(—POL—K—\right)_a—E$$

in the polymer gellant of Formula (VIII) is (with *a* = 0):

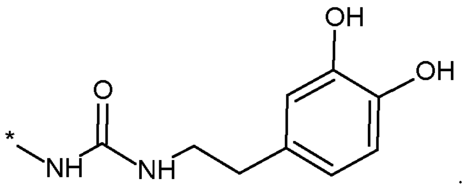

**PREPARATION OF THE POLYMER GELLANT**

The polymer gellant (VIII) can be prepared by any method known in the art, executed in the bulk or in solution. In an embodiment, the polymer gellant (VIII) is obtainable by a process wherein:

(i) one or more compounds A' selected from the group consisting of Formulas (I-A) to (V-A):

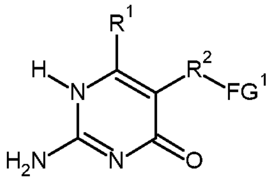

(I-A)

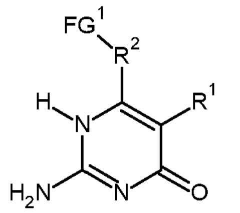

(II-A)

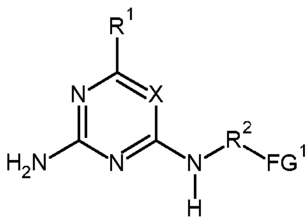

(III-A)

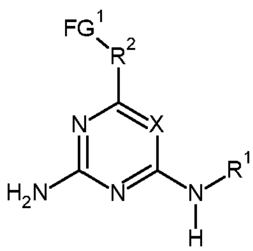

(IV-A)

$H_2N\text{-}R^3\text{-}NH_2$ (V-A)

are reacted, optionally in a solvent, with a diisocyanate compound according to Formula $OCN\text{-}R^4\text{-}NCO$ and a polymer according to Formula $FG^1\text{-}POL\text{-}FG^1$ to form an intermediate diisocyanate product with Formula (VI):

$$OCN-\left[R^4-L-POL-L\right]_n\left[R^4-L-A-L\right]_m R^4-NCO$$

(VI)

wherein:

- *m, n,* X, $R^1$, $R^2$, $R^3$, $R^4$, POL, A, and L are as defined hereinbefore;
- each $FG^1$ is a functional group independently selected from OH and $N(R^1)H$;

(ii) reacting the intermediate diisocyanate product of Formula (VI) with a compound according to formula (VII):

$$FG^1\!\left(-POL-K-\right)_a\!-E$$

(VII)

wherein *a*, K and E are as defined hereinbefore, to provide the polymer gellant according to Formula (VIII).

As will be appreciated by those skilled in the art, A' can also encompass moieties that are tautomers of Formulas (I-A) to (IV-A).

The following moieties in the intermediate diisocyanate product with Formula (VI) are randomly distributed along the polymer chain (*i.e.* a statistical polymer as opposed to a block copolymer):

$$*\!\left[R^4-L-POL-L\right]\!*\quad *\!\left[R^4-L-A-L\right]\!*.$$

The polymer according to Formula $FG^1$-POL-$FG^1$ preferably is bifunctional (telechelic), but small deviations from this bifunctionality are also encompassed by the invention.

In preferred embodiments, the polymer according to Formula $FG^1$-POL-$FG^1$ has about 1.8 to about 2 end-groups $FG^1$, preferably about 1.9 to about 2 end groups $FG^1$, most preferably higher than about 1.95 to about 2 end groups $FG^1$. Most preferably, $FG^1$ is OH and the hydroxyl value of HO-POL-OH is in between 26 and 115, even more preferably in between 35 and 60.

In a preferred embodiment moiety A' is represented by Formula (I-A), in which $R^1$ is selected from the group consisting of hydrogen and $C_1$ - $C_{20}$ alkyl, preferably methyl, and $R^2$ is selected from the group consisting of $C_1$ - $C_{20}$ alkylene, preferably ethylene. Most preferably, moiety A' is:

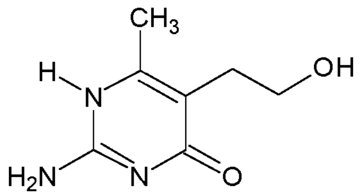

In another preferred embodiment, moiety A' is represented by Formula (V-A), in which $R^3$ is selected from the group consisting of a covalent bond, linear $C_2$ - $C_{12}$ alkylene groups and cyclic $C_3$ - $C_{12}$ alkylene groups.

In a very preferred embodiment, the compound according to Formula (VII) is dopamine.

Reaction solvents are preferably aprotic solvents known in the art, such as ethers, such as diethyl ether, THF, methyl-tetrahydrofuran, dioxane and methyl-tert-butyl ether, DMSO, dimethyl acetamide, DMF, NMP, trialkyl amines, chloroform, dichloromethane, diethylcarbonate, propylene carbonate, ketones such as acetone, MEK and methyl-tert-butyl ketone, esters such as ethyl acetate and butyl acetate, and toluene.

In a typical reaction, the required amounts of the one or more compounds A', the polymer according to Formula $FG^1$-POL-$FG^1$ and the diisocyanate compound according to Formula OCN-$R^4$-NCO are mixed at once in a 1-pot or semi 1-pot reaction in which the compound according to formula (VII) is added in a second stage.

Preferably, the reactants and solvents are dried, *i.e.* they contain no or little water. Preferably, the reaction is executed under an inert atmosphere of nitrogen or argon.

The polymer gellant (VIII) can be isolated by different types of work-up procedures that are known in the art, such as precipitation, extraction, washing, drying, freeze drying, spray drying, filtration and evaporation procedures, or combinations thereof.

Preferably, the reaction is performed with less than about 40% by weight of solvent, more preferably less than about 20% by weight of solvent, most preferably without solvent, at temperatures in between about 20°C and 150°C, possibly by using an (twin screw) extruder, kneader or mixer.

**THE LIQUID HYDROGEL FORMULATION ('COMPOSITION C1')**

The liquid hydrogel formulation ('composition C1') comprises the polymer gellant (VIII) dissolved in water, with optional further ingredients. The liquid hydrogel formulation preferably comprises 5 - 30 wt.% of the polymer gellant (VIII), more preferably 10 - 25 wt.%, even more preferably 11 - 23 wt.%, and most preferably 12 - 21 wt.%, based on the weight of the liquid hydrogel formulation ('composition C1').

The liquid hydrogel formulation preferably comprises 70 - 95 wt.% of water, more preferably 75 - 90 wt.%, most preferably 78 - 87 wt.%, based on the weight of the liquid hydrogel formulation.

The liquid hydrogel formulation preferably comprises 0.0005 - 19 wt.% of further ingredients, more preferably 0.01 - 18 wt.%, even more preferably 0.02 - 15 wt.%, most preferably 0.025 - 5 wt.%, based on the weight of the liquid hydrogel formulation.

In another embodiment, the hydrogel formulation comprises 0.0005 to 10 wt.% of further ingredients, preferably 0.0005 to 1 wt.%, and most preferably 0.005 to 1 wt.%, based on the weight of the liquid hydrogel formulation.

The optional further ingredients are functional ingredients that will contribute to the specific use of the hydrogel. Preferably, the liquid hydrogel formulation contains a pH-buffer known in the art, such as a PBS or borate buffer as a further ingredient. Preferably, the pH of the liquid hydrogel formulation is between about 3 and about 11, more preferably between about 3 and about 6, most preferably between about 4 and about 5.

In another preferred embodiment, the liquid hydrogel formulation comprises as a further ingredient an additional polymer in order to modify the rheological properties of the liquid hydrogel formulation. This additional polymer preferably is hydrophilic and may represent any type of polymer backbone known in the art, preferably polyethers, polyesters, polyamides, polyoxazolines, polyamines, polyacrylates, polymethacrylates, polyolefins, hydrogenated polyolefins, polysiloxanes, polycarbonates, (per)fluorinated polyethers, polyvinylenes, or co-polymers of such polymers. More preferably, the polymer backbone is a polyether, polyester, polyacrylate, polymethacrylate, polyolefin, hydrogenated polyolefin, polycarbonate, polyvinylene, or a co-polymer of such polymers. Even more preferred are polyethers, polyesters, or copolymers thereof. Most preferably, this additional polymer is a polyether, preferably a polyglycol, preferably a polyethylene glycol or a poly ethylene-co-propylene glycol (random or block), most preferably a polyethylene glycol. Preferably, the additional polymer comprises at least one moiety A as defined hereinbefore in the context of the polymer gellant according to Formula (VIII).

The liquid hydrogel formulation may comprise as further ingredients one or more selected from a solid filler, a diluent, a thickener, a carrier, and other excipients known in the art. A non-limited list of further ingredients includes (fluorescent) dyes, contrast agents, sugars, starches, cellulose and its derivatives, gelatin, talc, clays, waxes (natural and synthetic), oils from natural origin, fatty acids and their esters, and ionic additives. Preferably, clay particles are added as further ingredient to improve the hydrogel properties. Clay particles may be selected from laponite and bentonite, optionally in the presence of (ionic) compatibilizers known in the art.

In another embodiment, the liquid hydrogel formulation comprises as further ingredients one or more biologically active and/or pharmaceutically active compounds. The inventors have found that the cross-linked hydrogel ('composition C4') can act as a carrier material for biologically active and/or pharmaceutically active compounds that can provide controlled or prolonged release of the biologically active and/or pharmaceutically active compounds to the environment. A biologically active or pharmaceutically active compound, as used herein, includes a compound which provides a therapeutic, diagnostic or prophylactic effect, a compound that affects or participates in tissue growth, cell growth, cell differentiation, a compound that may be able to invoke a biological action such as an immune response, or a compound that could play any other role in one or more biological processes. Such compounds, peptide or non-peptide, protein or non-protein, include but are not limited to bone morphogenetic proteins, antimicrobial agents (including antibacterial, hemotherapeutic and anti-fungal agents), anti-viral agents, anti-tumor agents, hormones, hormone antagonistics, corticosteroids such as mineralocorticosteroids or glucocorticosteroids, androgents, estrogens, progestins immunogenic agents, anti-inflammatory agents, anti-gout agents, centrally acting analgesics, local anesthetics, centrally active muscle relaxants, paracrine factors, growth factors, nucleic acids, DNA-derivatives, RNA-derivatives, lipids, lipopolysaccharides, (poly)saccharides, vitamins, and peptides, polypeptides and proteins in general, preferably include but are not limited to bone morphogenetic proteins, antimicrobial agents (including antibacterial, hemotherapeutic and anti-fungal agents), anti-viral agents, anti-tumor agents, hormones, hormone antagonistics, corticosteroids such as mineralocorticosteroids or glucocorticosteroids, androgents, estrogens, progestins immunogenic agents, anti-inflammatory agents, anti-gout agents, centrally acting analgesics, local anesthetics, centrally active muscle relaxants, growth factors, nucleic acids, DNA-derivatives, RNA-derivatives, lipids, lipopolysaccharides, (poly)saccharides, vitamins, and peptides, polypeptides and proteins in general, more preferably, bone morphogenetic proteins and TGF-$\beta$. The biologically active compounds preferably comprise at least one 4H-unit per molecule up to a maximum of ten 4H-units, preferably one to four, and most preferably two to four 4H-units. These 4H-units are preferably covalently attached to the biologically active compound.

The liquid hydrogel formulation preferably comprises 0.0005 to 10 wt.% of one or more biologically active and/or pharmaceutically active compounds, such as the one or more biologically active and/or pharmaceutically active compounds as defined hereinbefore, more preferably 0.0005 to 1 wt.%, and most preferably 0.005 to 1 wt.%, based on the weight of the liquid hydrogel formulation.

In a less preferred embodiment, the liquid hydrogel formulation comprises as further ingredient a bioactive species selected from the group consisting of a living cell, an enzyme, or a micro-organism. A living cell in this embodiment means and includes individual animal and plant cells, cell clusters, tissues, organs and organisms, including organisms such as bacteria, fungi or moulds.

Preferably, the liquid hydrogel formulation comprises one or more further ingredients, wherein said one or more further ingredients are selected from the group consisting of anti-inflammatory drugs, corticosteroids, local anesthetics and combinations thereof.

In a preferred embodiment, the liquid hydrogel formulation comprises one or more further ingredients, wherein said one or more further ingredients are selected from the group consisting of peptides, proteins, antimicrobial agents, anti-viral agents, hormones, paracrine factors, growth factors, vitamins, anti-inflammatory drugs, corticosteroids, local anesthetics and combinations thereof, preferably in an amount of 0.0005 to 10 wt.%, based on the weight of the liquid hydrogel formulation, more preferably 0.0005 to 1 wt.%, and most preferably 0.005 to 1 wt.%.

Preferably, the liquid hydrogel formulation comprises one or more further ingredients which provide a therapeutic, diagnostic or prophylactic effect, or stimulate cell growth or differentiation, wherein said one or more further ingredients are selected from the group consisting of peptides, proteins, antimicrobial agents, anti-viral agents, hormones, paracrine factors, growth factors, vitamins, anti-inflammatory drugs, corticosteroids, local anesthetics and combinations thereof, preferably in an amount of 0.0005 to 10 wt.%, based on the weight of the liquid hydrogel formulation, more preferably 0.0005 to 1 wt.%, and most preferably 0.005 to 1 wt.%.

Preferred examples of anti-inflammatory drugs include non-steroidal anti-inflammatory drugs. Preferred examples of corticosteroids include glucocorticosteroids. Preferred examples of local anesthetics include local anesthetics with an amide group, such as lidocaine, bupivacaine and levobupivacaine.

Apart from the previous list, it is also possible to load the liquid hydrogel formulation with inorganic compounds as further ingredients, such as reactive oxygen scavengers and bone-extracts, such as apatite.

It is possible within the scope of the present invention to incorporate drugs of a polymeric nature, but also to incorporate drugs or vitamins of a relatively small molecular weight of less than about 1500 Da, or even less than about 500 Da.

In a preferred embodiment, the liquid hydrogel formulation ('composition C1') does not comprise:

- ingredients derived from animal or human tissue; and/or
- cells, cell extracts, proteins, glycosaminoglycans and biologically derived macromolecules; and/or
- stem cells and growth factors.

More preferably, the liquid hydrogel formulation ('composition C1') does not contain cells, lyophilized cell matrices, mammalian cell extracts, most preferably the liquid hydrogel formulation does not contain cells, lyophilized cell matrices, and platelet lysate.

The viscosity of the liquid hydrogel formulation ('composition C1') is low enough to allow administration of the formulation by a syringe or catheter. Without wishing to be bound by any theory, it is believed that the liquid hydrogel formulation needs a minimum viscosity in order to be able to fill the synovial joint satisfactorily.

The liquid hydrogel formulation ('composition C1') preferably has a loss factor (tan $\delta$) at 1 Hz and 20°C between 1.0 and 30000, more preferably in between 2.0 and 100, most preferably the loss factor is in between 2.0 and 10, as determined with a plate-plate rheometer with a gap distance of 0.50 mm.

In another preferred embodiment, the dynamic viscosity of the liquid hydrogel formulation ('composition C1') at 37°C is about 0.01 to about 20 Pa·s, more preferably about 0.1 to about 2.0 Pa·s, most preferably about 0.2 to about 1.2 Pa·s, as measured with a rheometer with a plate-plate geometry at a shear rate of 1 $s^{-1}$ and with a gap distance of 0.50 mm.

## THE AQUEOUS AUXILIARY FORMULATION ('COMPOSITION C2')

The aqueous auxiliary formulation ('composition C2') is added to the liquid hydrogel formulation ('composition C1') to start the chemical cross-linking reaction, *i.e.* to start the formation of the cross-linked hydrogel ('composition C4'). The aqueous auxiliary formulation comprises:

- one or more dissolved cross-linkers Z having two or more reactive groups that can chemically cross-link the polymer gellant according to Formula (VIII) in the liquid hydrogel formulation ('composition C1') by forming covalent bonds with two or more groups E; or
- one or more dissolved compounds Y, selected from the group consisting of oxidant, oxidase, peroxidase and combinations thereof, that initiate or mediate chemical cross-linking of two or more groups E on the polymer gellant according to Formula (VIII) in the liquid hydrogel formulation ('composition C1').

Since compound Y only initiates or mediates chemical cross-linking, it is not part of the cross-linked hydrogel structure ('composition C4'). A typical molar ratio of compound Y to E is between 0.01 to 2.0, preferably between 0.4 to 1.2, more preferably between 0.5 to 0.8. Preferably, compound Y is of synthetic origin and does not comprise enzymes, such as peroxidases.

In a first preferred embodiment, compound Y is an oxidant selected from:

- alkali metal periodates (wherein the alkali metal is lithium, sodium, potassium, rubidium or cesium);
- alkaline earth metal periodates (wherein the alkaline earth metal is magnesium, calcium or barium);
- hydrogen peroxide;
- organic peroxides ($R^{10}$-O-O-$R^{10}$), hydroperoxides ($R^{10}$-O-O-H) and peroxycarboxylic acids ($R^{10}$-C(O)-O-O-H), wherein $R^{10}$ is independently selected from the group of linear $C_1$ - $C_{12}$ alkyl groups, branched or cyclic $C_3$ - $C_{12}$ alkyl groups, $C_6$ - $C_{12}$ aryl groups, $C_7$ - $C_{14}$ alkaryl groups, $C_7$ - $C_{14}$ arylalkyl groups, and mixtures thereof;
- salts of a transition metal (wherein the transition metal is selected from the elements defined by Groups 3 to 12 and Period 4 to 6 of the Periodic Table and wherein the salt anion is preferably chloride;
- oxygen; and
- inorganic bases (preferably hydroxides from the alkali metals and the alkali earth metals defined above).

More preferably compound Y is only one species, even more preferably compound Y is sodium periodate, potassium periodate, hydrogen peroxide, iron(III)-salt, or iron(II)-salt, most preferably compound Y is sodium periodate or potassium periodate.

In a second preferred embodiment, the aqueous auxiliary formulation ('composition C2') comprises two different compounds Y, namely (i) an oxidant and (ii) a peroxidase or oxidase, wherein the peroxidase is preferably selected from horseradish peroxidase, cytochrome c peroxidase, glutathione peroxidase, mushroom tyrosinase and catalase, wherein the oxidase is preferably selected from glucose oxidase and cytochrome P450 oxidase, and wherein the oxidant preferably is hydrogen peroxide. In a very preferred embodiment, the aqueous auxiliary formulation ('composition C2') comprises two different compounds Y, wherein the two different compounds Y are hydrogen peroxide and horseradish peroxidase.

In a third preferred embodiment, the aqueous auxiliary formulation ('composition C2') comprises a cross-linker Z having two or more reactive groups that can chemically cross-link the polymer gellant according to Formula (VIII) in the liquid hydrogel formulation ('composition C1') by forming covalent bonds with two or more groups E, wherein the cross-linker Z is preferably chosen from boronic acids according to the formula $R^{10}$-$B(OH)_2$, wherein $R^{10}$ is as defined hereinbefore.

The aqueous auxiliary formulation ('composition C2') preferably has a pH of between about 3 and about 11, more preferably between about 7 and about 11, most preferably between about 8 and about 10.

The aqueous auxiliary formulation preferably comprises 80 - 99.5 wt.% of water, more preferably 85 - 99 wt.%, even more preferably 90 - 98 wt.%, based on the weight of the aqueous auxiliary formulation.

The concentration of the one or more compounds Y or the one or more compounds Z in the aqueous auxiliary formulation ('composition C2') is preferably in the range of 0.1 - 15 wt.%, more preferably 0.1 - 10 wt.%, even more preferably 0.2 - 8 wt.%, based on the weight of the aqueous auxiliary formulation.

The aqueous auxiliary formulation ('composition C2') preferably comprises 0 - 4 wt.% of further ingredients, more preferably 0 - 2 wt.%, even more preferably 0 - 1.5 wt.%, based on the weight of the aqueous auxiliary formulation.

In a preferred embodiment, the aqueous auxiliary formulation ('composition C2') does not comprise:

- ingredients derived from animal or human tissue; and/or
- cells, cell extracts, proteins, glycosaminoglycans and biologically derived macromolecules; and/or
- stem cells and growth factors.

In very preferred embodiments, the only further ingredients that are added to the aqueous auxiliary formulation are additives to establish the pH, such as buffering agents.

## THE LIQUID HYDROGEL COMPOSITION ('COMPOSITION C3')

The liquid hydrogel composition ('composition C3') is prepared by adding, preferably by mixing, liquid hydrogel formulation ('composition C1') and aqueous auxiliary formulation ('composition C2').

In a preferred embodiment, the liquid hydrogel formulation ('composition C1') and aqueous auxiliary formulation ('composition C2') are added, preferably mixed, in a weight ratio ranging from 1:20 to 1:30 of the aqueous auxiliary formulation to the liquid hydrogel formulation.

As will be appreciated by those skilled in the art, the liquid hydrogel formulation ('composition C1') and the aqueous auxiliary formulation ('composition C2') are preferably added or mixed in a weight ratio that results in the intended molar ratio of one or more compounds Y to E or in the intended molar ratio of the one or more cross-linkers Z to E in the resulting liquid hydrogel composition ('composition C3').

In a preferred embodiment, the amount of water in the liquid hydrogel composition ('composition C3') is between

51.0 and 99.9 wt.% by weight of the liquid hydrogel composition, the amount of the polymer gellant according to Formula (VIII) is between 0.1 - 49.0 wt.% by weight of the liquid hydrogel composition, and the amount of the further ingredients is between 0 and 19 wt.% by weight of the liquid hydrogel composition.

In another preferred embodiment, the amount of water in the liquid hydrogel composition ('composition C3') is between 80.0 and 99.9 wt.% by weight of the liquid hydrogel composition, the amount of the polymer gellant according to Formula (VIII) is between 0.1 and 20.0 wt.% by weight of the liquid hydrogel composition, and the amount of the further ingredients is between 0 and 2 wt.% by weight of the liquid hydrogel composition.

In a very preferred embodiment, the aqueous auxiliary formulation ('composition C2') has a pH of between about 8 and about 10 and the liquid hydrogel formulation ('composition C1') has a pH of between about 3 and about 6.5 Without wishing to be bound by any theory, the inventors believe that mixing both formulations with opposite pH's results in superior properties of the cross-linked hydrogel ('composition C4'). Additionally, a low pH for the liquid hydrogel formulation ('composition C1') will decrease the occurrence of its oxidative degradation thereby effectively serving as a preservative. The pH's of the liquid hydrogel formulation ('composition C1') and the aqueous auxiliary formulation ('composition C2') can also be used to tune the gelation time of the liquid hydrogel composition ('composition C3'), whereby a lower pH of the liquid hydrogel formulation ('composition C1') will result in slower gelation.

## THE CROSS-LINKED HYDROGEL ('COMPOSITION C4')

The composition of the cross-linked hydrogel ('composition C4') is similar to that of the liquid hydrogel composition ('composition C3'), provided that the water-gellant is in a cross-linked state and that the cross-linked hydrogel ('composition C4') can mix with synovial fluid that may be present in the cavity of the synovial joint.

Hence, in an embodiment the cross-linked hydrogel ('composition C4') comprises ingredients coming from the synovial fluid in the joint of the vertebrate, such as hyaluronic acid, lubricin, proteinases, collagenases and phagocytic cells, with the proviso that these ingredients were already present in the synovial fluid of the vertebrate before administering the formulations of the invention (*i.e.* (i) the liquid hydrogel composition ('composition C3') or (ii) the liquid hydrogel formulation ('composition C1') and the aqueous auxiliary formulation ('composition C2')). Preferably, the cross-linked hydrogel ('composition C4') comprises the vertebrate's lubricin.

In a preferred embodiment, the amount of water in the cross-linked hydrogel ('composition C4') is between 51.0 and 99.9 wt.% by weight of the cross-linked hydrogel, the amount of the polymer gellant according to formula (VIII) is between 0.1 - 49.0 wt.% by weight of the cross-linked hydrogel, and the amount of the further ingredients is between 0 and 19 wt.% by weight of the cross-linked hydrogel.

In another preferred embodiment, the amount of water in the cross-linked hydrogel ('composition C4') is between 80.0 and 99.9 wt.% by weight of the cross-linked hydrogel, the amount of the polymer gellant according to Formula (VIII) is between 0.1 and 20.0 wt.% by weight of the cross-linked hydrogel, and the amount of the further ingredients is between 0 and 2 wt.% by weight of the cross-linked hydrogel.

The cross-linked hydrogel ('composition C4') comprises covalent (irreversible) and reversible cross-links. These reversible cross-links make the hydrogel visco-elastic, meaning that the hydrogel exhibits a combination of properties of elastic solids and viscous liquids. Visco-elastic materials dissipate energy due to deformation and display a time-dependent mechanical response, while purely elastic materials will store energy. Specifically, visco-elastic materials exhibit stress relaxation in response to a deformation, such as compression. Other characteristics of visco-elastic materials are hysteresis in stress-strain curves obtained after loading and unloading of the material in which the area between the loading and unloading curves represents the energy dissipated, and frequency dependent values for the storage modulus (G'), loss modulus (G") and loss factor, as obtained with dynamic mechanical testing. Accordingly, the cross-linked hydrogel ('composition C4') is not a static three-dimensional object, but its reversible cross-links allow the cross-linked hydrogel to respond to deformations in a time-dependent manner. Since the extracellular matrix and tissues are visco-elastic and since cells are surrounded by and interact with this extracellular matrix, the visco-elastic nature of the cross-linked hydrogel ('composition C4') enhances cartilage tissue development and thereby contributes to the treatment and prevention of osteoarthritis.

The reversible cross-links in the cross-linked hydrogel ('composition C4') are preferably based on hydrogen bonding interactions, more preferably on ureidopyrimidone hydrogen bonding pairs incorporated in the polymer backbone such as *-L-A-L-*.

The cross-linked hydrogel ('composition C4') has a high number of covalent cross-links. Preferably, the average molecular weight per cross-link is lower than about 2500 Da, more preferably lower than about 1800 Da, most preferably lower than about 1200 Da, whereby the average molecular weight per cross-link is defined as the number average molecular weight ($M_n$) of the linear polymer group POL, as determined from the hydroxyl number using end group titration of its precursor $FG^1$-POL-$FG^1$, divided by the total number of groups E per molecule of the polymer gellant according to Formula (VIII) present in the liquid hydrogel formulation ('composition C1'), *i.e.* before cross-linking.

The cross-linked hydrogel ('composition C4') is preferably not adhesive to cartilage and bone.

The cross-linked hydrogel ('composition C4') preferably shows only a limited amount of swelling due to water absorption. The increase in weight of the cross-linked hydrogel ('composition C4') due to absorption of water after 1 day at 37°C in PBS is preferably less than about 300%, more preferably less than about 150%, most preferably less than about 100%, based on the original weight.

The cross-linked hydrogel ('composition C4') preferably keeps its structural integrity and will not dissolve or fall apart into small fragments upon exposure to an excess PBS-buffered water after 1 day at 37°C.

The cross-linked hydrogel ('composition C4') has a cushioning effect in the cavity of the synovial joint. Accordingly, the cross-linked hydrogel ('composition C4'), after 16 h of curing, preferably has a storage modulus (G') at 1 Hz and 37 °C of greater than 1 kPa, preferably greater than 7 kPa, most preferably greater than 10 kPa. The cross-linked hydrogel ('composition C4'), after 16 h of curing, preferably has a loss factor at 1 Hz and 37 °C of smaller than 0.2, more preferably smaller than 0.14, most preferably smaller than 0.07.

Preferably, the cross-linked hydrogel ('composition C4'), after 16 h of curing, has a loss modulus (G") at 1 Hz and 37 °C of greater than 0.01 kPa, more preferably greater than 0.05 kPa, most preferably greater than 0.1 kPa.

Preferably, the cross-linked hydrogel ('composition C4'), after 16 h of curing, has a loss factor at 1 Hz and 37 °C of larger than 0.001, more preferably larger than 0.003, most preferably larger than 0.005.

The cross-linked hydrogels ('composition C4') according to the present invention are, after 16 h of curing, preferably elastic and preferably have an elongation at break at 20 °C greater than about 100%, more preferably greater than about 150%, more preferably greater than about 200%, as determined by test method ASTM D 1708-96 with a crosshead speed of 50 mm/min at 20 °C.

Preferably, the cross-linked hydrogels ('composition C4'), after 16 h of curing, have a Young's modulus at 20°C between about 1 kPa to about 1 MPa, more preferably between about 10 kPa to about 100 kPa, and most preferably between about 15 kPa to about 50 kPa, as determined by test method ASTM D 1708-96 with a crosshead speed of 50 mm/min at 20 °C.

In another preferred embodiment of this invention, the cross-linked hydrogels ('composition C4'), after 16 h of curing, can withstand high compression forces. Preferably, the cross-linked hydrogels ('composition C4') can withstand a compression at 37 °C without failure of at least 1.0 MPa, more preferably of at least 2.0 MPa, even more preferably of at least 4.0 MPa and most preferably of at least 5.0 MPa. Failure in this application means the presence of cracks in the cross-linked hydrogel as visible by the naked eye or fractionation of the intact cross-linked hydrogel test sample in two or more fragments.

Preferably, the cross-linked hydrogel ('composition C4'), after 16 h of curing, can be compressed at 37 °C without failure to 10% of its original thickness, more preferably to 5% of its original thickness, most preferably to 3% of its original thickness.

After compression for 10 seconds at 37 °C to 10% of its original thickness, the cross-linked hydrogel ('composition C4') that has cured 16 h, preferably regains 90% of its original thickness within 30 minutes after the compression force is removed, more preferably within 10 minutes, and most preferably within 2 minutes.

Preferably, the cross-linked hydrogel ('composition C4'), after 16 h of curing, exerts a compressive stress of at least 0.4 MPa, more preferably of at least 0.6 MPa, even more preferably of at least 1.0 MPa and most preferably of at least 1.1 MPa, after 30 seconds stress relaxation at 95% strain in unconfined compression at 37 °C.

Preferably, the cross-linked hydrogel ('composition C4'), after 16 h of curing, exerts a stress relaxation after 60 seconds to a stress level of at most 95% of the maximum stress, more preferably at most 90% of the maximum stress after unconfined compression at 37 °C after a maximum stress of 1 MPa, preferably 2 MPa, most preferably 3 MPa.

Preferably, the cross-linked hydrogel ('composition C4'), after 16 h of curing, exerts an equilibrium modulus of at least 0.4 MPa, more preferably of at least 0.6 MPa, even more preferably of at least 0.8 MPa and most preferably of at least 1.4 MPa, as extracted from the linear region of the stress-strain curves in confined compression at 37 °C.

Preferably, the cross-linked hydrogel ('composition C4'), after 16 h of curing, exerts a maximum stress of at least 0.4 MPa, more preferably of at least 0.6 MPa, even more preferably of at least 0.8 MPa and most preferably of at least 1.0 MPa, at 25% strain in confined compression at 37 °C.

Preferably, the cross-linked hydrogel ('composition C4'), after 16 h of curing, exerts an equilibrium stress of at least 0.10 MPa, more preferably of at least 0.15 MPa, even more preferably of at least 0.20 MPa and most preferably of at least 0.30 MPa, at 25% strain in confined compression at 37 °C.

Preferably, the cross-linked hydrogel ('composition C4'), after 16 h of curing, exerts a maximum modulus of at least 1.0 MPa, more preferably of at least 2.5 MPa, even more preferably of at least 3.0 MPa and most preferably of at least 4.0 MPa, as extracted from the linear region of the stress-strain curves in confined compression at 37 °C.

The cross-linked hydrogels ('composition C4'), after 16 h of curing, show good durability towards cyclic deformation. Preferably they do not fail within 60.000 cycles in a diurnal loading regime at 37 °C in PBS whereby the diurnal loading regime consists of 50.000 cycles to 80% strain at 5 Hz followed by 10.000 cycles to 10% strain at 5 Hz, more preferably they do not fail within 120.000 cycles and most preferably they do not fail within 300.000 cycles.

In a preferred embodiment, the cross-linked hydrogel ('composition C4'), after 16 h of curing, has at least one of

the following properties (i) to (vii):

i) a Young's modulus in between 1 and 1000 kPa, as determined by test method ASTM D 1708-96 with a crosshead speed of 50 mm/min at 20 °C;
ii) an elongation at break of at least 100%, as determined by test method ASTM D 1708-96 with a crosshead speed of 50 mm /min at 20 °C;
iii) no failure at an unconfined compression of 4.0 MPa at 37 °C;
iv) no failure at an unconfined compression to at least 10% of its original thickness at 37 °C;
v) a maximum stress of at least 0.4 MPa at 25% strain in confined compression at 37 °C;
vi) a fatigue resistance of at least 60.000 cycles in a diurnal loading regime at 37 °C in PBS, whereby the diurnal loading regime consists of 50.000 cycles to 80% strain at 5 Hz followed by 10.000 cycles to 10% strain at 5 Hz; and
vii) an increase in weight of the cross-linked hydrogel ('composition C4') due to absorption of water after 1 day at 37°C in PBS that is less than about 100%, based on the original weight of the cross-linked hydrogel.

In a most preferred embodiment, the cross-linked hydrogel ('composition C4') has four of the properties (i) to (vii), even more preferably the cross-linked hydrogel ('composition C4') has all of the properties (i) to (vii).

In a very preferred embodiment, the cross-linked hydrogel ('composition C4'), after 16 h of curing, has at least one of the following properties (i) to (iii):

i) no failure at an unconfined compression of 4.0 MPa at 37 °C;
ii) no failure at an unconfined compression to at least 10% of its original thickness at 37 °C;
iii) a maximum stress of at least 0.4 MPa at 25% strain in confined compression at 37 °C.

In another embodiment, the cross-linked hydrogel ('composition C4') comprises as further ingredients one or more biologically active and/or pharmaceutically active compounds. The inventors have found that the cross-linked hydrogel ('composition C4') can act as a carrier material for biologically active and/or pharmaceutically active compounds that can provide controlled or prolonged release of the biologically active and/or pharmaceutically active compounds to the environment.

A biologically active or pharmaceutically active compound, as used herein, includes a compound which provides a therapeutic, diagnostic or prophylactic effect, a compound that stimulates or participates in tissue growth, cell growth, cell differentiation, a compound that may be able to invoke a biological action such as an immune response, or a compound that could play any other role in one or more biological processes. Such compounds, peptide or non-peptide, protein or non-protein, include but are not limited to bone morphogenetic proteins, antimicrobial agents (including antibacterial, hemotherapeutic and anti-fungal agents), anti-viral agents, anti-tumor agents, hormones, hormone antagonistics, corticosteroids such as mineralocorticosteroids or glucocorticosteroids, androgents, estrogens, progestins immunogenic agents, anti-inflammatory agents, anti-gout agents, centrally acting analgesics, local anesthetics, centrally active muscle relaxants, paracrine factors, growth factors, nucleic acids, DNA-derivatives, RNA-derivatives, lipids, lipopolysaccharides, (poly)saccharides, vitamins, and peptides, polypeptides and proteins in general, more preferably, bone morphogenetic proteins and TGF-$\beta$. The biologically active compounds preferably comprise at least one 4H-unit per molecule up to a maximum of ten 4H-units, preferably one to four, and most preferably two to four 4H-units. These 4H-units are preferably covalently attached to the biologically active compound.

The cross-linked hydrogel ('composition C4') preferably comprises 0.0005 to 10 wt.% of one or more biologically active and/or pharmaceutically active compounds, such as the one or more biologically active and/or pharmaceutically active compounds as defined hereinbefore, more preferably 0.0005 to 1 wt.%, and most preferably 0.005 to 1 wt.%, based on the weight of the cross-linked hydrogel.

In a preferred embodiment, the cross-linked hydrogel ('composition C4') comprises one or more further ingredients, wherein said one or more further ingredients are selected from the group consisting of peptides, proteins, antimicrobial agents, anti-viral agents, hormones, paracrine factors, growth factors, vitamins, anti-inflammatory drugs, corticosteroids, local anesthetics and combinations thereof, preferably in an amount of 0.0005 to 10 wt.%, based on the weight of the cross-linked hydrogel, more preferably 0.0005 to 1 wt.%, and most preferably 0.005 to 1 wt.%.

Preferably, the cross-linked hydrogel ('composition C4') comprises one or more further ingredients which provide a therapeutic, diagnostic or prophylactic effect, or stimulate cell growth or differentiation, wherein said one or more further ingredients are selected from the group consisting of peptides, proteins, antimicrobial agents, anti-viral agents, hormones, paracrine factors, growth factors, vitamins, anti-inflammatory drugs, corticosteroids, local anesthetics and combinations thereof, preferably in an amount of 0.0005 to 10 wt.%, based on the weight of the cross-linked hydrogel, more preferably 0.0005 to 1 wt.%, and most preferably 0.005 to 1 wt.%.

The cross-linked hydrogel ('composition C4') is only partly and at least not completely biodegraded *in situ* at the place of administration until after a period of about 1 months, preferably until after a period of about 2 months, most

preferably until after a period of about 3 months, wherein the biodegradation level is the remaining fraction after a certain period of time that is determined by:

*(mass of the liquid hydrogel composition ('composition $C_3$ ) administered - mass of the cross-linked hydrogel ('composition C4') after a certain period of time) divided by (mass of the liquid hydrogel composition ('composition $C_3$ ) administered) x 100%;*

or

*(combined masses of the liquid hydrogel formulation ('composition C1') and the aqueous auxiliary formulation ('composition C2') administered - mass of the cross-linked hydrogel ('composition C4') after a certain period of time) divided by (combined masses of the liquid hydrogel formulation ('composition C1') and the aqueous auxiliary formulation ('composition C2') administered) x 100%.*

Preferably, said cross-linked hydrogel ('composition C4') is biodegraded to a level of at most about 90%, more preferably at most about 70%, most preferably at most about 50% within a period of about 1 month, preferably within a period of about 2 months, most preferably within a period of about 3 months.

## THE INJECTABLE HYDROGEL ('COMPOSITION C5')

The injectable hydrogel ('composition C5') comprises one or more hydrophilic polymer gellants dissolved in water, with optional further ingredients. The injectable hydrogel preferably comprises 0.3 - 50.0 wt.% of the hydrophilic polymer, more preferably 1 - 25 wt.%, most preferably 5 - 20 wt.%, based on the weight of the injectable hydrogel ('composition C5').

The injectable hydrogel formulation preferably comprises 50 - 99 wt.% of water, more preferably 75 - 99 wt.%, most preferably 80 - 95 wt.%, based on the weight of the injectable hydrogel.

The injectable hydrogel formulation preferably comprises 0.01 - 18 wt.% of further ingredients, more preferably 0.02 - 15 wt.%, most preferably 0.025 - 5 wt.%, based on the weight of the injectable hydrogel.

The optional further ingredients are functional ingredients that will contribute to the specific use of the hydrogel. The injectable hydrogel ('composition C5') may comprise as further ingredients one or more selected from a pH-buffer, solid filler, a diluent, a thickener, a carrier, and other excipients known in the art.

In another embodiment, the injectable hydrogel comprises as further ingredients one or more biologically active and/or pharmaceutically active compounds. A biologically active or pharmaceutically active compound, as used herein, includes a compound which provides a therapeutic, diagnostic or prophylactic effect, a compound that affects or participates in tissue growth, cell growth, cell differentiation, a compound that may be able to invoke a biological action such as an immune response, or a compound that could play any other role in one or more biological processes. Such compounds, peptide or non-peptide, protein or non-protein, include but are not limited to bone morphogenetic proteins, antimicrobial agents (including antibacterial, hemotherapeutic and anti-fungal agents), anti-viral agents, anti-tumor agents, hormones, hormone antagonistics, corticosteroids such as mineralocorticosteroids or glucocorticosteroids, androgents, estrogens, progestins immunogenic agents, anti-inflammatory agents, non-steroidal anti-inflammatory drugs, anti-gout agents, centrally acting analgesics, local anesthetics, centrally active muscle relaxants, growth factors, nucleic acids, DNA-derivatives, RNA-derivatives, lipids, lipopolysaccharides, (poly)saccharides, vitamins, and peptides, polypeptides and proteins in general, more preferably, bone morphogenetic proteins and TGF-β.

In a less preferred embodiment, the injectable hydrogel comprises as further ingredient a bioactive species selected from the group consisting of a living cell, an enzyme, or a micro-organism. A living cell in this embodiment means and includes individual animal and plant cells, cell clusters, tissues, organs and organisms, including organisms such as bacteria, fungi or moulds.

In a preferred embodiment, the injectable hydrogel ('composition C5') does not comprise:

- ingredients derived from animal or human tissue; and/or
- cells, cell extracts, proteins, glycosaminoglycans and biologically derived macromolecules; and/or
- stem cells and growth factors.

More preferably, the injectable hydrogel ('composition C5') does not contain cells, lyophilized cell matrices, mammalian cell extracts, most preferably the liquid hydrogel formulation does not contain cells, lyophilized cell matrices, and platelet lysate.

The viscosity of the injectable hydrogel ('composition C5') is low enough to allow administration of the formulation

by a syringe or catheter.

The hydrophilic polymer gellant is a macromolecule that has a solubility in water of at least 1 g/L at 20 °C, more preferably at least 10 g/L at 20 °C and that comprises functional groups that may be used to facilitate chemical or physical cross-linking. Chemical cross-linking may be performed by functional groups that are able to react with the same functional group in which the hydrophilic polymer gellant itself is sufficient to result in across-linked hydrogel after initiation with appropriate agents known in the field, or chemical cross-linking may be performed by cross-linking reactions between dissimilar functional groups that can react under physiological conditions in relevant timeframes for the medical treatment and that are known in the field. Physical cross-linking may come from hydrogen bonding, hydrophobic interactions or ionic interactions that may be mediated by pH, metal ions, or temperature.

## MEDICAL TREATMENT OR PREVENTION

The references to the methods of treatment by therapy or surgery in this description are to be interpreted as references to compositions of the present invention for use in those methods.

The treatment and prevention as defined herein concerns osteoarthritis. The wording *'treatment and prevention of osteoarthritis'* as used herein comprises the prevention of initial (micro-)damage to the cartilage in a vertebrate at risk of developing osteoarthritis, the subclinical phase wherein initial (micro-)damage to the cartilage does not yet result in symptoms that require clinical medical interventions, up to the symptomatic phase wherein cartilage degeneration has proceeded to such extent that the use of pain killers and physiotherapy, or even clinical medical interventions, such as local injections or surgery, may be required (*i.e.* all three stages (a), (b) and (c) of the development of osteoarthritis as depicted in Figure 1).

Accordingly, the wording *'treatment and prevention of osteoarthritis'* as used herein also comprises the prevention or treatment of focal (osteo-)chondral (micro-) damage.

The inventors have found that a cross-linked hydrogel, preferably 'composition C4' acts as a cushion in between the articular joint surfaces in a synovial cavity, thereby spreading and reducing the joint contact loads (internal distraction and shock absorption). In this way, the origination of focal (osteo-)chondral (micro-)damage in a vertebrate at risk of developing osteoarthritis can be prevented. When focal (osteo- )chondral (micro-)damage is already present, the invention ensures that pain is reduced and further cartilage degeneration is prevented or reduced. Administration may be performed to the synovial joint right after trauma or overloading, or even prior to expected damage. The liquid hydrogel formulations or injectable hydrogel, preferably 'composition C1' and 'composition C2', or 'composition C3', can also be administered to the already degenerated, osteoarthritic synovial joint. In this case, the resulting cushion may acutely contribute to pain reduction and reduction or prevention of further cartilage degeneration.

In an embodiment, said treatment or prevention comprises removal of synovial fluid from the cavity of the synovial joint of the vertebrate before injecting 'composition C1' and 'composition C2', or 'composition C3'.

In a preferred embodiment, said treatment or prevention comprises administering 0.5 - 250 mL, more preferably 3 - 60 mL, most preferably 6 - 60 mL of the combined composition C1' and 'composition C2' or of 'composition C3'. The appropriate amount to be administered depends on the type of synovial joint, the type of vertebrate, on the (remaining) amount of synovial fluid being present in the synovial joint and on the specific medical condition. It is within the skills of the person skilled in the art to choose an appropriate amount.

In an embodiment, said treatment or prevention comprises repeated administration in order to fill the cavity of the synovial joint further or to replace damaged or degraded cross-linked hydrogel ('composition C4').

The cross-linked hydrogel ('composition C4') is formed after administration of the ingredients into the synovial fluid or into the cavity of a synovial joint of the vertebrate.

The cross-linked hydrogels ('compositions C4') can be prepared by three different methods:

(i) the liquid hydrogel formulation ('composition C1') and the aqueous auxiliary formulation ('composition C2') are mixed to provide liquid hydrogel composition 'composition C3' followed by administering liquid hydrogel composition 'composition C3' to the desired location, after which the cross-linked hydrogel ('composition C4') is formed in time;

(ii) the liquid hydrogel formulation ('composition C1') is *in situ* mixed with the aqueous auxiliary formulation ('composition C2') during administration to provide liquid hydrogel composition 'composition C3' to the desired location resulting in the formation of the cross-linked hydrogel ('composition C4'); or

(iii) the liquid hydrogel formulation ('composition C1') and the aqueous auxiliary formulation ('composition C2') are administered separately and subsequently in any order to the desired location where liquid hydrogel composition 'composition C3' is formed after which the cross-linked hydrogel ('composition C4') is formed in time.

Preferably, the cross-linked hydrogel ('composition C4') is formed, whereby the term *'formed'* in this sentence means that the storage modulus and the loss modulus of the 'composition C4' are equal, in between about 6 seconds and about 60 minutes, more preferably in between about 30 seconds and about 35 minutes, most preferably in between about

1 minute and about 15 minutes after mixing the liquid hydrogel formulation ('composition C1') with the aqueous auxiliary formulation ('composition C2'). As will be appreciated by the skilled person, the liquid hydrogel composition 'composition C3' needs to be administered before any cross-linking prevents administration. Accordingly, the liquid hydrogel composition 'composition C3' is preferably administered within 60 minutes, such as within 30 minutes, within 20 minutes, within 10 minutes, within 5 minutes, within 3 minutes, with 2 minutes, within 60 seconds, within 40 seconds, within 30 seconds, within 20 seconds, within 10 seconds, or within 6 seconds, from combining the liquid hydrogel formulation ('composition C1') and the aqueous auxiliary formulation ('composition C2').

Administration of the liquid hydrogel formulation ('composition C1'), the aqueous auxiliary formulation ('composition C2') or of the liquid hydrogel composition ('composition C3') can be done by any method known in the art, preferably using a technique chosen from the group consisting of injection, arthroscopic surgery, open surgery and combinations thereof, more preferably injection. The term *'injection'* as used herein includes injection through a syringe equipped with a needle, injection through a catheter equipped with a needle, injection through a double chamber syringe equipped with a needle and injection through a double chamber catheter equipped with a needle. Preferably, the liquid hydrogel formulation ('composition C1'), the aqueous auxiliary formulation ('composition C2') or the liquid hydrogel composition ('composition C3') is injected through a syringe equipped with a needle or a double chamber syringe equipped with a mixing chamber and a needle. Preferably, the needle gauge size is 15G or smaller, more preferably 18G or smaller. As will be appreciated by the skilled person, injection can be advantageously performed through the skin and soft tissue directly into the synovial fluid or into the cavity of the synovial joint.

In an embodiment, the liquid hydrogel formulation ('composition C1'), the aqueous auxiliary formulation ('composition C2') or the liquid hydrogel composition ('composition C3') are specifically suitable for administration to synovial joints that endure high compression loads.

In a preferred embodiment, the synovial joint of the vertebrate is chosen from the group of joints consisting of:

- shoulder girdle (glenohumeral, sternoclavicular, acromioclavicular);
- elbow (three articulations within one joint capsule);
- wrist (distal radio-ulnar, radiocarpal);
- hand including fingers (intercarpal, carpometacarpal (I - V), metacarpophalangeal (I - V), interphalangeal-I, proximal interphalangeal (II - V), distal interphalangeal (II - V));
- pelvic girdle (hip, sacroiliac);
- knee;
- ankle (talocrural, subtalar);
- foot including toes (talonavicular, talocalcaneonavicular, calcaneocuboid, calcaneonavicular, cuboideonavicular, intercuneiforms, tarsometatarsal (I - V), metatarsophalangeal (I - V), interphalangeal-I, proximal interphalangeal (II - V), distal interphalangeal (II - V));
- jaw (temporomandibular); and
- trunk (facet, costovertebral, sternocostal).

In a very preferred embodiment, the synovial joint of the vertebrate is chosen from the group consisting of shoulder girdle, elbow, wrist, hand and fingers, pelvic girdle, knee, ankle, foot and toes, jaw and trunk joints, and in a most preferred embodiment the synovial joint of the vertebrate is chosen from the group consisting of shoulder girdle, wrist, fingers, ankle, foot and jaw.

The compositions can be administered to any living vertebrate. In an embodiment, the vertebrate is chosen from the group consisting of humans, aves, companion animals, racing animals and cattle. Non-limiting examples of companion animals are cats, dogs and rabbits. Non-limiting examples of racing animals and cattle include horses, camels, sheep, cows and pigs. Non-limiting examples of aves include parrots, pigeons and eagles.

In a preferred embodiment, the vertebrate is human.

In another preferred embodiment, the vertebrate is chosen from the group consisting of aves, companion animals, racing animals and cattle, more preferably companion animals and racing animals.

In yet another preferred embodiment, the vertebrate is chosen from cats, dogs, horses, camels, pigeons and eagles, more preferably dogs and horses.

Preferably, the cross-linked hydrogel ('composition C4') is slowly biodegradable resulting in degradation after it has been administered, such that no surgical removal is needed at a later stage, thereby reducing clinical costs and distress for the patient. On the other hand, the degradation of the cross-linked hydrogel ('composition C4') is preferably slow enough to mediate enough clinical action needed for the treatment or prevention of osteoarthritis.

In another aspect, the invention relates to a cross-linked hydrogel ('composition C4') as defined hereinbefore for use in the prevention or treatment of joint pathologies in a vertebrate, preferably concerning synovial joints, most preferably concerning synovial joints chosen from the group consisting of shoulder, hip, knee, hand joints, foot joints, elbow, facet joint and jaw joint, said treatment comprising administering the liquid hydrogel formulation ('composition C1')

and the aqueous auxiliary formulation ('composition C2') or the liquid hydrogel composition ('composition C3') as defined hereinbefore to the joint of the vertebrate, wherein the cross-linked hydrogel ('composition C4') acts as a cushion between the articular joint surfaces thereby spreading and reducing the joint contact loads. Due to this cushioning effect, further degeneration is prevented or reduced, pain is reduced and remaining cartilage can be regenerated.

In yet another aspect, the invention relates to a cross-linked hydrogel ('composition C4') as defined hereinbefore for use in the prevention or treatment of focal (osteo-)chondral (micro-)damage in a vertebrate, preferably concerning synovial joints, most preferably concerning synovial joints chosen from the group consisting of shoulder, hip, knee, hand joints, foot joints, elbow, facet joint and jaw joint, said treatment comprising administering the liquid hydrogel formulation ('composition C1') and the aqueous auxiliary formulation ('composition C2') or the liquid hydrogel composition ('composition C3') as defined hereinbefore to a joint of the vertebrate, wherein the cross-linked hydrogel ('composition C4') acts as a cushion between the articular joint surfaces thereby spreading and reducing the joint contact loads. In this way, the origination of focal (osteo-)chondral (micro-)damage can be prevented.

In another aspect, the invention relates to an injectable hydrogel ('composition C5') that is injectable and that can, after physical and/or chemical cross-linking, withstand a compression at 37 °C without failure of at least 1.0 MPa, more preferably of at least 2.0 MPa, even more preferably of at least 4.0 MPa and most preferably of at least 5.0 MPa, for use in the prevention or treatment of joint pathologies in a vertebrate, preferably concerning synovial joints, most preferably concerning synovial joints chosen from the group consisting of shoulder, hip, knee, hand joints, foot joints, elbow, facet joint and jaw joint, said treatment comprising the injection of a hydrogel in the joint of the vertebrate, wherein the hydrogel acts as a cushion between the articular joint surfaces thereby spreading and reducing the joint contact loads. Due to this cushioning effect, further degeneration is prevented or reduced, pain is reduced and remaining cartilage can be regenerated.

In another aspect, the invention relates to an injectable hydrogel ('composition C5') that is injectable and that can, after physical and/or chemical cross-linking, withstand a compression at 37 °C without failure of at least 1.0 MPa, more preferably of at least 2.0 MPa, even more preferably of at least 4.0 MPa and most preferably of at least 5.0 MPa, for use in the prevention or treatment of osteoarthritis in a joint of a vertebrate, preferably concerning synovial joints, most preferably concerning synovial joints chosen from the group consisting of shoulder, hip, knee, hand joints, foot joints, elbow, facet joint and jaw joint, said treatment comprising the injection of the hydrogel in a joint of the vertebrate, wherein the hydrogel acts as a cushion between the articular joint surfaces thereby spreading and reducing the joint contact loads. In this way, the origination of focal (osteo-)chondral (micro-)damage can be prevented.

The first aspect can also be worded as a method of treatment or prevention of osteoarthritis in a vertebrate, wherein said treatment comprises combining liquid hydrogel formulation ('composition C1') and aqueous auxiliary formulation ('composition C2') as defined hereinbefore to form a liquid hydrogel composition ('composition C3') and injecting said liquid hydrogel composition ('composition C3') into the synovial fluid or into the cavity of a synovial joint of the vertebrate, to form a cross-linked hydrogel ('composition C4') in the synovial joint of the vertebrate.

The second aspect can also be worded as a method of treatment or prevention of osteoarthritis in a vertebrate, wherein said treatment comprises in a first step injecting one of the liquid hydrogel formulation ('composition C1') and the aqueous auxiliary formulation ('composition C2') as defined hereinbefore, preferably liquid hydrogel formulation ('composition C1'), into the synovial fluid or into the cavity of a synovial joint of the vertebrate and in a second step injecting the other of the liquid hydrogel formulation ('composition C1') and the aqueous auxiliary formulation ('composition C2') to form a liquid hydrogel composition ('composition C3') in the synovial joint of the vertebrate that reacts to a cross-linked hydrogel ('composition C4').

The third aspect can also be worded as a method of treatment or prevention of osteoarthritis in a vertebrate, wherein said treatment comprises injecting an injectable hydrogel ('composition C5') into the synovial fluid or into the cavity of a synovial joint of the vertebrate to form a physical and/or chemical cross-linked hydrogel.

Thus, the invention has been described by reference to certain embodiments discussed above.

Furthermore, for a proper understanding of this document and its claims, it is to be understood that the verb *'to comprise'* and its conjugations are used in its non-limiting sense to mean that items following the word are included, but items not specifically mentioned are not excluded. In addition, reference to an element by the indefinite article *'a'* or *'an'* does not exclude the possibility that more than one of the element is present, unless the context clearly requires that there be one and only one of the elements. The indefinite article *'a'* or *'an'* thus usually means *'at least one'.*

The following example serves to demonstrate the preferred embodiments of the present invention and should not be interpreted as limiting the scope of the invention.

## EXAMPLES

When not specifically mentioned, chemicals are obtained from Sigma Aldrich. IPDI is isophorone diisocyanate, DIPEA is diisopropylethylamine. Lyophilized horse radish peroxidase (HRP) 173 U/mg has been used. PBS is phosphate buffered saline in water at pH = 7.4 (0.01 M phosphate buffer, 0.0027 M potassium chloride and 0.137 M sodium chloride).

DBTDL is dibutyltin dilaurate.

All average molecular weights $M_n$ throughout this patent application have been defined and measured using the following SEC method, unless specified otherwise. SEC was performed with a GPC-system equipped with a KD-804 column (Showa Denko, 500 - 400000 Da) using RI detection with DMF comprising 10 mM LiBr as eluent at a flow rate of 1.0 mL/min at 50 °C. The molecular weights $M_n$ were calculated on selected peaks relative to PEO/PEG-standards (Polymer Laboratories, molecular weight range: 580 - 100000 g/mol).

**Example 1: synthesis of polymer gellants**

*Synthesis of Polymer Gellant 1 (Compound according to Formula (VIII)*

Telechelic hydroxy-terminated PEG-2000 (20.0 g, 10.0 mmol, compound according to Formula $FG^1$-POL-$FG^1$) was heated *in vacuo* in a 3-neck flask to 120°C together with 2-amino-4-hydroxy-5-(2-hydroxyethyl)-6-methyl-pyrimidine (845 mg, 5.00 mmol, compound according to Formula (IA)) and stirred for 1 h *in vacuo.* After cooling to 80°C, IPDI (4.44 g, 20.0 mmol, compound according to Formula OCN-$R^4$-NCO) was added and 1 drop DBTDL while stirring. The reaction mixture was stirred for 3 h at 80 °C. Subsequently, tyramine (1.260 g, 9.2 mmol, compound according to Formula (VII)) was added to the viscous reaction mixture followed by the addition of 2 mL DMSO. The mixture was stirred for another 2 hours at 80°C, followed by cooling to 70°C while stirring under an argon atmosphere. To this mixture 30 mL methyl-THF was added, followed by cooling and stirring until 25°C, after which 30 mL methyl-t-butyl ether was added resulting in the formation of a white precipitate, which was isolated by filtration. The dried precipitate was redissolved in 60 mL chloroform and 7 mL ethanol, filtered, and precipitated by the addition of methyl-t-butyl ether. The product was isolated by filtration and subsequent drying of the residue. Yield: 83%. SEC: $M_n$ = 4.1 kDa.

*Synthesis of Polymer Gellant 2 (Compound according to Formula (VIII))*

Telechelic hydroxy-terminated PEG-2000 (20.0 g, 10.0 mmol, compound according to Formula $FG^1$-POL-$FG^1$) was heated *in vacuo* in a 3-neck flask to 120°C together with 2-amino-4-hydroxy-5-(2-hydroxyethyl)-6-methyl-pyrimidine (846 mg, 5.00 mmol, compound according to Formula (IA)) and stirred for 1 h *in vacuo.* After cooling to 70°C, IPDI (4.44 g, 20.0 mmol, compound according to Formula OCN-$R^4$-NCO) was added and 1 drop DBTDL while stirring. After 1 h the reaction mixture was stirred for another 2 h at 120°C, followed by cooling to 100°C under an argon atmosphere. Subsequently, dopamine hydrochloride (1.713 g, 9.0 mmol, compound according to Formula (VII)) was dissolved in 4 mL DMSO in the presence of 1.1 mL DIPEA, and added to the viscous reaction mixture at 100°C. The mixture was stirred for 15 minutes at 100°C, followed by cooling to 70°C while stirring under an argon atmosphere. To this mixture 30 mL methyl-THF was added, followed by cooling and stirring until 25°C, after which 30 mL methyl-t-butyl ether was added resulting in the formation of a white precipitate, which was isolated by filtration. The dried precipitate was redissolved in 60 mL chloroform and 7 mL ethanol, filtered, and precipitated by the addition of methyl-t-butyl ether. The product was isolated by filtration and subsequent drying of the residue. Yield: 85%. SEC: $M_n$ = 4.3 kDa.

*Synthesis of Polymer Gellant 3 (Compound according to Formula (VIII))*

Telechelic hydroxy terminated PEG-3000 (16.0 g, 5.33 mmol, compound according to Formula $FG^1$-POL-$FG^1$) was heated *in vacuo* in a 3-neck flask to 120°C together with 2-amino-4-hydroxy-5-(2-hydroxyethyl)-6-methyl-pyrimidine (676 mg, 4.00 mmol, compound according to Formula (IA)) and stirred for 1 h *in vacuo.* After cooling to 70°C, IPDI (2.37 g, 10.7 mmol, compound according to Formula OCN-$R^4$-NCO) was added and 1 drop DBTDL while stirring. After 1 h the reaction mixture was stirred for another 2 h at 120°C, followed by cooling to 100°C under an argon atmosphere. Subsequently, dopamine hydrochloride (557 mg, 2.93 mmol, compound according to Formula (VII)) was dissolved in 2 mL DMSO in the presence of 0.55 mL DIPEA, and added to the viscous reaction mixture at 100°C. The mixture was stirred for 15 minutes at 100°C, followed by cooling to 70°C while stirring under an argon atmosphere. To this mixture 30 mL methyl-THF was added, followed by cooling and stirring until 25°C, after which 30 mL methyl-t-butyl ether was added resulting in the formation of a white precipitate, which was isolated by filtration. The dried precipitate was redissolved in 60 mL chloroform and 7 mL ethanol, filtered, and precipitated by the addition of methyl-t-butyl ether. The product was isolated by filtration and subsequent drying of the residue. Yield: 87%. SEC: $M_n$ = 5.0 kDa.

*Synthesis of Polymer Gellant 4 (Compound according to Formula (VIII))*

Telechelic hydroxy terminated PEG-6000 (29.9 g, 4.98 mmol, compound according to Formula $FG^1$-POL-$FG^1$) was heated *in vacuo* in a 3-neck flask to 120°C together with 2-amino-4-hydroxy-5-(2-hydroxyethyl)-6-methyl-pyrimidine (421 mg, 2.49 mmol, compound according to Formula (IA)) and stirred for 1 h *in vacuo.* After cooling to 70°C, IPDI (2.21 g,

10.0 mmol, compound according to Formula OCN-$R^4$-NCO) was added and 1 drop DBTDL while stirring. After 1 h the reaction mixture was stirred for another 2 h at 120°C, followed by cooling to 100°C under an argon atmosphere. Subsequently, dopamine hydrochloride (853 mg, 4.49 mmol, compound according to Formula (VII)) was dissolved in 3 mL DMSO in the presence of 0.85 mL DIPEA, and added to the viscous reaction mixture at 100°C. The mixture was stirred for 15 minutes at 100°C, followed by cooling to 70°C while stirring under an argon atmosphere. To this mixture 30 mL methyl-THF was added, followed by cooling and stirring until 25°C, after which 30 mL methyl-t-butyl ether was added resulting in the formation of a white precipitate, which was isolated by filtration. The dried precipitate was redissolved in 60 mL chloroform and 7 mL ethanol, filtered, and precipitated by the addition of methyl-*t*-butyl ether. The product was isolated by filtration and subsequent drying of the residue. Yield: 86%. SEC: $M_n$ = 11.8 kDa.

*Synthesis of Polymer Gellant 5 (Compound according to Formula (VIII))*

Telechelic hydroxy terminated PEG-2000 (20.0 g, 10.0 mmol, compound according to Formula $FG^1$-POL-$FG^1$) was heated *in vacuo* in a 3-neck flask to 120°C together with 2-amino-4-hydroxy-5-(2-hydroxyethyl)-6-methyl-pyrimidine (845 mg, 5.00 mmol, compound according to Formula (IA)) and stirred for 1 h *in vacuo.* After cooling to 90°C, 1,6-diisocyanato hexane (3.36 g, 20.0 mmol, compound according to Formula OCN-$R^4$-NCO) was added and 1 drop DBTDL while stirring for another 3 h at 90°C. Subsequently, dopamine hydrochloride (1.792 g, 9.43 mmol, compound according to Formula (VII)) was dissolved in 4 mL DMSO in the presence of 2 mL DIPEA, and added to the viscous reaction mixture at 90°C. The mixture was stirred for 60 minutes at 90°C, followed by cooling to 70°C while stirring under an argon atmosphere. To this mixture, 30 mL methyl-THF was added, followed by cooling and stirring until a temperature of 25°C was reached, after which 30 mL methyl-*t*-butyl ether was added resulting in the formation of a white precipitate, which was isolated by filtration. The dried precipitate was redissolved in 70 mL chloroform and 10 mL ethanol, filtered, and precipitated by the addition of methyl-t-butyl ether. The product was isolated by filtration and subsequent drying of the residue. Yield: 84%. SEC: $M_n$ = 2.7 kDa.

*Synthesis of Polymer Gellant 6 (Compound according to Formula (VIII))*

Telechelic hydroxy terminated PEG-3000 (30.0 g, 10.0 mmol, compound according to Formula $FG^1$-POL-$FG^1$) was heated *in vacuo* in a 3-neck flask to 120°C together with 2-amino-4-hydroxy-5-(2-hydroxyethyl)-6-methyl-pyrimidine (848 mg, 5.02 mmol, compound according to Formula (IA)) and stirred for 1 h *in vacuo.* After cooling to 90°C, 1,6-diisocyanato hexane (3.35 g, 19.9 mmol, compound according to Formula OCN-$R^4$-NCO) was added and 1 drop DBTDL while stirring for another 3 h at 90°C. Subsequently, dopamine hydrochloride (1.792 g, 9.43 mmol, compound according to Formula (VII)) was dissolved in 4 mL DMSO in the presence of 2 mL DIPEA, and added to the viscous reaction mixture at 90°C. The mixture was stirred for 60 minutes at 90°C, followed by cooling to 70°C while stirring under an argon atmosphere. To this mixture 40 mL methyl-THF was added, followed by cooling and stirring until a temperature of 25°C was reached, after which 40 mL methyl-t-butyl ether was added resulting in the formation of a white precipitate, which was isolated by filtration. The dried precipitate was redissolved in 100 mL chloroform and 15 mL ethanol, filtered, and precipitated by the addition of methyl-t-butyl ether. The product was isolated by filtration and subsequent drying of the residue. Yield: 84%. SEC: $M_n$ = 6.2 kDa.

**Example 2: Preparation of liquid hydrogel formulations ('composition C1')**

Liquid hydrogel formulations ('composition C1') were obtained by dissolving the desired amount of the polymer gellant prepared in Example 1 into PBS to which 1 N HCl was added to reach the desired pH value in the range of 3.0 to 6.0 as assessed with a pH meter. Table 1 lists typical formulations obtained with the polymers gellants of Example 1. The contents of the different ingredients are expressed as weight percentages based on the weight of the liquid hydrogel formulation ('composition C1'). The further ingredients are the dissolved compounds in the PBS with the adjusted pH, *i.e.* the phosphate buffer, HCl, potassium chloride and sodium chloride. The dynamic viscosity was measured at 37°C with a rheometer (Anton Paar) with a plate-plate geometry at a shear rate of 1 $s^{-1}$ and with a gap distance of 0.50 mm.

Table 1: Liquid hydrogel formulations ('composition C1') at 37 °C

| Liquid hydrogel formul. | Polymer gellant (content) | Water (content) | Further ingredients (content) | pH | Morphol. | Dynamic viscosity [Pa·s] |
|---|---|---|---|---|---|---|
| 1 | 1 (15 wt.%) | 84 wt.% | 1 wt.% | 6.0 | Liquid | 0.07 |
| 2 | 2 (15 wt.%) | 84 wt.% | 1 wt.% | 5.9 | Liquid | 0.14 |

(continued)

| Liquid hydrogel formul. | Polymer gellant (content) | Water (content) | Further ingredients (content) | pH | Morphol. | Dynamic viscosity [Pa·s] |
|---|---|---|---|---|---|---|
| 3 | 2 (20 wt.%) | 79 wt.% | 1 wt.% | 5.9 | Viscous liquid | 1.11 |
| 4 | 3 (13 wt.%) | 86 wt.% | 1 wt.% | 5.9 | Liquid | 0.14 |
| 5 | 3 (15 wt.%) | 84 wt.% | 1 wt.% | 5.9 | Viscous liquid | 0.29 |
| 6 | 4 (15 wt.%) | 84 wt.% | 1 wt.% | 6.0 | Viscous liquid | 0.47 |
| 7 | 3 (15 wt.%) | 84 wt.% | 1 wt.% | 4.6 | Viscous liquid | 0.29 |
| 8 | 3 (15 wt.%) | 84 wt.% | 1 wt.% | 3.5 | Viscous liquid | 0.29 |
| 9 | 5 (20 wt.%) | 79 wt.% | 1 wt.% | 5.3 | Liquid | 0.64 |
| 10 | 6 (20 wt.%) | 79 wt.% | 1 wt.% | 5.3 | Liquid | 0.58 |
| 11 | 6 (15 wt.%) | 84 wt.% | 1 wt.% | 5.3 | Liquid | 0.41 |

All liquid hydrogel formulations ('composition C1') 1 to 11 could be injected with a syringe equipped with a 15 G needle.

**Example 3: Preparation of liquid hydrogel compositions ('composition C3') and cross-linked hydrogels ('composition C4')**

Cross-linked hydrogels ('composition C4') were obtained by combining the selected liquid hydrogel formulation ('composition C1') from Table 1 of Example 2 with an aqueous auxiliary composition ('composition C2') to obtain liquid hydrogel compositions ('composition C3') and by giving the liquid hydrogel compositions ('composition C3') some time for cross-linking. The aqueous auxiliary formulations ('composition C2') comprised oxidants. See Table 2 for the compositions of the aqueous auxiliary formulations ('composition C2'). The cross-linking reaction either followed Method A, B or C as described *infra,* depending on the composition of the aqueous auxiliary formulation ('composition C2'), as depicted in Table 2. Cross-linked hydrogels ('composition C4') were formed in timeframes ranging from 50 seconds to 30 minutes after combining the liquid hydrogel formulation ('composition C1') with the aqueous auxiliary composition ('composition C2') to obtain liquid hydrogel composition ('composition C3'). The time needed to form cross-linked hydrogels was determined by visual inspection.

*Method A: Preparation of aqueous auxiliary formulation A ('composition C2') and cross-linked hydroel ('composition C4')*

Aqueous auxiliary formulation A ('composition C2') was prepared as follows. A stock solution was made comprising 0.28 M periodate ($NaIO_4$) dissolved in 10 mM $Na_4P_2O_7$ in PBS. The desired amount of this periodate stock solution (typically 0.7 - 1.0 mol equivalent of $NaIO_4$ to mono- or multihydroxyphenyl moiety) was added to a selected liquid hydrogel formulation ('composition C1') of Example 2 in a syringe, the mixture was shaken for 10-20 seconds and was subsequently injected into the location of choice through a 18G needle. Accordingly, the periodate in aqueous auxiliary formulation A ('composition C2') was used to initiate or mediate chemical cross-linking of two or more groups E on the polymer gellant according to Formula (VIII) in the liquid hydrogel formulation ('composition C1').

*Method B: Preparation of aqueous auxiliary formulation B ('Composition C2') and cross-linked hydroel ('composition C4')*

Aqueous auxiliary formulation B ('composition C2') was prepared as follows. 30 w% $H_2O_2$ was diluted with PBS-buffered water to 62 mM to which was added HRP (horse radish peroxidase) stock solution comprising lyophilized HRP in PBS at 400 U/mL. The amount of the aqueous auxiliary formulation B ('composition C2') added to liquid hydrogel formulation ('composition C1') is equal to 5-20 U/mL as based on the volume of the resulting liquid hydrogel composition ('composition C3'). The desired amount (typically 0.7 - 1.0 mol equivalent of $H_2O_2$ to mono- or multihydroxyphenyl moiety) of the $H_2O_2$- and HRP-comprising aqueous auxiliary formulation ('composition C2') was added to a liquid hydrogel formulation ('composition C1') of Example 2 in a syringe followed by shaking the syringe for 10-20 seconds and injecting this mixture in the location of choice through a 15G needle. Accordingly, the HRP in aqueous auxiliary formulation B was

used to initiate or mediate chemical cross-linking of two or more groups E on the polymer gellant according to Formula (VIII) in the liquid hydrogel formulation ('composition C1').

*Method C: Preparation of aqueous auxiliary formulation C ('composition C2') and cross-linked hydroel ('composition C4')*

Aqueous auxiliary formulation C ('composition C2') was prepared as follows. A stock solution was made comprising 0.28 M periodate ($NaIO_4$) dissolved in PBS. The desired amount of this periodate stock solution (typically 0.7 - 1.0 mol equivalent of $NaIO_4$ to mono- or multihydroxyphenyl moiety) was added to a selected liquid hydrogel formulation ('composition C1') of Example 2 in a syringe, the mixture was shaken for 10-20 seconds and was subsequently injected into the location of choice through a 18G needle. Accordingly, the periodate in aqueous auxiliary formulation C ('composition C2') was used to initiate or mediate chemical cross-linking of two or more groups E on the polymer gellant according to Formula (VIII) in the liquid hydrogel formulation ('composition C1').

Table 2: Aqueous auxiliary formulations ('composition C2') at 37 °C

| **Aqueous auxiliary formulation** ('composition C2') | **Content compound(s) used to (initiate/mediate) cross-linking** | **Water content** | **Further ingredients (content)** | **Morphology** | **pH** |
|---|---|---|---|---|---|
| A | 6.0 wt.% | 92.7 wt.% | 1.3 wt.% | Liquid | 8.7 |
| B | 2.1 wt.% | 96.9 wt.% | 1.0 wt.% | Liquid | 7.4 |
| C | 6.0 wt.% | 93.0 wt.% | 1.0 wt.% | Liquid | 7.4 |

All aqueous auxiliary formulations A, B and C ('composition C2') could be injected with a syringe equipped with a 15 G needle.

Table 3: Liquid hydrogel compositions ('composition C3') and cross-linked hydrogels ('composition C4')

| **Cross-linked hydrogel** ('comp. C4') | **Liquid hydrogel formulation** ('comp. C1') | **Aq. auxiliary formulation** ('comp. C2') | **Mole equiv. oxidator** [-] | **Time for injection (*)** [s] |
|---|---|---|---|---|
| 1 | 2 | A | 0.7 | 50 |
| 2 | 2 | A | 1.0 | 70 |
| 3 | 2 | B | 0.7 | 120 |
| 4 | 4 | A | 0.7 | 70 |
| 5 | 5 | A | 0.7 | 60 |
| 6 | 5 | B | 0.7 | 240 |
| 7 | 6 | A | 0.7 | 80 |
| 8 | 7 | A | 0.7 | 150 |
| 9 | 8 | A | 0.7 | 360 |
| 13 | 9 | C | 0.7 | 330 |
| 14 | 10 | C | 0.7 | 270 |

(continued)

| **Cross-linked hydrogel** ('comp. C4') | **Liquid hydrogel formulation** ('comp. C1') | **Aq. auxiliary formulation** ('comp. C2') | **Mole equiv. oxidator** [-] | **Time for injection (*)** [s] |
|---|---|---|---|---|
| 15 | 11 | C | 0.7 | 360 |
| (*) time for injection is the duration in which 'composition C3' can be manually injected through a 15G needle starting from the time of addition of 'composition C2' to 'composition C1', *i.e.* from the time 'composition C3' is formed | | | | |

*Rheological characterization of cross-linked hydrogels ('composition C4')*

The desired amount of aqueous auxiliary formulation A ('composition C2'), typically 0.7 - 1.0 mol equivalent of $NaIO_4$ to mono- or multihydroxyphenyl moiety, was added to a liquid hydrogel formulation ('composition C1') of Example 2 in a syringe, the mixture was shaken for 10-20 seconds and was ejected through a 15G needle onto the lower plate of a measuring cell in a plate-plate rheometer (Anton Paar) after which the upper plate was lowered and the build-up in time of the storage (G') and loss (G") moduli of the 'composition C4' was measured in a closed thermal chamber saturated with water vapour at 37 °C. Results are presented in Table 4. The elapsed time is defined as the period starting after full addition of auxiliary formulation A ('composition C2') to a liquid hydrogel formulation ('composition C1') of Example 2, *i.e.* from the formation of 'composition C3', until the value of G' equals that of G" at 1 Hz. Accordingly, the periodate in aqueous auxiliary formulation A ('composition C2') was used to initiate or mediate chemical cross-linking of two or more groups E on the polymer gellant according to Formula (VIII) in the liquid hydrogel formulation ('composition C1').

Table 4: Gelation of liquid hydrogels 'composition C3' to cross-linked hydrogels ('composition C4')

| **Cross-linked hydrogel** ('comp. C4') | **Liquid hydrogel formulation** ('comp. C1') | **Aq. auxiliary formulation** ('comp. C2') | **Mole equiv. oxidator** [-] | **Elapsed time when G' = G"** [s] |
|---|---|---|---|---|
| 10 | 5 | A | 0.7 | 150 |
| 11 | 7 | A | 0.7 | 330 |
| 12 | 8 | A | 0.7 | 700 |
| 13 | 9 | C | 0.7 | 50 |
| 14 | 10 | C | 0.7 | 230 |

**Example 4: Mechanical characterization of cross-linked hydrogels ('composition C4')**

*Tensile performance of cross-linked hydrogels ('composition C4')*

Strips were cut from films of the cross-linked hydrogels obtained after 16 h of curing in a Teflon mould in a closed environment saturated with water vapour at a temperature of 37 °C. The cross-linked hydrogels were brought to room temperature and cut in strips with typical dimensions of 30 x 4 x 2 mm (l x w x h). Their tensile performance was directly measured in order to prevent significant water evaporation. Results are depicted in Table 5. The Young's modulus ($E_{mod}$) was determined by test method ASTM D 1708-96 with a crosshead speed of 50 mm/min at 20 °C. The elongation at break and the tensile strength were determined by test method ASTM D 1708-96 with a crosshead speed of 50 mm/min at 20 °C.

Table 5: Tensile performance of the cross-linked hydrogels ('composition C4')

| **Cross-linked hydrogels** ('composition C4') | **Young's Modulus** [kPa] | **Tensile strength** [kPa] | **Elongation@break** [%] |
|---|---|---|---|
| 1 | 19 | 33 | > 200 |
| 2 | 24 | 29 | > 150 |
| 4 | 16 | 23 | > 200 |
| 5 | 30 | 25 | > 100 |
| 7 | 5 | 14 | > 300 |

*Rheological performance of the cross-linked hydrogels ('composition C4')*

Disks were punched out of films of the cross-linked hydrogels obtained after 16 h of curing in a Teflon mould in a closed environment saturated with water vapour at a temperature of 37 °C. The hydrogel disks (typically 2 - 3 mm thick) were analyzed with a plate-plate (25 mm) rheometer (Anton Paar) equipped with a closed thermal chamber saturated with water vapour at 37 °C. Results are presented in Table 6.

Table 6: Storage moduli, loss moduli and loss factors of the cross-linked hydrogels ('composition C4') at 37 °C

| **Cross-linked hydrogel** ('composition C4') | **G' @1Hz** [kPa] | **G" @1Hz** [kPa] | **Tan δ @1Hz** [-] |
|---|---|---|---|
| 1 | 8.6 | 0.57 | 0.066 |
| 2 | 8.8 | 0.54 | 0.061 |
| 5 | 12 | 0.66 | 0.057 |
| 7 | 2.2 | 0.18 | 0.081 |
| 13 | 23 | 0.13 | 0.006 |
| 14 | 33 | 0.14 | 0.004 |

*Static unconfined compression of the cross-linked hydrogels ('composition C4') at constant strain*

Disks with a diameter of 8.0 mm were punched out of films of the cross-linked hydrogels obtained after 16 h of curing in a Teflon mould in a closed environment saturated with water vapour at a temperature of at 37 °C. The hydrogel disks (typically 2 - 3 mm thick) were directly mounted on a mechanical tester for compression tests positioned in a temperature-controlled chamber filled with 0.15 M PBS solution at 37 °C. Unconfined compression was performed using 10 subsequent loading and unloading cycles whereby the loading is incrementally increased from 10% up to 95% of the sample thickness at a speed of 1 mm/s and a relaxation time of 30 seconds at the max amplitude of each cycle, while coming back to 10% strain after each stress relaxation amplitude. Maximum stress at 95% strain and the stress after 30 s relaxation at 95% strain are listed in Table 7. Additionally, the presence of cracks and/or sample deformations was assessed by visual observation of the test sample with the naked eye after completion of all loading cycles up to 95% and subsequent removal of the test sample from the testing device. Also the change in thickness of the test sample was measured after 10 minutes after completion of all loading cycles up to 95% and subsequent removal of test sample from the testing device. The remaining thickness was calculated by dividing the thickness after testing by the thickness of the sample before testing times 100%.

Table 7: Properties of cross-linked hydrogel formulations after unconfined compression to 95%

| **Cross-linked hydrogel** ('composition C4') | **Maximum Stress @95% strain** [MPa] | **Stress after 30s stress relaxation @95% strain** [MPa] | **Remaining thickness after testing** [%] | **Cracks or deformations after 95% strain cycle** |
|---|---|---|---|---|
| 4 | 2.2 | 1.1 | 98 | Not observed |
| 5 | 1.1 | 0.6 | 93 | Not observed |
| 14 | 5.4 | 5.0 | 98 | Not observed |

*Static unconfined compression of the cross-linked hydrogels ('composition C4') at constant stress*

Disks with a diameter of 8.0 mm were punched out of films of the cross-linked hydrogels obtained after 16 h of curing in a Teflon mould in a closed environment saturated with water vapour at a temperature of at 37 °C. The hydrogel disks (typically 2 - 3 mm thick) were directly mounted on a mechanical tester for compression tests positioned in a temperature-controlled chamber filled with 0.15 M PBS solution at 37 °C. Unconfined compression was performed using two subsequent loading and unloading cycles whereby the first loading cycle was to 1 MPa stress at a speed of 1 mm/s and a relaxation time of 60 seconds at the max amplitude of each cycle, followed by coming back to 0.05 MPa stress. The second loading cycle was to 2 MPa stress at a speed of 1 mm/s and a relaxation time of 60 seconds at the max amplitude of each cycle, followed by coming back to 0.05 MPa stress. The presence of cracks and/or sample deformations was assessed by visual observation of the test sample with the naked eye after completion of the two loading cycles and

subsequent removal of the test sample from the testing device. Also the change in thickness of the test sample was measured after 10 minutes after completion of all loading cycles up to 90% and subsequent removal of test sample from the testing device. The remaining thickness was calculated by dividing the thickness after testing by the thickness of the sample before testing times 100%.

Table 7A: Properties of cross-linked hydrogel formulations after unconfined compression to 1 MPa or 2 MPa

| **Cross-linked hydrogel** ('composition C4') | **Remaining stress after 60s stress relaxation and initial stress of 1 MPa** [MPa] | **Remaining stress after 60s stress relaxation and initial stress of 2 MPa** [MPa] | **Remaining thickness after testing** [%] | **Cracks or deformations after 2 MPa stress cycle** |
|---|---|---|---|---|
| 13 | 0.88 | 1.75 | 98 | Not observed |
| 14 | 0.91 | 1.76 | 98 | Not observed |

*Static confined compression of the cross-linked hydrogels ('composition C4')*

Disks with a diameter of 3.5 mm were punched out of films of the cross-linked hydrogels obtained after 16 h of curing in a Teflon mould in a closed environment saturated with water vapour at a temperature of 37 °C. The hydrogel disks (typically 2 - 3 mm thick) were directly mounted on a mechanical tester for compression tests using a 10 μm porous confined chamber (D = 3 mm) that was submerged in a temperature-controlled chamber filled with 0.15 M PBS solution at 37 °C. Confined compression was performed using 5 stress relaxation ramps of amplitude 5% from 5% to 25% cumulative strain. After each ramp the sample was given time for stress relaxation until the decrease in force was less than 0.1 N/min (typically 20 - 60 s). Maximum stress at 25% strain and the stress at equilibrium after relaxation at 25% strain are listed in Table 8. Additionally, the equilibrium modulus as extracted from the linear region of the stress-strain curves in confined compression is given.

Table 8: Properties of cross-linked hydrogels after confined compression to 25%

| **Cross-linked hydrogel** ('composition C4') | **Maximum Stress @25% strain** [MPa] | **Equilibrium stress @25% strain** [MPa] | **Equilibrium modulus** [MPa] |
|---|---|---|---|
| 4 | 0.84 | 0.33 | 1.5 |
| 5 | 0.26 | 0.11 | 0.5 |

The high compression strains and stresses that these cross-linked hydrogels can withstand without failure clearly show that these cross-linked hydrogels display a cushioning action that can be used inside a joint.

**Example 5: *Ex vivo* injection of liquid hydrogel compositions ('composition C3')**

Liquid hydrogel compositions ('composition C3') were injected in six *ex-vivo* experiments. Liquid hydrogel compositions ('composition C3') were prepared as described in Example 3, warmed to approximately 37 °C and injected into the cavity of synovial joints within 1-2 minutes from mixing the liquid hydrogel formulation ('composition C1') and the aqueous auxiliary formulation ('composition C2'). Injection took place through the skin and soft tissue into the cavity of synovial joints. Further details and results of the *ex-vivo* injections are depicted in Table 9.

After 1 hour, the thus treated synovial joints were dissected to inspect the synovial cavity and the cross-linked hydrogel ('composition C4') therein. It was concluded that cross-linked hydrogels, with sufficient mechanical strength to provide a cushioning effect, were positioned in the synovial cavities. No adhesion of the cross-linked hydrogel formulations to cartilage material was observed. Accordingly, it can be concluded from this *ex vivo* study that the liquid hydrogel compositions ('composition C3') of the invention can be injected into the cavity of synovial joints and that the material forms cross-linked hydrogels ('composition C4') that stay in the synovial joint and that provide a cushioning effect (internal distraction and shock absorption) in the synovial joint. Hence, the disclosed method can be used for the treatment or prevention of osteoarthritis.

In five comparative *ex-vivo* experiments, a commercial injectable hyaluronic acid solution (Hyalur, Croma-Pharma GmbH) was warmed to approximately 37 °C and injected into the cavity of synovial joints. Injection took again place through the skin and soft tissue into the cavity of the synovial joints. Further details and results of the *ex-vivo* injections are presented in Table 9.

After 1 hour, the thus treated synovial joints were dissected to inspect the synovial cavity and the hyaluronic acid

solution therein. It was concluded that the injected hyaluronic acid solution had turned into a sticky viscous liquid without any elastic or compressive strength and without any load bearing capacity. The sticky viscous liquid leaked from the joint upon opening of the synovial joint cavity.

## Example 6: *In vivo* injection of liquid hydrogel compositions ('composition C3')

Liquid hydrogel compositions ('composition C3') were injected in three *in-vivo* experiments into the knee of a goat or into the knee of a rabbit. Liquid hydrogel compositions ('composition C3') were prepared as described in Example 3, warmed to approximately 37 °C and injected into the cavity of synovial joints within 1-2 minutes from mixing the liquid hydrogel formulation ('composition C1') and the aqueous auxiliary formulation ('composition C2'). Injection took place through the skin and soft tissue into the cavity of synovial joints. Further details and results of the *in-vivo* injections are presented in Table 10.

In case of the goats, the animal was sacrificed 4 hours after injection and the thus treated synovial joints were dissected to inspect the synovial cavity and the cross-linked hydrogel ('composition C4') therein. It was concluded that cross-linked hydrogels, with sufficient mechanical strength to provide a cushioning effect, were positioned in the synovial cavities. No adhesion of the cross-linked hydrogel formulations to cartilage material was observed. In case of the rabbits, the animal was sacrificed 4 or 10 days after injection and the thus treated synovial joints were dissected to inspect the synovial cavity and the cross-linked hydrogel ('composition C4') therein. It was concluded that cross-linked hydrogels, with sufficient mechanical strength to provide a cushioning effect, were positioned in the synovial cavities. No adhesion of the cross-linked hydrogel formulations to cartilage material was observed

Accordingly, it can be concluded from this *in vivo* study that the liquid hydrogel compositions ('composition C3') of the invention can be injected into the cavity of synovial joints and that the material forms cross-linked hydrogels ('composition C4') that stay in the synovial joint and that provide a cushioning effect (internal distraction and shock absorption) in the synovial joint. Hence, the disclosed method can be used for the treatment or prevention of osteoarthritis.

## Example 7: Drug release from cross-linked hydrogel ('composition C4')

A liquid hydrogel composition ('composition C3') comprising liquid hydrogel formulation 9, aqueous auxiliary formulation C and 0.05 wt.% deprotonated ibuprofen (isobutylphenylpropionic acid), an example of a nonsteroidal anti-inflammatory drug, was injected into a vial (200-300 mg). After 16 hours of curing, PBS solution was added to the vial in an amount equal to 10 times the weight of the cross-linked hydrogel ('composition C4') comprising 0.05 wt.% ibuprofen and the mixture was kept at 37 °C. The PBS supernatant was removed and replaced by fresh PBS several times. The hydrogel remained intact and did not dissolve during the full experiment. The removed supernatants were analyzed with UV/Vis spectroscopy on the presence of ibuprofen by monitoring its absorption at 225 nm corrected for the absorption values obtained from the same cross-linked hydrogel but not loaded with ibuprofen that was tested in the same way. Results are depicted in Table 11. The cumulative release (fraction of all ibuprofen released at that time point) clearly increases with time. After 8 h, 61% of all the ibuprofen has been released and after 32 h, 72% has been released with 28% still in the hydrogel.

Table 9: *Ex vivo* injection of liquid hydrogel compositions

| Sample | Liquid hydrogel formulation (C1) | Aq. auxiliary formulation (C2) / mole equiv. oxidator | Joint (medical term)/origin | Injected volume (mL) | Method of injection | Method of guidance | Hydrogel localization upon dissection | Further observations upon dissection |
|---|---|---|---|---|---|---|---|---|
| 8 | 5 | A/0.7 | Ankle (talocrural)/ human | 10 | Needle and single-chamber syringe | 2-mm diameter arthroscopy | Intra-articular | - Hydrogel evenly spread in joint with no disruptions<br>- Hydrogel did not adhere to cartilage<br>- Hydrogel was elastic, non-tacky, ductile and did not fracture upon manual compression<br>- Hydrogel remained in place upon opening of the joint cavity and moving of the joint<br>- Hydrogel did not spread outside the joint |
| 9 | 5 | A/0.7 | Ankle (talocrural) / human | 10 | 2-mm diameter arthroscope and single-chamber syringe | - | Intra-articular | - Hydrogel evenly spread in joint with no disruptions<br>- Hydrogel did not adhere to cartilage<br>- Hydrogel was elastic, non-tacky, ductile and did not fracture upon manual compression<br>- Hydrogel remained in place upon opening of the joint cavity and moving of the joint |

| | | | | | | | | |
|---|---|---|---|---|---|---|---|---|
| | | | | | | | | - Hydrogel did not spread outside the joint |
| 10 | 5 | A/0.7 | Ankle (talocrural) / human | 10 | Needle and single-chamber syringe | Palpation | Intra-articular | - Hydrogel evenly spread in joint with no disruptions<br>- Hydrogel did not adhere to cartilage<br>- Hydrogel was elastic, non-tacky, ductile and did not fracture upon manual compression<br>- Hydrogel remained in place upon opening of the joint cavity and moving of the joint<br>- Hydrogel did not spread outside the joint |
| 11 | 5 | A/0.7 | Ankle / goat | 5 | Needle and single-chamber syringe | 2-mm diameter arthroscopy | -Intra-articular | - Hydrogel evenly spread in joint with no disruptions<br>- Hydrogel did not adhere to cartilage<br>- Hydrogel was elastic, non-tacky, ductile and did not fracture upon manual compression<br>- Hydrogel remained in place upon opening of the joint cavity and moving of the joint<br>- Hydrogel did not spread outside the joint |
| 12 | 5 | A/0.7 | Ankle / goat | 5 | Needle and single-chamber syringe | 2-mm diameter arthroscopy | Intra-articular | - Hydrogel evenly spread in joint with no disruptions<br>- Hydrogel did not adhere to cartilage<br>- Hydrogel was elastic, non-tacky, ductile and did not fracture upon manual compression<br>- Hydrogel remained in place upon opening of the joint cavity and moving of the joint<br>- Hydrogel did not spread outside the joint |

| | | | | | | | | |
|---|---|---|---|---|---|---|---|---|
| 13 | 5 | A/0.7 | Ankle / goat | 10 | Needle and single-chamber syringe | 2-mm diameter arthroscopy | Intra-articular | - Hydrogel evenly spread in joint with no disruptions<br>- Hydrogel did not adhere to cartilage<br>- Hydrogel was elastic, non-tacky, ductile and did not fracture upon manual compression<br>- Hydrogel remained in place upon opening of the joint cavity and moving of the joint<br>- Hydrogel did not spread outside the joint |
| 14 | 5 | A/0.7 | Ankle / goat | 10 | Needle and single-chamber syringe | 2-mm diameter arthroscopy | Intra-articular | - Hydrogel evenly spread in joint with no disruptions<br>- Hydrogel did not adhere to cartilage<br>- Hydrogel was elastic, non-tacky, ductile and did not fracture upon manual compression<br>- Hydrogel remained in place upon opening of the joint cavity and moving of the joint<br>- Hydrogel did not spread outside the joint |
| 15 (Comp.) | Hyaluronic acid | Not applicable | Ankle (talocrural)/ human | 2 | Needle and single-chamber syringe | Palpation | Intra-articular | Sticky viscous liquid<br>- Leaked from joint upon opening of the joint capsule<br>- Not elastic, no compressive strength<br>- No load bearing capacity |
| 16 (Comp.) | Hyaluronic acid | Not applicable | Ankle (talocrural) / human | 2 | Needle and single- | Palpation | Intra-articular | - Sticky viscous liquid<br>- Leaked from joint upon opening of the joint capsule<br>- Not elastic, no compressive strength |

| | | | | | | | | |
|---|---|---|---|---|---|---|---|---|
| | | | | | chamber syringe | | | - No load bearing capacity |
| 17 (Comp.) | Hyalur. acid | Not applicable | Ankle (talocrural) / human | 2 | Needle and single-chamber syringe | Palpation | Intra-articular | - Sticky viscous liquid<br>- Leaked from joint upon opening of the joint capsule<br>- Not elastic, no compressive strength<br>- No load bearing capacity |
| 18 (Comp.) | Hyalur. acid | Not applicable | Ankle (talocrural) / human | 2 | Needle and single-chamber syringe | Palpation | Intra-articular | - Sticky viscous liquid<br>- Leaked from joint upon opening of the joint capsule<br>- Not elastic, no compressive strength<br>- No load bearing capacity |
| 19 (Comp.) | Hyalur. acid | Not applicable | Ankle (talocrural) / human | 2 | Needle and single-chamber syringe | Palpation | Intra-articular | - Sticky viscous liquid<br>- Leaked from joint upon opening of the joint capsule<br>- Not elastic, no compressive strength<br>- No load bearing capacity |

Table 10: *In vivo* injection of liquid hydrogel compositions

| Sample | Liquid hydrogel formulation (C1) | Aq. auxiliary formulation (C2) / mole equiv. oxidator | Joint / origin | Injected volume (mL) | Method of injection | Method of guidance | Time of implantation | Hydrogel localization upon dissection | Further observations upon dissection |
|---|---|---|---|---|---|---|---|---|---|
| 1 | 5 | A/0.7 | Knee / goat | 12 | Needle and single-chamber syringe | Palpation | 4 hours | Intra-articular | - Hydrogel fully spread in entire joint cavity with no disruptions<br>- Hydrogel did not adhere to cartilage<br>- Hydrogel was elastic, non-tacky, ductile and did not fracture upon manual compression<br>- Hydrogel remained in place upon opening of the joint cavity and moving of the joint<br>- Hydrogel did not spread outside the joint cavity |
| 2 | 10 | A/0.7 | Knee / rabbit | 4 | Needle and single-chamber syringe | palpation | 4 days | Intra-articular | - Hydrogel fully spread in entire joint cavity with no disruptions<br>- Hydrogel did not adhere to cartilage<br>- Hydrogel was elastic, non-tacky, ductile and did not fracture upon manual compression<br>- Hydrogel remained in place upon opening of the joint cavity and moving of the joint |

| | | | | | | | | | |
|---|---|---|---|---|---|---|---|---|---|
| | | | | | | | | | - Hydrogel did not spread outside the joint cavity<br>- Compression strength of explant > 1 MPa at 90% compression (20 °C) |
| 3 | 10 | A/0.7 | Knee / rabbit | 4 | Needle and single-chamber syringe | palpation | 10 days | Intra-articular | - Hydrogel fully spread in entire joint cavity with no disruptions<br>- Hydrogel did not adhere to cartilage<br>- Hydrogel was elastic, non-tacky, ductile and did not fracture upon manual compression<br>- Hydrogel remained in place upon opening of the joint cavity and moving of the joint<br>- Hydrogel did not spread outside the joint cavity<br>- Compression strength of explant > 1 MPa at 90% compression (20 °C) |

Table 11: Cumulative release at 37°C of ibuprofen from cross-linked hydrogel comprising 0.05 w% ibuprofen

| **Time (h)** | 1 | 2 | 4 | 6 | 8 | 22 | 32 |
|---|---|---|---|---|---|---|---|
| **Cumulative release (%)** | 47 | 49 | 53 | 58 | 61 | 68 | 72 |

## Claims

**1.** A kit of parts, consisting of compositions (C1) and (C2) in separate containers, for use in the treatment or prevention of osteoarthritis in a vertebrate, wherein:

(a) composition (C1) is a liquid hydrogel formulation comprising 50.0 - 99.7 wt.% of water, based on the weight of composition (C1), 0.3 - 50.0 wt.% of a polymer gellant according to Formula (VIII) and 0 - 20 wt.% of further ingredients:

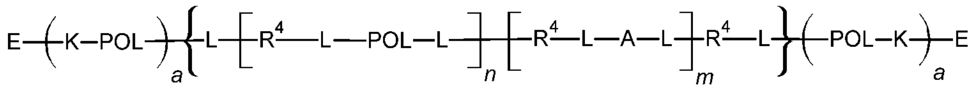

(VIII)

wherein:

- *m* and *n* are each integers independently in the range of 1 to 20;
- *a* is an integer chosen from 0 or 1;
- K is a linking group selected from amide, ester or ether;
- E is a group that can react with another group E initiated or mediated by one or more compounds Y, wherein the one or more compounds Y are selected from the group consisting of oxidant, oxidase, peroxidase and combinations thereof, in aqueous auxiliary formulation (C2), thereby forming one or more covalent bonds between different groups E, or E is a group that can react with one or more cross-linkers Z in aqueous auxiliary formulation (C2), wherein the one or more cross-linkers Z have two or more reactive groups, thereby forming covalent bonds between E and the one or more cross-linkers Z;
- POL is a linear polymeric group having an average molecular weight $M_n$, as determined by end group titration, of about 250 to about 30000 Da;
- A represents moieties selected from the group consisting of Formulas (I-B) to (V-B), wherein A is connected to L via the bonds marked with an asterisk:

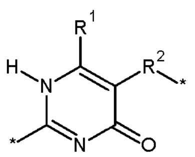

(I-B)

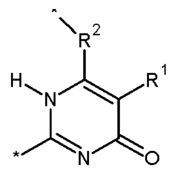

(II-B)

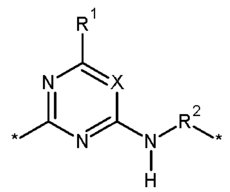

(III-B)

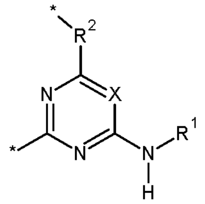

(IV-B)

$*-R^3-*$ (V-B)

- X is N or $CR^1$;
- each L independently is a urethane or urea linking group, with the proviso that any moiety A according to Formula (I-B) and (II-B) is always coupled to a urea linking group L on the 2-position of the 4-pyrimidone, any moiety A according to Formula (III-B) and (IV-B) is always coupled to a urea linking group L on the 4-position of the 1,3,5-triazine for X=N, or on the 2-position of the pyrimidine for X is $CR^1$, and any moiety A according to Formula (V-B) is always coupled to two urea linking groups L;
- $R^1$ is selected from the group consisting of hydrogen and $C_1$ - $C_{20}$ alkyl;
- $R^2$ is selected from the group consisting of $C_1$ - $C_{20}$ alkylene;
- $R^3$ is selected from the group consisting of a direct covalent bond, linear $C_1$ - $C_{24}$ alkylene groups, branched $C_2$ - $C_{24}$ alkylene groups, cyclic $C_3$ - $C_{24}$ alkylene groups, $C_6$ - $C_{24}$ arylene groups, $C_7$ - $C_{24}$ alkarylene groups and $C_7$ - $C_{24}$ arylalkylene groups, wherein the alkylene groups, arylene groups, alkarylene groups and arylalkylene groups optionally comprise 1 - 5 heteroatoms selected from the group consisting of O, N and S; and
- $R^4$ is selected from the group consisting of linear or branched $C_2$ - $C_{20}$ alkylene groups and cyclic $C_3$ - $C_{24}$ alkylene groups; and

(b) composition (C2) is an aqueous auxiliary formulation comprising 75.0 - 99.9 wt.% of water, based on the weight of composition (C2), 0 - 5 wt.% of further ingredients and 0.1 - 20 wt.% of:

- one or more dissolved cross-linkers Z having two or more reactive groups that can chemically cross-link the polymer gellant according to Formula (VIII) by forming covalent bonds with two or more groups E; or
- one or more dissolved compounds Y, selected from the group consisting of oxidant, oxidase, peroxidase and combinations thereof, that initiate or mediate chemical cross-linking of two or more groups E on the polymer gellant according to Formula (VIII);

wherein said treatment comprises combining compositions (C1) and (C2) to form a liquid hydrogel composition (C3) and administering said liquid hydrogel composition (C3) to the synovial fluid or to the cavity of a synovial joint of the vertebrate, to form a cross-linked hydrogel (C4) in the cavity of the synovial joint of the vertebrate.

**2.** A kit of parts, consisting of compositions (C1) and (C2) in separate containers, for use in the treatment or prevention of osteoarthritis in a vertebrate, wherein

(a) composition (C1) is a liquid hydrogel formulation comprising 50.0 - 99.7 wt.% of water, based on the weight of composition (C1), 0.3 - 50.0 wt.% of a polymer gellant according to Formula (VIII) and 0 - 20 wt.% of further ingredients:

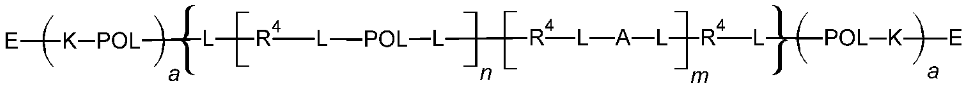

(VIII)

wherein:

- *m* and *n* are each integers independently in the range of 1 to 20;
- *a* is an integer chosen from 0 or 1;
- K is a linking group selected from amide, ester or ether;
- E is a group that can react with another group E initiated or mediated by one or more compounds Y, wherein the one or more compounds Y are selected from the group consisting of oxidant, oxidase, peroxidase and combinations thereof, in aqueous auxiliary formulation (C2), thereby forming one or more covalent bonds between different groups E, or E is a group that can react with one or more cross-linkers Z in aqueous auxiliary formulation (C2), wherein the one or more cross-linkers Z have two or more reactive groups, thereby forming covalent bonds between E and the one or more cross-linkers Z;
- POL is a linear polymeric group having an average molecular weight $M_n$, as determined by end group titration, of about 250 to about 30000 Da;
- A represents moieties selected from the group consisting of Formulas (I-B) to (V-B), wherein A is connected to L via the bonds marked with an asterisk:

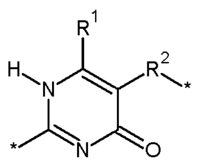

(I-B)

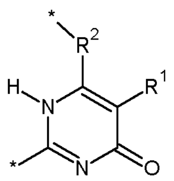

(II-B)

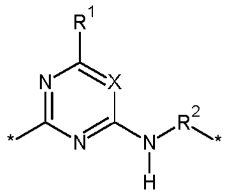

(III-B)

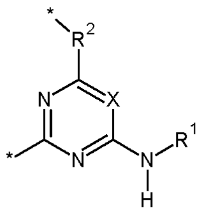

(IV-B)

$*\text{-}R^3\text{-}*$ (V-B)

- X is N or $CR^1$;
- each L independently is a urethane or urea linking group, with the proviso that any moiety A according to Formula (I-B) and (II-B) is always coupled to a urea linking group L on the 2-position of the 4-pyrimidone, any moiety A according to Formula (III-B) and (IV-B) is always coupled to a urea linking group L on the 4-position of the 1,3,5-triazine for X=N, or on the 2-position of the pyrimidine for X is $CR^1$, and any moiety A according to Formula (V-B) is always coupled to two urea linking groups L;
- $R^1$ is selected from the group consisting of hydrogen and $C_1$ - $C_{20}$ alkyl;
- $R^2$ is selected from the group consisting of $C_1$ - $C_{20}$ alkylene;
- $R^3$ is selected from the group consisting of a direct covalent bond, linear $C_1$ - $C_{24}$ alkylene groups, branched $C_2$ - $C_{24}$ alkylene groups, cyclic $C_3$ - $C_{24}$ alkylene groups, $C_6$ - $C_{24}$ arylene groups, $C_7$ - $C_{24}$ alkarylene groups and $C_7$ - $C_{24}$ arylalkylene groups, wherein the alkylene groups, arylene groups, alkarylene groups and arylalkylene groups optionally comprise 1 - 5 heteroatoms selected from the group consisting of O, N and S; and
- $R^4$ is selected from the group consisting of linear or branched $C_2$ - $C_{20}$ alkylene groups and cyclic $C_3$ - $C_{24}$

alkylene groups; and

(b) composition (C2) is an aqueous auxiliary formulation comprising 75.0 - 99.9 wt.% of water, based on the weight of composition (C2), 0 - 5 wt.% of further ingredients and 0.1 - 20 wt. % of:

• one or more dissolved cross-linkers Z having two or more reactive groups that can chemically cross-link the polymer gellant according to Formula (VIII) by forming covalent bonds with two or more groups E; or
• one or more dissolved compounds Y, selected from the group consisting of oxidant, oxidase, peroxidase and combinations thereof, that initiate or mediate chemical cross-linking of two or more groups E on the polymer gellant according to Formula (VIII);

wherein said treatment comprises in a first step administering one of the compositions (C1) or (C2), preferably composition (C1), to the synovial fluid or to the cavity of a synovial joint of the vertebrate and in a second step administering the other of the compositions (C1) or (C2), to form a liquid hydrogel composition (C3) in the cavity of the synovial joint of the vertebrate that reacts to a cross-linked hydrogel (C4).

**3.** Kit of parts for use according to claim 1 or 2, wherein the polymer gellant according to Formula (VIII) in composition (C1) is obtainable by a process wherein:

(i) one or more compounds A' selected from the group consisting of Formulas (I-A) to (V-A):

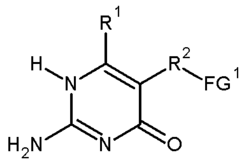

(I-A)

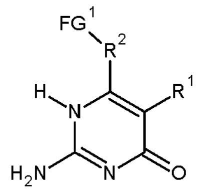

(II-A)

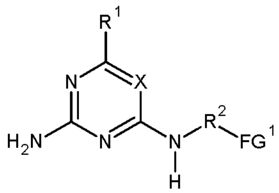

(III-A)

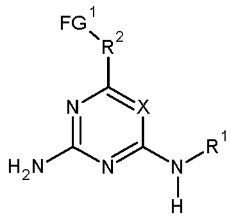

(IV-A)

$H_2N\text{-}R^3\text{-}NH_2$ (V-A)

are reacted, optionally in a solvent, with a diisocyanate compound according to Formula $OCN\text{-}R^4\text{-}NCO$ and a polymer according to Formula $FG^1\text{-}POL\text{-}FG^1$ to form an intermediate diisocyanate product with Formula (VI):

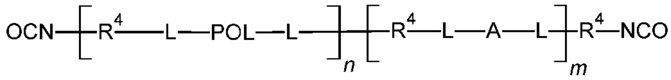

(VI)

wherein:

• *m, n,* X, $R^1$, $R^2$, $R^3$, $R^4$, POL, A, and L are as defined in claim 1 or claim 2;
• each $FG^1$ is a functional group independently selected from OH and $N(R^1)H$;

(ii) reacting the intermediate diisocyanate product of Formula (VI) with a compound according to formula (VII):

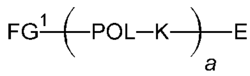

(VII)

wherein a, K and E are as defined in claim 1 or 2, to provide the polymer gellant according to Formula (VIII).

4. Kit of parts for use according to any one of claims 1 - 3, wherein composition (C1) comprises one or more further ingredients, wherein said one or more further ingredients are selected from the group consisting of anti-inflammatory drugs, corticosteroids, local anesthetics and combinations thereof.

5. Kit of parts for use according to any one of claims 1 - 3, wherein composition (C1) comprises one or more further ingredients, wherein said one or more further ingredients are selected from the group consisting of peptides, proteins, antimicrobial agents, anti-viral agents, hormones, paracrine factors, growth factors, vitamins, anti-inflammatory drugs, corticosteroids, local anesthetics and combinations thereof.

6. Kit of parts for use according to any one of claims 1 - 4, wherein compositions (C1) and (C2) do not comprise:

- ingredients derived from animal or human tissue; and/or
- cells, cell extracts, proteins, glycosaminoglycans and biologically derived macromolecules; and/or
- stem cells and growth factors.

7. Kit of parts for use according to any one of claims 4 - 6 wherein composition (C1) comprises the one or more further ingredients in an amount of 0.0005 to 10 wt.%, based on the weight of composition (C1), preferably 0.0005 to 1 wt.%, most preferably 0.005 to 1 wt.%

8. Kit of parts for use according to any one of claims 1 - 7, wherein POL is selected from the group consisting of polyethers, polyesters, polycarbonates, polyamides, polyoxazolines, polyacrylates, polymethacrylates, polyolefins, hydrogenated polyolefins, polysiloxanes, polycarbonates, (per)fluorinated polyethers, polyvinylenes, or co-polymers of such polymers, and combinations thereof, preferably wherein POL is selected from polyethylene glycols and polyoxazolines.

9. Kit of parts for use according to any one of claims 1 - 8, wherein A Formula (VIII) is:

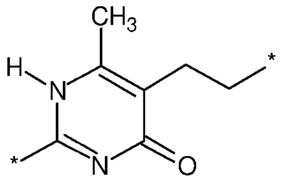

and wherein *-L-A-L-* is:

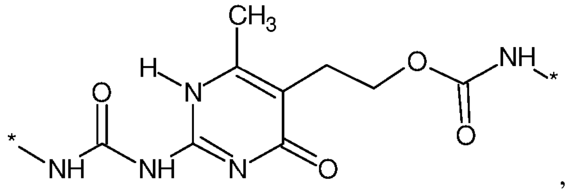

,

or
wherein *-L-A-L-* is:

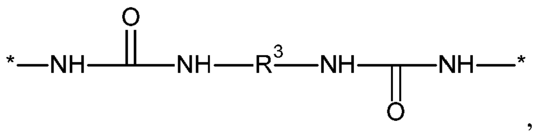

wherein $R^3$ is selected from the group consisting of a covalent bond, linear $C_2$ - $C_{12}$ alkylene groups and cyclic $C_3$ - $C_{12}$ alkylene.

**10.** Kit of parts for use according to any one of claims 1 - 9, wherein

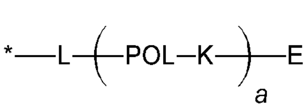

in the polymer gellant of Formula (VIII) is

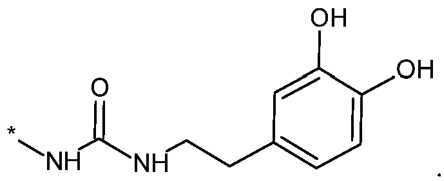

**11.** Kit of parts for use according to any one of claims 1 - 10, wherein the liquid hydrogel formulation of composition (C1) has a dynamic viscosity at 37°C of about 0.01 to about 20 Pa·s, as measured with a rheometer with a plate-plate geometry at a shear rate of 1 $s^{-1}$ and with a gap distance of 0.50 mm, and/or wherein the amount of water in the liquid hydrogel composition (C3) or in the cross-linked hydrogel (C4) is between 51.0 and 99.9 wt.% by weight of the composition or the cross-linked hydrogel, respectively, wherein the amount of the polymer gellant according to Formula (VIII) in the liquid hydrogel composition (C3) or in the cross-linked hydrogel (C4) is between 0.1 - 49.0 wt.% by weight of the composition or the cross-linked hydrogel, respectively, and wherein the amount of the further ingredients in the liquid hydrogel composition (C3) or in the cross-linked hydrogel (C4) is between 0 and 19 wt.% by weight of the composition or the cross-linked hydrogel, respectively.

**12.** Kit of parts for use according to any one of claims 1 - 11, wherein said treatment or prevention comprises removal of synovial fluid from the cavity of the synovial joint of the vertebrate before injecting compositions (C1) and (C2), or (C3), and/or wherein said treatment or prevention comprises administering 0.5 - 250 mL of the combined compositions (C1) and (C2) or of composition (C3), preferably 3 - 60 mL, more preferably 6 - 60 mL.

**13.** Kit of parts for use according to any one of claims 1 - 12, wherein the synovial joint of the vertebrate is chosen from the group consisting of shoulder girdle, elbow, wrist, hand and fingers, pelvic girdle, knee, ankle, foot and toes, jaw and trunk joints, and/or wherein the vertebrate is chosen from the group consisting of humans, aves, companion animals, racing animals and cattle.

**14.** Kit of parts for use according to any one of claims 1 - 13, wherein the polymer gellant according to Formula (VIII) has an average molecular weight $M_n$, as determined using SEC with a GPC-system equipped with a KD-804 column (Showa Denko, 500 - 400000 Da) using RI detection with DMF comprising 10 mM LiBr as eluent at a flow rate of 1.0 mL/min at 50 °C, with the SEC-data being relative to PEO/PEG-standards, of between 1 and 40 kDa, preferably between 1.2 and 20 kDa, and/or wherein the pH of Composition (C1) is between 3 and 6.5, and wherein the pH of Composition (C2) is between 8 and 10, and/or wherein the liquid hydrogel formulation of composition (C1) and (C2) or (C3) are administered using a technique chosen from the group consisting of injection, arthroscopic surgery and open surgery, preferably injection.

**15.** Kit of parts for use according to any one of claims 1 - 14, wherein the cross-linked hydrogel (C4), after 16 h of curing, has at least one of the following properties (i) to (iii):

i) no failure at an unconfined compression of 4.0 MPa at 37 °C;
ii) no failure at an unconfined compression to at least 10% of its original thickness at 37 °C;

iii) a maximum stress of at least 0.4 MPa at 25% strain in confined compression at 37 °C.

**Patentansprüche**

**1.** Kit aus Teilen, bestehend aus den Zusammensetzungen (C1) und (C2) in getrennten Behältern, zur Verwendung bei einer Behandlung oder Vorbeugung von Osteoarthritis bei einem Wirbeltier, wobei:

(a) Zusammensetzung (C1) eine flüssige Hydrogelformulierung ist, umfassend zu 50,0-99,7 Gew.-% Wasser, basierend auf dem Gewicht der Zusammensetzung (C1), zu 0,3-50,0 Gew.-% ein Polymergelierungsmittel gemäß Formel (VIII) und zu 0-20 Gew.-% weitere Inhaltsstoffe:

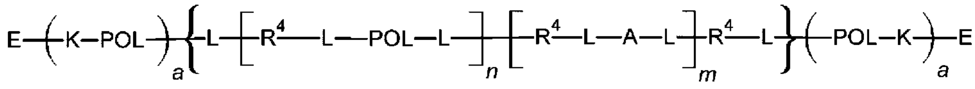

(VIII)

wobei:

- m und *n* jeweils unabhängig voneinander ganze Zahlen in dem Bereich von 1 bis 20 sind;
- a eine ganze Zahl ist, die aus 0 oder 1 gewählt ist;
- K eine Verknüpfungsgruppe ist, die aus Amid, Ester oder Ether ausgewählt ist;
- E eine Gruppe ist, die mit einer anderen Gruppe E, die durch eine oder mehrere Verbindungen Y initiiert oder vermittelt wird, wobei die eine oder die mehreren Verbindungen Y aus der Gruppe ausgewählt sind, bestehend aus Oxidationsmittel, Oxidase, Peroxidase und Kombinationen davon, in wässriger Hilfsstoffformulierung (C2) reagieren kann, wodurch eine oder mehrere kovalente Bindungen zwischen unterschiedlichen Gruppen E ausgebildet werden, oder E eine Gruppe ist, die mit einem oder mehreren Vernetzern Z in wässriger Hilfsstoffformulierung (C2) reagieren kann, wobei der eine oder die mehreren Vernetzer Z zwei oder mehr reaktive Gruppen aufweisen, wodurch kovalente Bindungen zwischen E und dem einen oder den mehreren Vernetzern Z ausgebildet werden;
- POL eine lineare Polymergruppe ist, die ein durchschnittliches Molekulargewicht $M_n$, wie durch Endgruppentitration bestimmt, von etwa 250 bis etwa 30.000 Da aufweist;
- A Einheiten darstellt, die aus der Gruppe ausgewählt sind, bestehend aus den Formeln (I-B) bis (V-B), wobei A über die Bindungen, die mit einem Sternchen markiert sind, mit L verbunden ist:

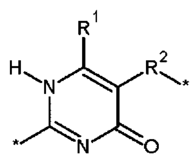

(I-B)

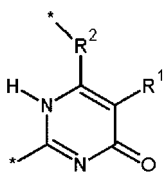

(II-B)

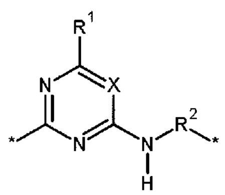

(III-B)

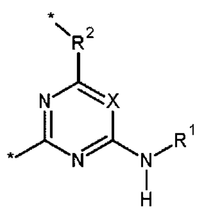

(IV-B)

*-$R^3$-* (V-B)

• X N oder CR1 ist;

• jedes L unabhängig eine Urethan- oder Harnstoff-Verknüpfungsgruppe ist, mit der Maßgabe, dass eine beliebige Einheit A gemäß Formel (I-B) und (II-B) immer an eine Harnstoff-Verknüpfungsgruppe L an der 2-Position des 4-Pyrimidons gekoppelt ist, eine beliebige Einheit A gemäß Formel (III-B) und (IV-B) immer an eine Harnstoff-Verknüpfungsgruppe L an der 4-Position des 1,3,5-Triazins für X=N gekoppelt ist oder an der 2-Position des Pyrimidins für X $CR^1$ ist, und eine beliebige Einheit A gemäß Formel (V-B) immer an zwei Harnstoff-Verknüpfungsgruppen L gekoppelt ist;

• $R^1$ aus der Gruppe ausgewählt ist, bestehend aus Wasserstoff und $C_1$-$C_{20}$-Alkyl;

• $R^2$ aus der Gruppe ausgewählt ist, bestehend aus $C_1$-$C_{20}$-Alkylen;

• $R^3$ aus der Gruppe ausgewählt ist, bestehend aus einer direkten kovalenten Bindung, linearen $C_1$-$C_{24}$-Alkylengruppen, verzweigten $C_2$-$C_{24}$-Alkylengruppen, cyclischen $C_3$-$C_{24}$-Alkylengruppen, $C_6$-$C_{24}$-Arylengruppen, $C_7$-$C_{24}$-Alkarylengruppen und $C_7$-$C_{24}$-Arylalkylengruppen, wobei die Alkylengruppen, Arylengruppen, Alkarylengruppen und Arylalkylengruppen optional 1-5 Heteroatome umfassen, die aus der Gruppe ausgewählt sind, bestehend aus O, N und S; und

• $R^4$ aus der Gruppe ausgewählt ist, bestehend aus linearen oder verzweigten $C_2$-$C_{20}$-Alkylengruppen und cyclischen $C_3$-$C_{24}$-Alkylengruppen; und

(b) Zusammensetzung (C2) eine wässrige Hilfsstoffformulierung ist, umfassend zu 75,0-99,9 Gew.-% Wasser, basierend auf dem Gewicht der Zusammensetzung (C2), zu 0-5 Gew.-% weitere Inhaltsstoffe und zu 0,1-20 Gew.-%:

• einen oder mehrere gelöste Vernetzer Z, die zwei oder mehr reaktive Gruppen aufweisen, die das Polymergelierungsmittel gemäß Formel (VIII) durch Ausbilden kovalenter Bindungen mit zwei oder mehr Gruppen E chemisch vernetzen können; oder

• eine oder mehrere gelöste Verbindungen Y, die aus der Gruppe ausgewählt sind, bestehend aus Oxidationsmittel, Oxidase, Peroxidase und Kombinationen davon, die eine chemische Vernetzung von zwei oder mehr Gruppen E auf dem Polymergeliermittel gemäß Formel (VIII) initiieren oder vermitteln;

wobei die Behandlung ein Kombinieren der Zusammensetzungen (C1) und (C2), um eine flüssige Hydrogelzusammensetzung (C3) auszubilden, und ein Verabreichen der flüssigen Hydrogelzusammensetzung (C3) in die Synovialflüssigkeit oder in den Hohlraum eines Synovialgelenks des Wirbeltiers umfasst, um in dem Hohlraum des Synovialgelenks des Wirbeltiers ein vernetztes Hydrogel (C4) auszubilden.

**2.** Kit aus Teilen, bestehend aus den Zusammensetzungen (C1) und (C2) in getrennten Behältern, zur Verwendung bei der Behandlung oder Vorbeugung von Osteoarthritis bei einem Wirbeltier, wobei

(a) Zusammensetzung (C1) eine flüssige Hydrogelformulierung ist, umfassend zu 50,0-99,7 Gew.-% Wasser, basierend auf dem Gewicht der Zusammensetzung (C1), zu 0,3-50,0 Gew.-% ein Polymergelierungsmittel gemäß Formel (VIII) und zu 0-20 Gew.-% weitere Inhaltsstoffe:

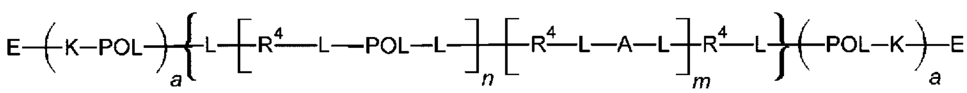

(VIII)

wobei:

• m und *n* jeweils unabhängig voneinander ganze Zahlen in dem Bereich von 1 bis 20 sind;

• a eine ganze Zahl ist, die von 0 oder 1 gewählt ist;

• K eine Verknüpfungsgruppe ist, die aus Amid, Ester oder Ether ausgewählt ist;

• E eine Gruppe ist, die mit einer anderen Gruppe E, die durch eine oder mehrere Verbindungen Y initiiert oder vermittelt wird, wobei die eine oder die mehreren Verbindungen Y aus der Gruppe ausgewählt sind, bestehend aus Oxidationsmittel, Oxidase, Peroxidase und Kombinationen davon, in wässriger Hilfsstoffformulierung (C2) reagieren kann, wodurch eine oder mehrere kovalente Bindungen zwischen unterschiedlichen Gruppen E ausgebildet werden, oder E eine Gruppe ist, die mit einem oder mehreren Vernetzern Z in wässriger Hilfsstoffformulierung (C2) reagieren kann, wobei der eine oder die mehreren Vernetzer Z zwei

oder mehr reaktive Gruppen aufweisen, wodurch kovalente Bindungen zwischen E und dem einen oder den mehreren Vernetzern Z ausgebildet werden;

• POL eine lineare Polymergruppe ist, die ein durchschnittliches Molekulargewicht $M_n$, wie durch Endgruppentitration bestimmt, von etwa 250 bis etwa 30.000 Da aufweist;

• A Einheiten darstellt, die aus der Gruppe ausgewählt sind, bestehend aus den Formeln (I-B) bis (V-B), wobei A über die Bindungen, die mit einem Sternchen markiert sind, mit L verbunden ist:

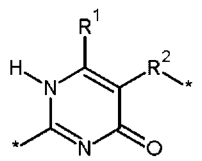

(I-B)

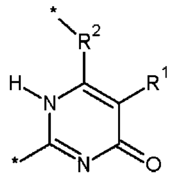

(II-B)

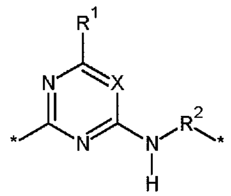

(III-B)

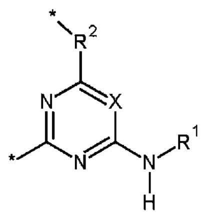

(IV-B)

$*-R^3-*$ (V-B)

• X N oder $CR^1$ ist;

• jedes L unabhängig eine Urethan- oder Harnstoff-Verknüpfungsgruppe ist, mit der Maßgabe, dass eine beliebige Einheit A gemäß Formel (I-B) und (II-B) immer an eine Harnstoff-Verknüpfungsgruppe L an der 2-Position des 4-Pyrimidons gekoppelt ist, eine beliebige Einheit A gemäß Formel (III-B) und (IV-B) immer an eine Harnstoff-Verknüpfungsgruppe L an der 4-Position des 1,3,5-Triazins für X=N gekoppelt ist oder an der 2-Position des Pyrimidins für X $CR^1$ ist, und eine beliebige Einheit A gemäß Formel (V-B) immer an zwei Harnstoff-Verknüpfungsgruppen L gekoppelt ist;

• $R^1$ aus der Gruppe ausgewählt ist, bestehend aus Wasserstoff und $C_1$-$C_{20}$-Alkyl;

• $R^2$ aus der Gruppe ausgewählt ist, bestehend aus $C_1$-$C_{20}$-Alkylen;

• $R^3$ aus der Gruppe ausgewählt ist, bestehend aus einer direkten kovalenten Bindung, linearen $C_1$-$C_{24}$-Alkylengruppen, verzweigten $C_2$-$C_{24}$-Alkylengruppen, cyclischen $C_3$-$C_{24}$-Alkylengruppen, $C_6$-$C_{24}$-Arylengruppen, $C_7$-$C_{24}$-Alkarylengruppen und $C_7$-$C_{24}$-Arylalkylengruppen, wobei die Alkylengruppen, Arylengruppen, Alkarylengruppen und Arylalkylengruppen optional 1-5 Heteroatome umfassen, die aus der Gruppe ausgewählt sind, bestehend aus O, N und S; und

• $R^4$ aus der Gruppe ausgewählt ist, bestehend aus linearen oder verzweigten $C_2$-$C_{20}$-Alkylengruppen und cyclischen $C_3$-$C_{24}$-Alkylengruppen; und

(b) Zusammensetzung (C2) eine wässrige Hilfsstoffformulierung ist, umfassend zu 75,0-99,9 Gew.-% Wasser, basierend auf dem Gewicht der Zusammensetzung (C2), zu 0-5 Gew.-% weitere Inhaltsstoffe und zu 0,1-20 Gew.-%:

• einen oder mehrere gelöste Vernetzer Z, die zwei oder mehr reaktive Gruppen aufweisen, die das Polymergelierungsmittel gemäß Formel (VIII) durch Ausbilden kovalenter Bindungen mit zwei oder mehr Gruppen E chemisch vernetzen können; oder

• eine oder mehrere gelöste Verbindungen Y, die aus der Gruppe ausgewählt sind, bestehend aus Oxidationsmittel, Oxidase, Peroxidase und Kombinationen davon, die eine chemische Vernetzung von zwei oder mehr Gruppen E auf dem Polymergeliermittel gemäß Formel (VIII) initiieren oder vermitteln;

wobei die Behandlung in einem ersten Schritt das Verabreichen einer der Zusammensetzungen (C1) oder (C2), vorzugsweise der Zusammensetzung (C1), in die Synovialflüssigkeit oder in den Hohlraum eines

Synovialgelenks des Wirbeltiers, und in einem zweiten Schritt das Verabreichen der anderen der Zusammensetzungen (C1) oder (C2) umfasst, um in dem Hohlraum des Synovialgelenks des Wirbeltiers eine flüssige Hydrogelzusammensetzung (C3) auszubilden, die auf ein vernetztes Hydrogel (C4) reagiert.

**3.** Kit aus Teilen zur Verwendung nach Anspruch 1 oder 2, wobei das Polymergelierungsmittel gemäß Formel (VIII) in Zusammensetzung (C1) durch ein Verfahren erhältlich ist, wobei:

(i) eine oder mehrere Verbindungen A', die aus der Gruppe ausgewählt sind, bestehend aus Formeln (I-A) bis (V-A):

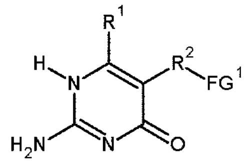

(I-A)

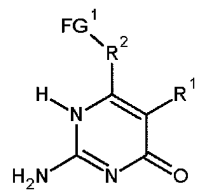

(II-A)

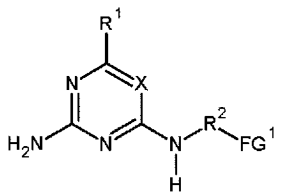

(III-A)

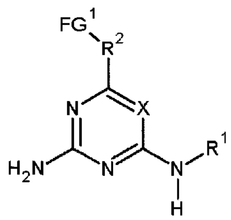

(IV-A)

$H_2N\text{-}R^3\text{-}NH_2$ (V-A)

reagiert werden, optional in einem Lösungsmittel, mit einer Diisocyanatverbindung gemäß Formel OCN-$R^4$-NCO und einem Polymer gemäß Formel $FG^1$-POL-$FG^1$, um ein Diisocyanat-Zwischenprodukt mit Formel (VI) auszubilden:

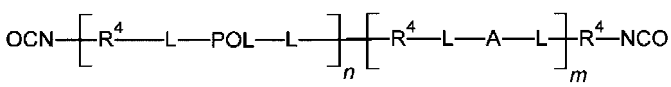

(VI)

wobei:

- m, *n,* X, $R^1$, $R^2$, $R^3$, $R^4$, POL, A und L wie in Anspruch 1 oder 2 definiert sind;
- jedes $FG^1$ eine funktionelle Gruppe ist, die aus OH und $N(R^1)H$ unabhängig ausgewählt ist;

(ii) Reagieren des Diisocyanat-Zwischenprodukts von Formel (VI) mit einer Verbindung gemäß Formel (VII):

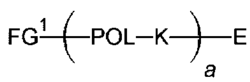

(VII)

wobei a, K und E wie in Anspruch 1 oder 2 definiert sind, um das Polymergelierungsmittel gemäß Formel (VIII) bereitzustellen.

**4.** Kit aus Teilen zur Verwendung nach einem der Ansprüche 1 bis 3, wobei die Zusammensetzung (C1) einen oder mehrere weitere Inhaltsstoffe umfasst, wobei der eine oder die mehreren weiteren Inhaltsstoffe aus der Gruppe ausgewählt sind, bestehend aus entzündungshemmenden Arzneimitteln, Corticosteroiden, Lokalanästhetika und Kombinationen davon.

**5.** Kit aus Teilen zur Verwendung nach einem der Ansprüche 1 bis 3, wobei die Zusammensetzung (C1) einen oder mehrere weitere Inhaltsstoffe umfasst, wobei der eine oder die mehreren weiteren Inhaltsstoffe aus der Gruppe ausgewählt sind, bestehend aus Peptiden, Proteinen, antimikrobiellen Mitteln, antiviralen Mitteln, Hormonen, parakrinen Faktoren, Wachstumsfaktoren, Vitaminen, entzündungshemmenden Arzneimitteln, Corticosteroiden, Lokalanästhetika und Kombinationen davon.

**6.** Kit aus Teilen zur Verwendung nach einem der Ansprüche 1 bis 4, wobei die Zusammensetzungen (C1) und (C2) nicht umfassen:

- Inhaltsstoffe, die aus tierischem oder menschlichem Gewebe gewonnen werden; und/oder
- Zellen, Zellextrakte, Proteine, Glycosaminoglycane und biologisch gewonnene Makromoleküle; und/oder
- Stammzellen und Wachstumsfaktoren.

**7.** Kit aus Teilen zur Verwendung nach einem der Ansprüche 4 bis 6, wobei die Zusammensetzung (C1) den einen oder die mehreren weiteren Inhaltsstoffe in einer Menge von 0,0005 bis 10 Gew.-%, basierend auf dem Gewicht der Zusammensetzung (C1), vorzugsweise 0,0005 bis 1 Gew.-%, am meisten bevorzugt 0,005 bis 1 Gew.-%, umfasst.

**8.** Kit aus Teilen zur Verwendung nach einem der Ansprüche 1 bis 7, wobei POL aus der Gruppe ausgewählt ist, bestehend aus Polyethern, Polyestern, Polycarbonaten, Polyamiden, Polyoxazolinen, Polyacrylaten, Polymethacrylaten, Polyolefinen, hydrierten Polyolefinen, Polysiloxanen, Polycarbonaten, (per)fluorierten Polyethern, Polyvinylenen oder Copolymeren derartiger Polymere und Kombinationen davon, vorzugsweise wobei POL aus Polyethylenglycolen und Polyoxazolinen ausgewählt ist.

**9.** Kit aus Teilen zur Verwendung nach einem der Ansprüche 1 bis 8, wobei A Formel (VIII) ist:

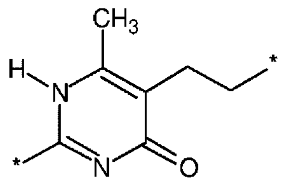

und wobei *-L-A-L-* ist:

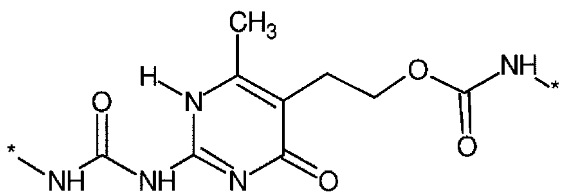

,

oder
wobei *-L-A-L-* ist:

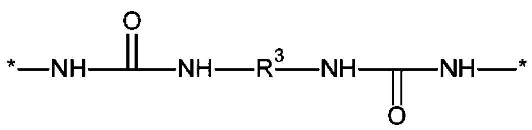

,

wobei $R^3$ aus der Gruppe ausgewählt ist, bestehend aus einer kovalenten Bindung, linearen $C_2$-$C_{12}$-Alkylengruppen und cyclischem $C_3$-$C_{12}$-Alkylen.

**10.** Kit aus Teilen zur Verwendung nach einem der Ansprüche 1 bis 9, wobei

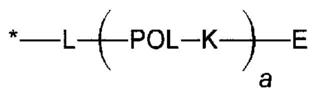

in dem Polymergeliermittel von Formel (VIII)

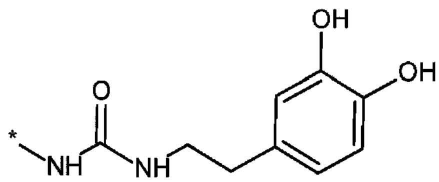

ist.

**11.** Kit aus Teilen zur Verwendung nach einem der Ansprüche 1 bis 10, wobei die flüssige Hydrogelformulierung der Zusammensetzung (C1) eine dynamische Viskosität bei 37 °C von etwa 0,01 bis etwa 20 Pa·s aufweist, wie mit einem Rheometer mit einer Platte-Platte-Geometrie bei einer Scherrate von 1 $s^{-1}$ und mit einem Spaltabstand von 0,50 mm gemessen, und/oder wobei die Wassermenge in der flüssigen Hydrogelzusammensetzung (C3) oder in dem vernetzten Hydrogel (C4) zwischen 51,0 und 99,9 Gew.-% der Zusammensetzung beziehungsweise des vernetzten Hydrogels beträgt, wobei die Menge des Polymergeliermittels gemäß Formel (VIII) in der flüssigen Hydrogelzusammensetzung (C3) oder in dem vernetzten Hydrogel (C4) zwischen 0,1-49,0 Gew.-% der Zusammensetzung beziehungsweise des vernetzten Hydrogels beträgt, und wobei die Menge der weiteren Inhaltsstoffe in der flüssigen Hydrogelzusammensetzung (C3) oder in dem vernetzten Hydrogel (C4) zwischen 0 und 19 Gew.-% der Zusammensetzung beziehungsweise des vernetzten Hydrogels beträgt.

**12.** Kit aus Teilen zur Verwendung nach einem der Ansprüche 1 bis 11, wobei die Behandlung oder Vorbeugung eine Entfernung von Synovialflüssigkeit aus dem Hohlraum des Synovialgelenks des Wirbeltiers vor einem Injizieren der Zusammensetzungen (C1) und (C2) oder (C3) umfasst, und/oder wobei die Behandlung oder Vorbeugung das Verabreichen von 0,5-250 ml der kombinierten Zusammensetzungen (C1) und (C2) oder der Zusammensetzung (C3), vorzugsweise 3-60 ml, mehr bevorzugt 6-60 ml, umfasst.

**13.** Kit aus Teilen zur Verwendung nach einem der Ansprüche 1 bis 12, wobei das Synovialgelenk des Wirbeltiers aus der Gruppe gewählt ist, bestehend aus Schultergürtel, Ellbogen, Handgelenk, Hand und Fingern, Beckengürtel, Knie, Knöchel, Fuß und Zehen, Kiefer- und Rumpfgelenken, und/oder wobei das Wirbeltier aus der Gruppe gewählt ist, bestehend aus Menschen, Vögeln, Haustieren, Renntieren und Rindern.

**14.** Kit aus Teilen zur Verwendung nach einem der Ansprüche 1 bis 13, wobei das Polymergeliermittel gemäß Formel (VIII) ein durchschnittliches Molekulargewicht $M_n$, wie unter Verwendung von SEC mit einem GPC-System bestimmt, ausgestattet mit einer KD-804-Säule (Showa Denko, 500-400.000 Da), unter Verwendung von RI-Detektion mit DMF, umfassend 10 mM LiBr als Eluent bei einer Flussrate von 1,0 ml/min bei 50 °C, wobei die SEC-Daten relativ zu PEO/PEG-Standards sind, zwischen 1 und 40 kDa, vorzugsweise zwischen 1,2 und 20 kDa, aufweist, und/oder wobei der pH-Wert der Zusammensetzung (C1) zwischen 3 und 6,5 liegt, und wobei der pH-Wert der Zusammensetzung (C2) zwischen 8 und 10 liegt, und/oder wobei die flüssige Hydrogelformulierung der Zusammensetzung (C1) und (C2) oder (C3) unter Verwendung einer Technik verabreicht wird, die aus der Gruppe gewählt ist, bestehend aus Injektion, arthroskopischer Chirurgie und offener Chirurgie, vorzugsweise Injektion.

**15.** Kit aus Teilen zur Verwendung nach einem der Ansprüche 1 bis 14, wobei das vernetzte Hydrogel (C4) nach 16 h Aushärtung mindestens eine der folgenden Eigenschaften (i) bis (iii) aufweist:

i) kein Versagen bei einem einaxialen Druck von 4,0 MPa bei 37 °C;
ii) kein Versagen bei einem einaxialen Druck auf mindestens 10 % seiner ursprünglichen Dicke bei 37 °C;
iii) eine maximale mechanische Spannung von mindestens 0,4 MPa bei 25 % Dehnung unter einaxialem Druck

bei 37 °C.

**Revendications**

1. Kit de pièces, constitué de compositions (C1) et (C2) dans des récipients séparés, pour utilisation dans le traitement ou la prévention de l'arthrose chez un vertébré, dans lequel :

(a) la composition (C1) est une formulation d'hydrogel liquide comprenant 50,0 à 99,7 % en poids d'eau, sur la base du poids de la composition (C1), 0,3 à 50,0 % en poids d'un gélifiant polymère selon la Formule (VIII) et 0 à 20 % en poids d'autres ingrédients :

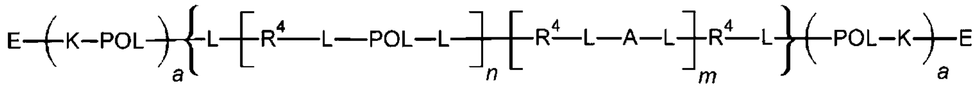

(VIII)

dans laquelle :

- m et *n* sont chacun des nombres entiers indépendants compris dans la plage de 1 à 20 ;
- a est un nombre entier choisi parmi 0 ou 1 ;
- K est un groupe de liaison choisi parmi amide, ester ou éther ;
- E est un groupe qui peut réagir avec un autre groupe E déclenché ou médié par un ou plusieurs composés Y, le ou les composés Y étant choisis dans le groupe constitué d'oxydant, oxydase, peroxydase et combinaisons de ceux-ci, dans la formulation auxiliaire aqueuse (C2), permettant ainsi de former une ou plusieurs liaisons covalentes entre différents groupes E, ou E est un groupe qui peut réagir avec un ou plusieurs agents de réticulation Z dans une formulation auxiliaire aqueuse (C2), le ou les agents de réticulation Z ayant deux groupes réactifs ou plus, formant ainsi des liaisons covalentes entre E et le ou les agents de réticulation Z ;
- POL est un groupe polymère linéaire ayant une masse moléculaire moyenne $M_n$, comme déterminé par titrage des groupes terminaux, d'environ 250 et à environ 30 000 Da ;
- A représente des groupements choisis dans le groupe constitué des Formules (I-B) à (V-B), A étant relié à L par le biais des liaisons marquées d'un astérisque :

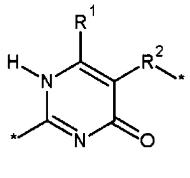

(I-B)

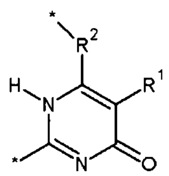

(II-B)

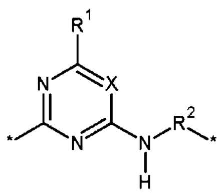

(III-B)

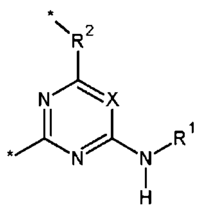

(IV-B)

$*-R^3-*$ (V-B)

- X est N ou $CR^1$ ;
- chaque L est indépendamment un groupe de liaison uréthane ou urée, à condition que tout groupement A

selon les Formules (I-B) et (II-B) est toujours couplé à un groupe de liaison urée L sur la position 2 de la 4-pyrimidone, tout groupement A selon les Formules (III-B) et (IV-B) est toujours couplé à un groupe de liaison urée L sur la position 4 de la 1,3,5-triazine pour X=N, ou sur la position 2 de la pyrimidine pour X est $CR^1$, et tout groupement A selon la Formule (V-B) est toujours couplé à deux groupes de liaison urée L ;

- $R^1$ est choisi dans le groupe constitué d'hydrogène et alkyle en $C_1$-$C_{20}$ ;
- $R^2$ est choisi dans le groupe constitué d'alkylène en $C_1$-$C_{20}$ ;
- $R^3$ est choisi dans le groupe constitué d'une liaison covalente directe, de groupes alkylène linéaire en $C_1$-$C_{24}$, groupes alkylène ramifié en $C_2$-$C_{24}$, groupes alkylène cyclique en $C_3$-$C_{24}$, groupes arylène en $C_6$-$C_{24}$, groupes alcarylène en $C_7$-$C_{24}$ et groupes arylalkylène en $C_7$-$C_{24}$, les groupes alkylène, groupes arylène, groupes alcarylène et groupes arylalkylène comprenant facultativement de 1 à 5 hétéroatomes choisis dans le groupe constitué de O, N et S ; et
- $R^4$ est choisi dans le groupe constitué de groupes alkylène linéaire ou ramifié en $C_2$-$C_{20}$ et des groupes alkylène cyclique en $C_3$-$C_{24}$ ; et

(b) la composition (C2) est une formulation auxiliaire aqueuse comprenant 75,0 à 99,9 % en poids d'eau, sur la base du poids de la composition (C2), 0 à 5 % en poids d'autres ingrédients et 0,1 à 20 % en poids de :

- un ou plusieurs agents de réticulation dissous Z ayant deux groupes réactifs ou plus qui peuvent réticuler chimiquement le gélifiant polymère selon la Formule (VIII) en formant des liaisons covalentes avec deux groupes E ou plus ; ou
- un ou plusieurs composés dissous Y, choisis dans le groupe constitué d'oxydant, oxydase, peroxydase et combinaisons de ceux-ci, qui déclenchent ou médient une réticulation chimique de deux groupes E ou plus sur le gélifiant polymère selon la Formule (VIII) ;

dans lequel ledit traitement comprend la combinaison des compositions (C1) et (C2) pour former une composition d'hydrogel liquide (C3) et l'administration de ladite composition d'hydrogel liquide (C3) au liquide synovial ou à la cavité d'une articulation synoviale du vertébré, afin de former un hydrogel réticulé (C4) dans la cavité de l'articulation synoviale du vertébré.

**2.** Kit de pièces, constitué de compositions (C1) et (C2) dans des récipients séparés, pour utilisation dans le traitement ou la prévention de l'arthrose chez un vertébré, dans lequel

(a) la composition (C1) est une formulation d'hydrogel liquide comprenant 50,0 à 99,7 % en poids d'eau, sur la base du poids de la composition (C1), 0,3 à 50,0 % en poids d'un gélifiant polymère selon la Formule (VIII) et 0 à 20 % en poids d'autres ingrédients :

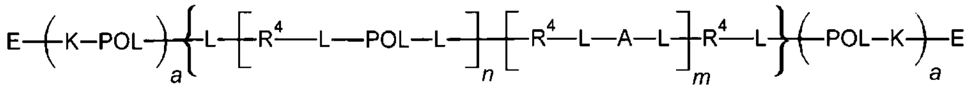

(VIII)

dans laquelle :

- m et *n* sont chacun des nombres entiers indépendants compris dans la plage de 1 à 20 ;
- a est un nombre entier choisi parmi 0 ou 1 ;
- K est un groupe de liaison choisi parmi amide, ester ou éther ;
- E est un groupe qui peut réagir avec un autre groupe E déclenché ou médié par un ou plusieurs composés Y, le ou les composés Y étant choisis dans le groupe constitué d'oxydant, oxydase, peroxydase et combinaisons de ceux-ci, dans la formulation auxiliaire aqueuse (C2), permettant ainsi de former une ou plusieurs liaisons covalentes entre différents groupes E, ou E est un groupe qui peut réagir avec un ou plusieurs agents de réticulation Z dans une formulation auxiliaire aqueuse (C2), le ou les agents de réticulation Z ayant deux groupes réactifs ou plus, formant ainsi des liaisons covalentes entre E et le ou les agents de réticulation Z ;
- POL est un groupe polymère linéaire ayant une masse moléculaire moyenne $M_n$, comme déterminé par titrage des groupes terminaux, d'environ 250 et à environ 30 000 Da ;
- A représente des groupements choisis dans le groupe constitué des Formules (I-B) à (V-B), A étant relié à L par le biais des liaisons marquées d'un astérisque :

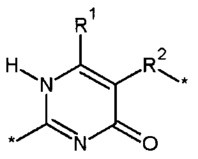

(I-B)

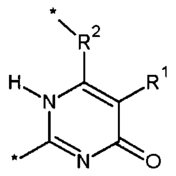

(II-B)

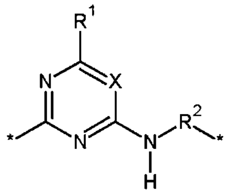

(III-B)

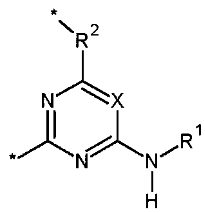

(IV-B)

$*-R^3-*$ (V-B)

- X est N ou $CR^1$ ;
- chaque L est indépendamment un groupe de liaison uréthane ou urée, à condition que tout groupement A selon les Formules (I-B) et (II-B) est toujours couplé à un groupe de liaison urée L sur la position 2 de la 4-pyrimidone, tout groupement A selon les Formules (III-B) et (IV-B) est toujours couplé à un groupe de liaison urée L sur la position 4 de la 1,3,5-triazine pour X=N, ou sur la position 2 de la pyrimidine pour X est $CR^1$, et tout groupement A selon la Formule (V-B) est toujours couplé à deux groupes de liaison urée L ;
- $R^1$ est choisi dans le groupe constitué d'hydrogène et alkyle en $C_1$-$C_{20}$ ;
- $R^2$ est choisi dans le groupe constitué d'alkylène en $C_1$-$C_{20}$ ;
- $R^3$ est choisi dans le groupe constitué d'une liaison covalente directe, de groupes alkylène linéaire en $C_1$-$C_{24}$, groupes alkylène ramifié en $C_2$-$C_{24}$, groupes alkylène cyclique en $C_3$-$C_{24}$, groupes arylène en $C_6$-$C_{24}$, groupes alcarylène en $C_7$-$C_{24}$ et groupes arylalkylène en $C_7$-$C_{24}$, les groupes alkylène, groupes arylène, groupes alcarylène et groupes arylalkylène comprenant facultativement de 1 à 5 hétéroatomes choisis dans le groupe constitué de O, N et S ; et
- $R^4$ est choisi dans le groupe constitué de groupes alkylène linéaire ou ramifié en $C_2$-$C_{20}$ et des groupes alkylène cyclique en $C_3$-$C_{24}$ ; et

(b) la composition (C2) est une formulation auxiliaire aqueuse comprenant 75,0 à 99,9 % en poids d'eau, sur la base du poids de la composition (C2), 0 à 5 % en poids d'autres ingrédients et 0,1 à 20 % en poids de :

- un ou plusieurs agents de réticulation dissous Z ayant deux groupes réactifs ou plus qui peuvent réticuler chimiquement le gélifiant polymère selon la Formule (VIII) en formant des liaisons covalentes avec deux groupes E ou plus ; ou
- un ou plusieurs composés dissous Y, choisis dans le groupe constitué d'oxydant, oxydase, peroxydase et combinaisons de ceux-ci, qui déclenchent ou médient une réticulation chimique de deux groupes E ou plus sur le gélifiant polymère selon la Formule (VIII) ;

dans lequel ledit traitement comprend, dans une première étape, l'administration de l'une des compositions (C1) ou (C2), de préférence de la composition (C1), au liquide synovial ou à la cavité d'une articulation synoviale du vertébré et, dans une seconde étape, l'administration de l'autre des compositions (C1) ou (C2), pour former une composition d'hydrogel liquide (C3) dans la cavité de l'articulation synoviale du vertébré qui réagit en un hydrogel réticulé (C4).

**3.** Kit de pièces pour utilisation selon la revendication 1 ou 2, dans lequel le gélifiant polymère selon la Formule (VIII) dans la composition (C1) peut être obtenu par un procédé dans lequel :

(i) un ou plusieurs composés A' choisis dans le groupe constitué des Formules (I-A) à (V-A) :

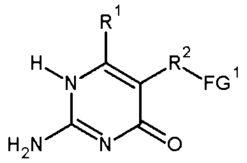

(I-A)

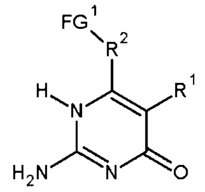

(II-A)

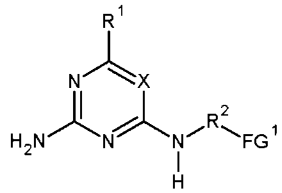

(III-A)

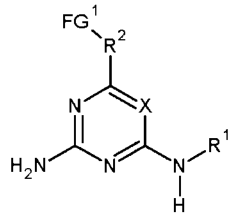

(IV-A)

$H_2N\text{-}R^3\text{-}NH_2$ (V-A)

sont amenés à réagir, facultativement dans un solvant, avec un composé diisocyanate selon la Formule $OCN\text{-}R^4\text{-}NCO$ et un polymère selon la Formule $FG^1\text{-}POL\text{-}FG^1$ afin de former un produit diisocyanate intermédiaire de Formule (VI) :

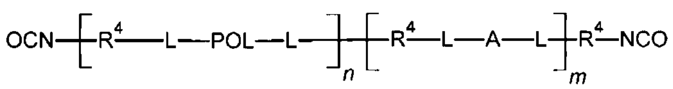

(VI)

dans laquelle :

- m, *n,* X, $R^1$, $R^2$, $R^3$, $R^4$, POL, A, et L sont tels que définis dans la revendication 1 ou la revendication 2 ;
- chaque $FG^1$ est un groupe fonctionnel choisi indépendamment parmi OH et $N(R^1)H$ ;

(ii) la réaction du produit diisocyanate intermédiaire de la Formule (VI) avec un composé selon la Formule (VII) :

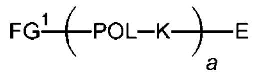

(VII)

dans laquelle a, K et E sont tels que définis dans la revendication 1 ou 2, afin d'obtenir le polymère gélifiant selon la Formule (VIII).

**4.** Kit de pièces pour utilisation selon l'une quelconque des revendications 1 à 3, dans lequel la composition (C1) comprend un ou plusieurs autres ingrédients, dans lequel ledit ou lesdits autres ingrédients sont choisis dans le groupe constitué de médicaments anti-inflammatoires, corticostéroïdes, anesthésiques locaux et combinaisons de ceux-ci.

**5.** Kit de pièces pour utilisation selon l'une quelconque des revendications 1 à 3, dans lequel la composition (C1) comprend un ou plusieurs autres ingrédients, dans lequel ledit ou lesdits autres ingrédients sont choisis dans le groupe constitué de peptides, protéines, agents antimicrobiens, agents antiviraux, hormones, facteurs paracrines, facteurs de croissance, vitamines, médicaments anti-inflammatoires, corticostéroïdes, anesthésiques locaux et

combinaisons de ceux-ci.

6. Kit de pièces pour utilisation selon l'une quelconque des revendications 1 à 4, dans lequel les compositions (C1) et (C2) ne comprennent pas :

   • des ingrédients dérivés de tissus animaux ou humains ; et/ou
   • des cellules, extraits cellulaires, protéines, glycosaminoglycanes et macromolécules d'origine biologique ; et/ou
   • des cellules souches et facteurs de croissance.

7. Kit de pièces pour utilisation selon l'une quelconque des revendications 4 à 6, dans lequel la composition (C1) comprend le ou les autres ingrédients en une quantité de 0,0005 à 10 % en poids, sur la base du poids de la composition (C1), de préférence de 0,0005 à 1 % en poids, le plus préférablement de 0,005 à 1 % en poids

8. Kit de pièces pour utilisation selon l'une quelconque des revendications 1 à 7, dans lequel POL est choisi dans le groupe constitué de polyéthers, polyesters, polycarbonates, polyamides, polyoxazolines, polyacrylates, polyméthacrylates, polyoléfines, polyoléfines hydrogénées, polysiloxanes, polycarbonates, polyéthers (per)fluorés, polyvinylènes, ou copolymères de ces polymères, et combinaisons de ceux-ci, de préférence dans lequel POL est choisi parmi des polyéthylènes glycols et polyoxazolines.

9. Kit de pièces pour utilisation selon l'une quelconque des revendications 1 à 8, dans lequel A la Formule (VIII) est :

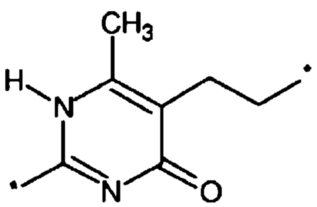

et dans lequel *-L-A-L-* est :

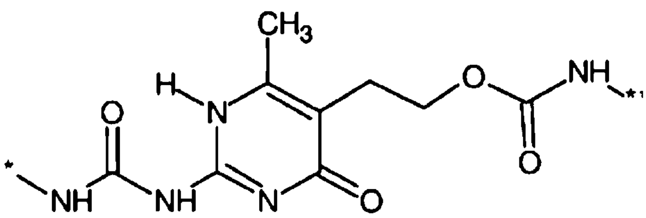

,

ou
dans lequel *-L-A-L-* est :

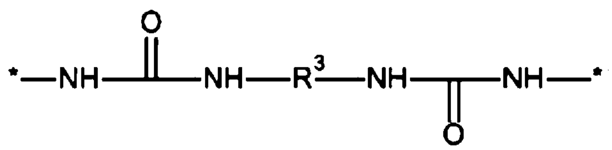

,

dans lequel $R^3$ est choisi dans le groupe constitué d'une liaison covalente, groupes alkyle linéaire en $C_2$-$C_{12}$ et alkylène cyclique en $C_3$-$C_{12}$.

10. Kit de pièces pour utilisation selon l'une quelconque des revendications 1 à 9, dans lequel

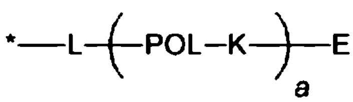

dans le gélifiant polymère de Formule (VIII) est

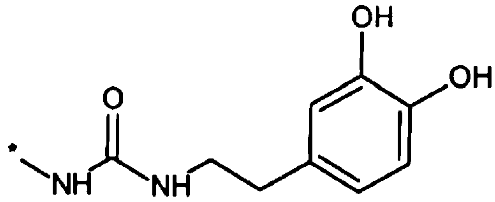

.

**11.** Kit de pièces pour utilisation selon l'une quelconque des revendications 1 à 10, dans lequel la formulation d'hydrogel liquide de la composition (C1) a une viscosité dynamique à 37 °C d'environ 0,01 à environ 20 Pa·s, comme mesuré avec un rhéomètre à géométrie plaque/plaque à un taux de cisaillement de 1 $s^{-1}$ et avec une distance d'espacement de 0,50 mm, et/ou dans lequel la quantité d'eau dans la composition d'hydrogel liquide (C3) ou dans l'hydrogel réticulé (C4) est comprise entre 51,0 et 99,9 % en poids de la composition ou de l'hydrogel réticulé, respectivement. dans lequel la quantité du gélifiant polymère selon la Formule (VIII) dans la composition d'hydrogel liquide (C3) ou dans l'hydrogel réticulé (C4) est comprise entre 0,1 et 49,0 % en poids de la composition ou de l'hydrogel réticulé, respectivement, et dans lequel la quantité des autres ingrédients dans la composition d'hydrogel liquide (C3) ou dans l'hydrogel réticulé (C4) est comprise entre 0 et 19 % en poids de la composition ou de l'hydrogel réticulé, respectivement.

**12.** Kit de pièces pour utilisation selon l'une quelconque des revendications 1 à 11, dans lequel ledit traitement ou ladite prévention comprend l'élimination du liquide synovial de la cavité de l'articulation synoviale du vertébré avant l'injection des compositions (C1) et (C2), ou (C3), et/ou dans lequel ledit traitement ou ladite prévention comprend l'administration de 0,5 à 250 ml des compositions combinées (C1) et (C2) ou de la composition (C3), de préférence de 3 à 60 ml, plus préférablement de 6 à 60 ml.

**13.** Kit de pièces pour utilisation selon l'une quelconque des revendications 1 à 12, dans lequel l'articulation synoviale du vertébré est choisie dans le groupe constitué de ceinture scapulaire, coude, poignet, main et doigts, ceinture pelvienne, genou, cheville, pied et orteils, mâchoire et articulations du tronc, et/ou dans lequel le vertébré est choisi dans le groupe constitué des êtres humains, oiseaux, animaux de compagnie, animaux de course et bétail.

**14.** Kit de pièces pour utilisation selon l'une quelconque des revendications 1 à 13, dans lequel le gélifiant polymère selon la Formule (VIII) a une masse moléculaire moyenne $M_n$, comme déterminé par SEC avec un système GPC équipé d'une colonne KD-804 (Showa Denko, 500 à 400 000 Da) utilisant une détection RI avec du DMF comprenant 10 mM LiBr comme éluant à un débit de 1,0 mL/min à 50 °C, les données SEC étant relatives aux témoins PEO/PEG, entre 1 et 40 kDa, de préférence entre 1,2 et 20 kDa, et/ou dans lequel le pH de la composition (C1) est compris entre 3 et 6,5, et dans lequel le pH de la composition (C2) est compris entre 8 et 10, et/ou dans lequel la formulation d'hydrogel liquide de la composition (C1) et (C2) ou (C3) est administrée à l'aide d'une technique choisie dans le groupe constitué d'une injection, d'une chirurgie arthroscopique et d'une chirurgie ouverte, de préférence une injection.

**15.** Kit de pièces pour utilisation selon l'une quelconque des revendications 1 à 14, dans lequel l'hydrogel réticulé (C4), après 16 h de durcissement, a au moins l'une des propriétés (i) à (iii) suivantes :

i) pas de rupture à une compression non confinée de 4,0 MPa à 37 °C ;
ii) pas de rupture à une compression non confinée à au moins 10 % de son épaisseur d'origine à 37 °C ;
iii) une contrainte maximale d'au moins 0,4 MPa à 25 % de déformation en compression confinée à 37 °C.

*Fig. 1*

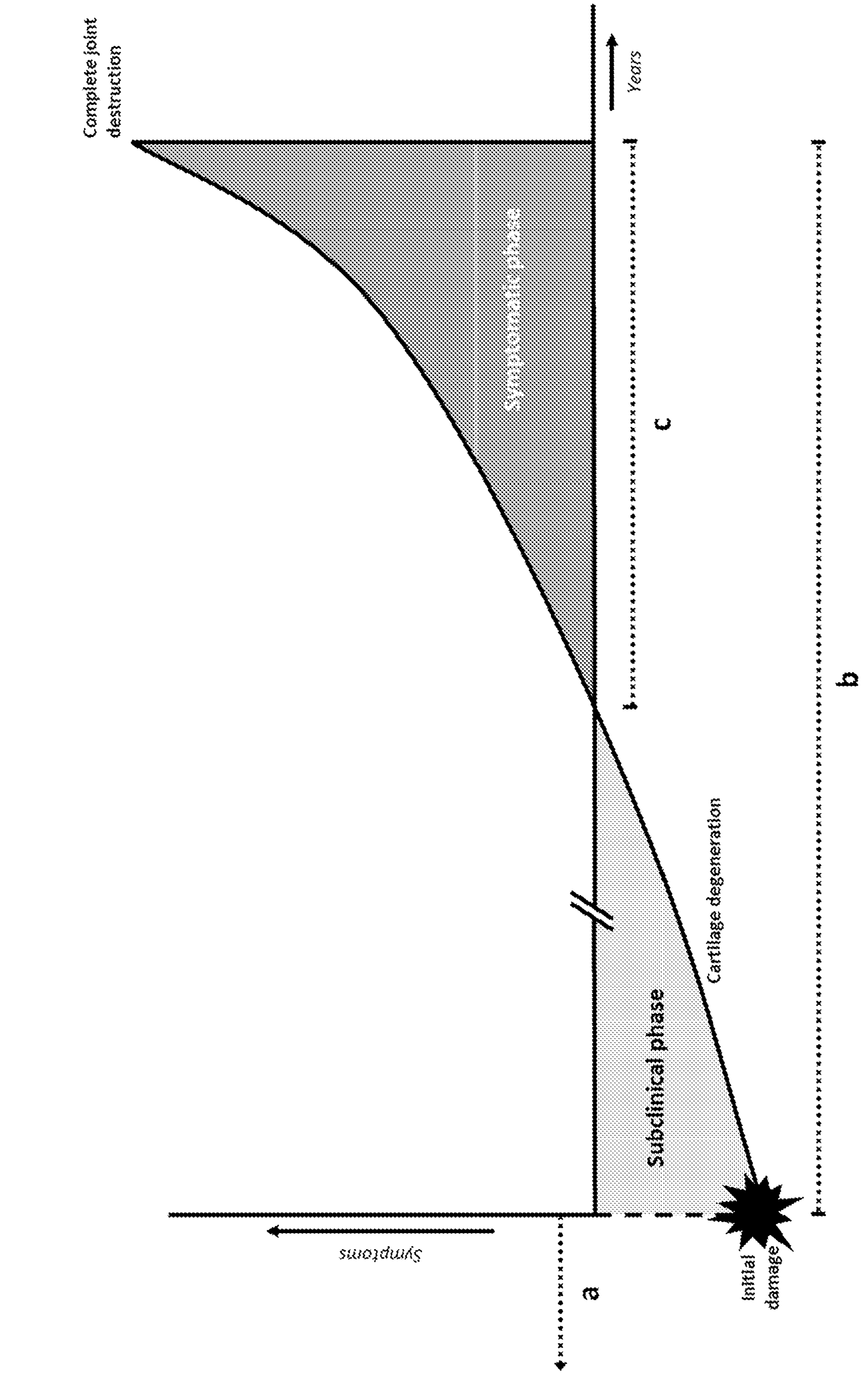
Complete joint destruction
Years
Symptomatic phase
c
b
Cartilage degeneration
Subclinical phase
Symptoms
a
Initial damage

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011059325 A2
- WO 2006118460 A1
- EP 1972661 A1
- US 10358521 E1
- US 2017340774 A1


### Non-patent literature cited in the description


- **D.L. BADER et al.** *Arthritis*, 2011, vol. 2011, 979032
- **K. WIEGANT et al.** *Osteoarthritis Cartilage*, 2013, vol. 21, 1660-1667
- **E.C. RODRIGUEZ-MERCHAN.** *Arch. Bone Jt. Surg.*, 2017, vol. 5, 208-212
- **E. JOOYBAR et al.** *Acta Biomat.*, 2019, vol. 83, 233-244
- **M.M.C. BASTINGS et al.** *Adv. Healthcare Mat.*, 2014, vol. 3, 70-78
- **P.Y.W. DANKERS et al.** *Adv. Mater.*, 2012, vol. 24, 2703-2709
- **H. WANG et al.** *Adv. Mat.*, 2015, vol. 27, 3717-3736
- **A.R. CAMERON et al.** *Biomaterials*, 2014, vol. 35, 1857-1868
- **C. CORREIA et al.** *Tissue Engin. A*, 2012, vol. 18, 1979-1991